# EUROPEAN PATENT SPECIFICATION

(11) **EP 2 310 000 B1**
(45) Date of publication and mention of the grant of the patent: **18.09.2019**
(21) Application number: 09771218.6
(22) Date of filing: 26.06.2009
(51) Int. Cl.: A61K 9/51, A61K 31/48

(54) **DERMAL DELIVERY**
DERMALE VERABREICHUNG
ADMINISTRATION DERMIQUE

(30) Priority: 26.06.2008 US 76065 P
(43) Date of publication of application: 20.04.2011
(73) Proprietor: Anterios, Inc., Madison, NJ 07940 (US)
(72) Inventor: EDELSON, Jonathan, Scarsdale NY 10583 (US); KOTYLA, Timothy, Lowell MA 01854 (US); ZHANG, Boke, Brighton MA 02135 (US)
(74) Representative: Cornish, Kristina Victoria Joy
(86) International application number: PCT/US2009/048972
(87) International publication number: WO 2009/158687

(56) References cited:
- WO-A-03/000243
- WO-A-2005/027872
- WO-A-2008/045107
- US-A1- 2003 113 349
- US-A1- 2005 136 024
- US-A1- 2007 036 831

## Description

### Background

Conditions or disorders associated with sweat glands or sebaceous glands can cause a great deal of unhappiness and psychological debilitation for those who suffer from them, and current treatments are not very successful and often have undesirable side effects. For example, according to studies, acne often leads to reduced self esteem, and sometimes even to depression or suicide (see, *e.g.,* Goodman, 2006, Aust. Fam. Physician 35:503, 2006; Purvis et al., 2006, J. Paediatr. Child. Health 42:793;. Similar challenges are observed with hyperhidrosis (excessive sweating), bromhidrosis (body odor), chromhidrosis (colored sweat), psoriasis, dermal infection (*e.g.,* herpes simplex virus infection, human papillomavirus infection, fungal infection, *etc*.), hair loss, actinic keratosis, rosacea, and other afflictions of the skin.

### Summary of the Invention

The protection sought for this invention is as defined in the claims.

The present invention provides nanoemulsions as claimed for use in methods of treating conditions or disorders associated with dermal structures (*e.g.,* rosacea, sebaceous glands, such as excess sebum producing disorders (*e.g.* acne)). Specifically, the present invention demonstrates that nanoparticle compositions (*e.g.,* nanoemulsions) can deliver active agents efficiently and specifically to the dermis. For example, the present invention demonstrates dermal delivery without significant side effects associated with delivery to other areas (*e.g.,* to subdermal or extradermal structures and/or to tissues other than dermis).

The present invention therefore provides nanoemulsions as claimed for use in methods of treating conditions or disorders associated with dermal structures by applying to a skin surface a composition containing a nanoparticle composition (*e.g.,* a nanoemulsion) that includes a therapeutic agent useful in the treatment of the condition or disorder. In general, a nanoparticle composition is arranged and constructed such that an amount of therapeutic agent is delivered to dermal structures that is sufficient to treat the condition or disorder. In general, a nanoparticle composition is arranged and constructed such that it does not induce unwanted clinical effects inside and/or outside of the dermis.

For example, the present invention provides nanoemulsions as claimed for use in methods comprising dermal administration of a nanoparticle composition (*e.g.* a nanoemulsion) containing a therapeutic agent without clinically significant side effects such as systemic side effects, damage to nervous tissue underlying the dermis (*e.g.,* neuronal paralysis), unwanted effects on muscles (*e.g.,* muscle paralysis), undesirable blood levels of therapeutic agent, *etc.*

To give but one example, the present invention provides nanoemulsions as claimed for use in methods for treating conditions associated with skin glands utilizing nanoparticle compositions (*e.g.,* nanoemulsions) containing botulinum toxin. Furthermore, the data presented herein demonstrate effective and efficient delivery of botulinum toxin to the dermis (which houses the sebaceous glands) using such nanoparticle compositions. Additionally, the data presented herein demonstrate that delivery of botulinum toxin to the dermis can be achieved without unwanted clinical effects associated with such delivery (*e.g.,* one or more of systemic side effects, damage to underlying nervous tissue [*e.g.,* neuronal paralysis], unwanted effects on muscles [*e.g.,* muscle paralysis], undesirable blood levels, *etc*.).

The present invention therefore also demonstrates the usefulness of such botulinum nanoparticle compositions in the treatment of other disorders and conditions associated with the dermis, or defects therein. For example, as addressed below in Example 5, the present invention provides botulinum nanoparticle compositions for use in the treatment of acne. For example, as addressed below in Example 6, the present invention provides use of botulinum nanoparticle compositions in the treatment of rosacea.

According to the present invention, nanoparticle compositions (*e.g.* nanoemulsions) containing one or more therapeutic agents are useful in various cosmetic and medical applications. In some embodiments, such nanoparticle compositions are utilized to treat acne. In some embodiments, such nanoparticle compositions are used to treat disorders or conditions associated with sebaceous glands, such as excess sebum-producing disorders (*e.g.,* seborrhea, seborrheic dermatitis, *etc*.). In some embodiments, such nanoparticle compositions are used to treat disorders or conditions associated with any component of the dermis that is present at around the same level of depth as sebaceous glands. In some embodiments, such nanoparticle compositions are used to treat rosacea.

Nanoparticle compositions formulated and used according to the present invention achieve transdermal delivery of therapeutic agents. Such compositions therefore avoid problems often associated with other delivery systems, including injection and oral delivery systems. Botulinum toxin, for example, is most commonly delivered by injection. Indeed, injection is currently the only delivery method that is approved by the United States Food and Drug Administration (USFDA). Improper injection techniques can damage tissue and /or can deliver therapeutic agents (*e.g.,* botulinum toxin) to unintended and/or undesirable locations. Pain, hematoma, ecchymosis, and bruising can also occur. Efforts have been made to develop transdermal delivery systems, including for botulinum toxin; however, these systems typically employ one or more agents that disrupt the skin, either chemically or mechanically. The present invention, by contrast, provides the surprising finding that certain nanoparticle compositions can efficiently and appropriately deliver therapeutic agents, including botulinum toxin, to the dermal layer (*e.g.,* to sebaceous gland regions) of the skin. The present invention therefore surprisingly demonstrates that inventive nanoparticle compositions are useful in the treatment of a variety of disorders or conditions associated with the sweat or sebaceous glands, and not just with certain of such disorders or conditions (*e.g.,* ones that might be tolerant of less precise or less efficient delivery). The present invention also demonstrates the effective and efficient delivery of therapeutically active agents to the dermis and therefore illustrates the usefulness of nanoparticle compositions in the treatment of disorders or conditions of the dermis (*e.g.,* rosacea).

The inventors have discovered that certain nanoparticle compositions (*e.g.* nanoemulsions) can achieve transdermal delivery of therapeutic agents without changing or altering the structure of the skin (see, *e.g.,* co-pending U.S. Patent Application U.S.S.N. 11/607,436, entitled "BOTULINUM NANOEMULSIONS," filed December 1, 2006;). For example, abrasive agents or agents that erode or deteriorate the superficial layer of the skin are not required to achieve transdermal delivery of botulinum toxin according to the present invention. Thus, in many embodiments, transdermal delivery of therapeutic agents (*e.g.,* botulinum toxin) is accomplished without significant irritation of the skin.

In some embodiments, nanoparticle compositions (*e.g.* nanoemulsions) for use in accordance with the present invention are prepared by exposure to high shear forces; in some embodiments, nanoparticle compositions are prepared by microfluidization; in some embodiments, nanoparticle compositions are prepared by high pressure homogenization.

According to the present invention, transdermal delivery of therapeutic agents (*e.g.,* botulinum toxin) may be accomplished in any of a variety of formats. In some embodiments, a nanoparticle composition comprising one or more therapeutic agents (*e.g.,* botulinum toxin) is incorporated within a cream, gel, powder, or lotion such that the therapeutic agent(s) are administered to a subject by application to the skin. In some embodiments, a nanoparticle composition comprising one or more therapeutic agents (*e.g.,* botulinum toxin) is incorporated within an ointment and/or liniment such that the therapeutic agent(s) are administered to a subject by application to the skin. The nanoparticle composition comprising one or more therapeutic agents (*e.g.,* botulinum toxin) is incorporated within a nanoemulsion, such that the therapeutic agent(s) are administered to a subject by application to the skin. In some embodiments, a nanoparticle composition is incorporated within a transdermal patch such that a therapeutic agent (*e.g.,* botulinum toxin) is administered to a subject from the patch.

The nanoparticle compositions of the present invention are emulsions containing a population of particles having maximum and minimum diameters, wherein the difference between the maximum and minimum diameters does not exceed about 600 nanometers (nm), about 550 nm, about 500 nm, about 450 nm, about 400 nm, about 350 nm, about 300 nm, about 250 nm, about 200 nm, about 150 nm, about 100 nm, about 90 nm, about 80 nm, about 70 nm, about 60 nm, about 50 nm, or fewer than about 50 nm.

In some embodiments, particles (*e.g.,* particles containing one or more therapeutic agents) within nanoparticle compositions (*e.g.* nanoemulsions) have diameters that are smaller than about 600 nm, about 550 nm, about 500 nm, about 450 nm, about 400 nm, about 350 nm, about 300 nm, about 250 nm, about 200 nm, about 150 nm, about 130 nm, about 120 nm, about 115 nm, about 110 nm, about 100 nm, about 90 nm, about 80 nm, about 70 nm, about 60 nm, about 50 nm, about 40 nm, about 30 nm, about 20 nm, or less than about 20 nm.

In some embodiments, particles (*e.g.,* particles containing one or more therapeutic agents) within nanoparticle compositions (*e.g.* nanooemulsions) have diameters within the range of about 10 and about 600 nm. In some embodiments, particles within nanoparticle compositions (*e.g.* nanoemulsions) have diameters within the range of about 10 nm and about 300 nm, about 10 nm and about 200 nm, about 10 nm and about 150 nm, about 10 nm and about 130 nm, about 10 nm and about 120 nm, about 10 nm and about 115 nm, about 10 nm and about 110 nm, about 10 nm and about 100 nm, or about 10 nm and about 90 nm.

In some embodiments, particles (*e.g.,* particles containing one or more therapeutic agents) within nanoparticle compositions (*e.g.* nanoemulsions) have an average particle size that is under about 300 nm, about 250 nm, about 200 nm, about 150 nm, about 130 nm, about 120 nm, about 115 nm, about 110 nm, about 100 nm, or about 90 nm. In some embodiments, the average particle size is within the range of about 10 nm and about 300 nm, about 50 nm and about 250 nm, about 60 nm and about 200 nm, about 65 nm and about 150 nm, about 70 nm and about 130 nm. In some embodiments, the average particle size is about 80 nm and about 110 nm, about 70 nm and about 90 nm, about 60 nm and about 80 nm, about 50 nm and about 70 nm, about 10 nm and about 50 nm. In some embodiments, the average particle size is about 90 nm and about 100 and nm.

In some embodiments, a majority of the particles (*e.g.,* particles containing one or more therapeutic agents) within compositions in accordance with the invention have diameters below a specified size or within a specified range. In some embodiments, the majority is more than 50%, 60%, 70%, 75%, 80%, 85%, 90%, 95%, 96%, 97%, 98%, 99%, 99.5%, 99.6%, 99.7%, 99.8%, 99.9% or more of the particles in the composition.

In some embodiments, nanoparticle compositions (*e.g.* nanoemulsions) are substantially free of particles (*e.g.,* particles containing one or more therapeutic agents) having diameters greater than about 120 nm. In some embodiments, particles (*e.g.,* particles containing one or more therapeutic agents) within nanoparticle compositions (*e.g.* nanoemulsions) have diameters within the range of about 30 nm and about 115 nm. In some embodiments, most of the particles within the composition have diameters within this range; in some embodiments, such compositions are substantially free of particles having diameters larger than about 115 nm. In some embodiments, particles within nanoparticle compositions (*e.g.* nanoemulsions) have diameters within the range of about 30 nm to about 70 nm or 40 nm to 90 nm. In some embodiments, most of the particles within such compositions have diameters within this range; in some embodiments the compositions are substantially free of particles with diameters larger than about 70 nm.

In some embodiments, nanoparticle compositions (*e.g.* nanoemulsions) have at least two distinct populations of particles. For example, in some such embodiments, a majority of the particles in nanoparticle compositions (*e.g.* nanoemulsions) have diameters within the range of about 30 nm and about 70 nm, while a second population of particles has diameters within the range of about 70 nm and about 120 nm. In some such embodiments, the composition is not contaminated with particles greater than 120 nm in diameter.

In some embodiments, at least one therapeutic agent (*e.g.,* botulinum toxin) is present partially or entirely within nanoparticles in nanoparticle compositions (*e.g.* nanoemulsions); in some embodiments, at least one therapeutic agent is adsorbed on the surface of nanoparticles in nanoparticle compositions; in some embodiments, at least one therapeutic agent is associated with the interface between the nanoparticles and the dispersion medium. In some embodiments, at least one therapeutic agent is found in two or more of these locations within the nanoparticle composition.

The therapeutic agent to be incorporated within and/or associated with nanoparticles is any agent (*e.g.* botulinum toxin) that is useful for treating skin disorders at the dermal level (*e.g.,* any agent useful for treating acne, rosaceaand/or excess sebum-producing disorders (*e.g.,* seborrhea, seborrheic dermatitis, *etc.*

The therapeutic agent is botulinum toxin.

In some embodiments, botulinum toxin is selected from the group consisting of type A, type B, type C1, type C2, type D, type E, type F, and type G. In some embodiments, botulinum toxin is present as an isolated protein; in some embodiments, botulinum toxin is present as part of a protein complex.

### Definitions

*Abrasion:* The term "abrasion," as used herein, refers to any means of altering, disrupting, removing, or destroying the top layer of the skin. In some embodiments, abrasion refers to a mechanical means of altering, disrupting, removing, or destroying the top layer of the skin. In some embodiments, abrasion refers to a chemical means of altering, disrupting, removing, or destroying the top layer of skin. To give but a few examples, agents such as exfoliants, fine particles (*e.g.,* magnesium or aluminum particles), acids (*e.g.,* alpha-hydroxy acids or beta-hydroxy acids), and/or alcohols may cause abrasion. In general, permeation enhancers such as those described, for example, by Donovan (see, *e.g.,* U.S. Patent Publications 2004/009180 and 2005/175636; and PCT Publication WO 04/06954;), and Graham (see, *e.g.,* U.S. Patent 6,939,852 and U.S. Patent Publication 2006/093624;), *etc.,* are expected to cause abrasion. Of course, those of ordinary skill in the art will appreciate that a particular agent may cause abrasion when present at one concentration, or in association with one or more other agents, but may not cause abrasion under different circumstances. Thus, whether or not a particular material is an "abrasive agent" depends on context. Abrasion can readily be assessed by those of ordinary skill in the art, for example by observation of redness or irritation of the skin and/or histologic examination of skin showing alteration, disruption, removal, or erosion of the stratum corneum.

*Administration:* The term "administration," as used herein to refers to the delivery of a nanoparticle composition to a subject, is not limited to any particular route but rather refers to any route accepted as appropriate by the medical community. For example, the present invention contemplates routes of delivering or administering that include, but are not limited to, transdermal.

*Amino acid:* As used herein, term "amino acid," in its broadest sense, refers to any compound and/or substance that can be incorporated into a polypeptide chain. In some embodiments, an amino acid has the general structure H₂N-C(H)(R)-COOH. In some embodiments, an amino acid is a naturally-occurring amino acid. In some embodiments, an amino acid is a synthetic amino acid; in some embodiments, an amino acid is a D-amino acid; in some embodiments, an amino acid is an L-amino acid. "Standard amino acid" refers to any of the twenty standard L-amino acids commonly found in naturally occurring peptides. "Nonstandard amino acid" refers to any amino acid, other than the standard amino acids, regardless of whether it is prepared synthetically or obtained from a natural source. Amino acids, including carboxy- and/or amino-terminal amino acids in peptides, can be modified by methylation, amidation, acetylation, and/or substitution with other chemical groups that can change the peptide's circulating half-life without adversely affecting their activity. Amino acids may participate in a disulfide bond. The term "amino acid" is used interchangeably with "amino acid residue," and may refer to a free amino acid and/or to an amino acid residue of a peptide. It will be apparent from the context in which the term is used whether it refers to a free amino acid or a residue of a peptide.

*Animal:* As used herein, the term "animal" refers to any member of the animal kingdom. In some embodiments, "animal" refers to humans, at any stage of development. In some embodiments, "animal" refers to non-human animals, at any stage of development. In certain embodiments, the non-human animal is a mammal (*e.g.,* a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, and/or worms. In some embodiments, an animal may be a transgenic animal, genetically-engineered animal, and/or a clone.

*Approximately:* As used herein, the terms "approximately" or "about" in reference to a number are generally taken to include numbers that fall within a range of 5%, 10%, 15%, or 20% in either direction (greater than or less than) of the number unless otherwise stated or otherwise evident from the context (except where such number would be less than 0% or exceed 100% of a possible value).

*Biologically active agent:* As used herein, the phrase "biologically active agent" refers to any substance that has activity in a biological system and/or organism. For instance, a substance that, when administered to an organism, has a biological effect on that organism is considered to be biologically active. In particular embodiments, where a polypeptide (*e.g.,* botulinum toxin) is biologically active, a portion of that polypeptide that shares at least one biological activity of the whole polypeptide is typically referred to as a "biologically active" portion.

*Botulinum nanoparticle composition:* The term "botulinum nanoparticle composition," as used herein, refers to any nanoparticle composition in which at least one nanoparticle includes botulinum toxin. The botulinum toxin may be present within the nanoparticle, on the nanoparticle surface and/or within a micellar membrane defining the nanoparticle.

*Botulinum toxin:* The term "botulinum toxin," as used herein, refers to any neurotoxin produced by *Clostridium botulinum.* Except as otherwise indicated, the term encompasses fragments or portions (*e.g.,* the light chain and/or the heavy chain) of such neurotoxin that retain appropriate activity (*e.g.,* muscle relaxant activity). The phrase "botulinum toxin," as used herein, encompasses the botulinum toxin serotypes A, B, C, D, E, F, and G; mutants thereof; variants thereof; fragments thereof; characteristic portions thereof; and/or fusions thereof. Botulinum toxin, as used herein, also encompasses both a botulinum toxin complex (*i.e.,* for example, the 300, 600, and 900 kD complexes) as well as the purified (*i.e.,* for example, isolated) botulinum toxin (*i.e.,* for example, about 150 kD). "Purified botulinum toxin" is defined as a botulinum toxin that is isolated, or substantially isolated, from other proteins, including proteins that form a botulinum toxin complex. A purified toxin may be greater than 80% pure, greater than 85% pure, greater than 90 % pure, greater than 95% pure, greater than 98% pure, and/or greater than 99% pure. Those of ordinary skill in the art will appreciate that the present invention is not limited to any particular source of botulinum toxin. For example, botulinum toxin for use in accordance with the present invention may be isolated from *Clostridium botulinum,* may be chemically synthesized, may be produced recombinantly (*i.e.,* in a host cell or organism other than *Clostridium botulinum*), *etc.*

*Cosmetic formulation:* The term "cosmetic formulation" is used herein to refer to a topically applied composition that contains one or more agents having cosmetic properties. To give but a few examples, a cosmetic formulation may be a skin softener, nutrition lotion type emulsion, cleansing lotion, cleansing cream, skin milk, emollient lotion, massage cream, emollient cream, make-up base, lipstick, facial pack or facial gel, cleaner formulation such as shampoos, rinses, body cleanser, hair-tonics, or soaps, and/or a dermatological composition such as a lotion, ointment, gel, cream, patch, deodorant, and/or spray.

*Cream:* The term "cream" refers to a spreadable composition, typically formulated for application to the skin. Creams typically contain an oil and/or fatty acid based-matrix. Creams formulated according to the present invention may contain nanoparticles and may be capable of substantially complete penetration (*e.g.,* of such nanoparticles) through the skin upon topical administration. Such a cream could also act as a carrier for incorporated materials (*e.g.,* for example, for one or more therapeutic agents).

*Dispersion medium:* The term "dispersion medium" as used herein, refers to a liquid medium in which particles (*e.g.,* nanoparticles) are dispersed. In general, a dispersion is formed when at least two immiscible materials are combined. An "oil-in-water" dispersion is one in which oily particles are dispersed within an aqueous dispersion medium. A "water-in-oil" dispersion is one in which aqueous particles are dispersed within an oily dispersion medium. Those of ordinary skill in the art will appreciate that a dispersion can be formed from any two immiscible media and is not limited strictly to combinations of aqueous and oily media. The term "dispersion medium" therefore applies broadly to *any* dispersion medium notwithstanding that it is common to refer to "aqueous" and "oily" categories.

*Encapsulated:* The term "encapsulated" (also "encapsulate" or "encapsulating") is used herein to mean that the encapsulated entity is completely surrounded by another material. To give but one example, a biologically active agent (*e.g.,* botulinum toxin) may be encapsulated within a nanoparticle in an emulsion in accordance with the invention. Such encapsulation may be achieved, for example, during formation of a nanoparticle composition (*e.g.,* a nanoemulsion), for example during micro fluidization.

*In conjunction with:* As used herein, the phrase "delivered in conjunction with" refers to the co-delivery of two or more substances or agents. In particular, according to the present invention, the phrase is used herein in reference to delivery of a biologically active agent with nanoparticles and/or nanoparticle compositions in accordance with the invention. A substance or agent is delivered in conjunction with nanoparticles when the substance or agent is combined with nanoparticles and/or nanoparticle compositions; is encapsulated or completely surrounded by nanoparticles; is embedded within a nanoparticle micellar membrane; and/or is associated with the outer surface of a nanoparticle micellar membrane. A substance or agent to be delivered in conjunction with nanoparticles and/or nanoparticle compositions may or may not be covalently linked to the nanoparticles and/or nanoparticle compositions. A substance or agent to be delivered in conjunction with nanoparticles and/or nanoparticle compositions may or may not be attached to the nanoparticles and/or nanoparticle compositions by adsorption forces.

*Isolated:* As used herein, the term "isolated" refers to a substance and/or entity that has been (1) separated from at least some of the components with which it was associated when initially produced (whether in nature and/or in an experimental setting), and/or (2) produced, prepared, and/or manufactured by the hand of man. Isolated substances and/or entities may be separated from at least about 10%, about 20%, about 30%, about 40%, about 50%, about 60%, about 70%, about 80%, about 90%, or more of the other components with which they were initially associated. In some embodiments, isolated substances and/or entities are more than 90%, 91%, 92%, 93%, 94%, 95%, 96%, 97%, 98%, or 99% pure.

*Microfluidized:* As used herein, the term "microfluidized" means exposed to high shear forces. In some embodiments, such exposure to high shear forces is accomplished by exposure to high pressure; in some embodiments such high pressure is within the range of about 15,000 psi to about 26,000 psi. In some embodiments, such exposure to high shear forces is accomplished by cavitation. In some embodiments, such exposure to high shear forces is accomplished by passing a sample through an instrument such as, for example, a Microfluidizer® (Microfluidics Corporation/MFIC Corporation) or other like device that may be useful in creating a uniform nanoparticle composition. In some embodiments, a sample is microfluidized through exposure to high shear forces for a period of time less than about 10 minutes. In some embodiments, the period of time is less than about 9, about 8, about 7, about 6, about 5, about 4, about 3, about 2, or about 1 minute(s). In some embodiments, the period of time is within the range of about 1 - about 2 minutes. In some embodiments, the period of time is about 30 seconds. In some embodiments, a sample is "microfluidized" through a single exposure to high shear forces; such embodiments are referred to as "single pass" microfluidization.

*Nanoemulsion:* An emulsion is traditionally defined in the art "as a system ... consisting of a liquid dispersed with or without an emulsifier in an immiscible liquid usually in droplets of larger than colloidal size" Medline Plus Online Medical Dictionary, Merriam Webster (2005). The term "nanoemulsion," as used herein, refers to an emulsion in which at least some of the droplets (or particles) have diameters in the nanometer size range. As will be understood by those of ordinary skill in the art, a nanoemulsion is characterized by droplets or particles one thousand fold smaller than microemulsion droplets or particles.

*Nanoparticle:* As used herein, the term "nanoparticle" refers to any particle having a diameter of less than 1000 nanometers (nm). In some embodiments, a nanoparticle has a diameter of less than 300 nm, as defined by the National Science Foundation. In some embodiments, a nanoparticle has a diameter of less than 100 nm as defined by the National Institutes of Health. In some embodiments, nanoparticles are micelles in that they comprise an enclosed compartment, separated from the bulk solution by a micellar membrane. A "micellar membrane" comprises amphiphilic entities which have aggregated to surround and enclose a space or compartment (*e.g.,* to define a lumen).

*Nanoparticle composition:* As used herein, the term "nanoparticle composition" refers to any substance that contains at least one nanoparticle. In some embodiments, a nanoparticle composition is a uniform collection of nanoparticles. Nanoparticle compositions can be dispersions or emulsions. In general, a dispersion or emulsion is formed when at least two immiscible materials are combined. An "oil-in-water" dispersion is one in which oily particles (or hydrophobic or non-polar) are dispersed within an aqueous dispersion medium. A "water-in-oil" dispersion is one in which aqueous (or hydrophilic or polar) particles are dispersed within an oily dispersion medium. Those of ordinary skill in the art will appreciate that a dispersion can be formed from any two immiscible media and is not limited strictly to combinations of aqueous and oily media. The term "dispersion medium" therefore applies broadly to *any* dispersion medium notwithstanding that it is common to refer to "aqueous" and "oily" categories. The nanoparticle compositions of the present invention are nanoemulsions. Nanoparticle compositions can be micelles. In some particular embodiments, a nanoparticle composition comprises amphiphilic entity nanoparticles as described in co-pending PCT application serial number PCT/US07/86018, entitled "Amphiphilic Entity Nanoparticles" and filed on November 30, 2007. In some particular embodiments, a nanoparticle composition comprises a nanoemulsion as described in co-pending U.S. Patent Application U.S.S.N. 11/607,436, entitled "BOTULINUM NANOEMULSIONS," filed December 1, 2006. In some embodiments, a nanoparticle composition (*e.g.* a nanoemulsion) is stable. In some embodiments, a nanoparticle composition includes one or more biologically active agents to be delivered in conjunction with the nanoparticles.

*Not contaminated with:* The phrase "not contaminated with," when used herein to refer to a nanoparticle composition, is synonymous with "substantially free of' and describes a nanoparticle composition containing no more than about 50% of the recited material. For example, if a nanoparticle composition is said to be "substantially free of' particles whose diameter is outside of a stated range, then no more than about 50% of the particles in that composition have diameters outside of the range. In some embodiments, no more than 25% of the particles are outside of the range. In some embodiments, no more than 20%, 19%, 18%, 17%, 16%, 15%, 14%, 13%, 12%, 10%, 9%, 8%, 7%, 6%, 5%, 4%, 3%, 2%, 1%, 0.5% or less of the particles have diameters outside of the stated range.

*Nucleic acid:* As used herein, the term "nucleic acid," in its broadest sense, refers to any compound and/or substance that is or can be incorporated into an oligonucleotide chain. In some embodiments, a nucleic acid is a compound and/or substance that is or can be incorporated into an oligonucleotide chain via a phosphodiester linkage. In some embodiments, "nucleic acid" refers to individual nucleic acid residues (*e.g.,* nucleotides and/or nucleosides). In some embodiments, "nucleic acid" refers to an oligonucleotide chain comprising individual nucleic acid residues. As used herein, the terms "oligonucleotide" and "polynucleotide" can be used interchangeably. In some embodiments, "nucleic acid" encompasses RNA as well as single and/or double-stranded DNA and/or cDNA. Furthermore, the terms "nucleic acid," "DNA," "RNA," and/or similar terms include nucleic acid analogs, *e.g.,* analogs having other than a phosphodiester backbone. For example, the so-called "peptide nucleic acids," which are known in the art and have peptide bonds instead of phosphodiester bonds in the backbone, are considered within the scope of the present invention. The term "nucleotide sequence encoding an amino acid sequence" includes all nucleotide sequences that are degenerate versions of each other and/or encode the same amino acid sequence. Nucleotide sequences that encode proteins and/or RNA may include introns. Nucleic acids can be purified from natural sources, produced using recombinant expression systems and optionally purified, chemically synthesized, *etc.* Where appropriate, *e.g.,* in the case of chemically synthesized molecules, nucleic acids can comprise nucleoside analogs such as analogs having chemically modified bases or sugars, backbone modifications, *etc.* A nucleic acid sequence is presented in the 5' to 3' direction unless otherwise indicated. The term "nucleic acid segment" is used herein to refer to a nucleic acid sequence that is a portion of a longer nucleic acid sequence. In many embodiments, a nucleic acid segment comprises at least 3, 4, 5, 6, 7, 8, 9, 10, or more residues. In some embodiments, a nucleic acid is or comprises natural nucleosides (*e.g.,* adenosine, thymidine, guanosine, cytidine, uridine, deoxyadenosine, deoxythymidine, deoxyguanosine, and deoxycytidine); nucleoside analogs (*e.g.,* 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (*e.g.,* methylated bases); intercalated bases; modified sugars (*e.g.,* 2'-fluororibose, ribose, 2'-deoxyribose, arabinose, and hexose); and/or modified phosphate groups (*e.g.,* phosphorothioates and 5'-*N-*phosphoramidite linkages). In some embodiments, the present invention is specifically directed to "unmodified nucleic acids," meaning nucleic acids (*e.g.,* polynucleotides and residues, including nucleotides and/or nucleosides) that have not been chemically modified in order to facilitate or achieve delivery (*e.g.,* transdermal delivery).

*Patient:* As used herein, the term "patient" or "subject" refers to any organism to which a composition in accordance with the invention may be administered, *e.g.,* for experimental, diagnostic, prophylactic, cosmetic, and/or therapeutic purposes. Typical patients include animals (*e.g.,* mammals such as mice, rats, rabbits, non-human primates, and humans). In some embodiments, a patient is a human.

*Pharmaceutically acceptable:* The term "pharmaceutically acceptable" as used herein, refers to agents that, within the scope of sound medical judgment, are suitable for use in contact with the tissues of human beings and animals without excessive toxicity, irritation, allergic response, or other problem or complication, commensurate with a reasonable benefit/risk ratio.

*Premix:* As used herein, the term "premix" refers to any combination of components that is subsequently used to generate a nanoparticle composition according to the present invention. For example, a premix is any collection of ingredients that, when subjected to high shear forces, generates nanoparticles according to the present invention. In some embodiments, a premix contains two or more immiscible solvents. In some embodiments, a premix contains components that self-assemble into nanoparticles. In some embodiments, a premix contains components that self-assemble into micelles. In some embodiments, a premix contains one or more amphiphilic entities as described in co-pending PCT application serial number PCT/US07/86018, entitled "Amphiphilic Entity Nanoparticles" and filed on November 30, 2007. In some embodiments, a premix contains one or more therapeutic agents; in some embodiments, a premix contains at least one other biologically active agent. In some embodiments, a premix is agitated, mixed, and/or stirred; in some embodiments, a premix is agitated, mixed, and/or stirred prior to being subjected to high shear force. In some embodiments, a premix comprises at least one solubilized component (*i.e.,* at least one component that is in solution); in some such embodiments, the premix is subjected to high shear force after such solubilization is achieved.

*Pure:* As used herein, a substance and/or entity is "pure" if it is substantially free of other components. For example, a preparation that contains more than about 90% of a particular substance and/or entity is typically considered to be a pure preparation. In some embodiments, a substance and/or entity is at least 91%, 92%, 93%, 94%, 95%, 96%, 97%, 98%, or 99% pure.

*Refractory:* The term "refractory" as used herein, refers to any subject that does not respond with an expected clinical efficacy following the delivery of a biologically active agent or pharmaceutical composition as normally observed by practicing medical personnel.

*Self-administration:* The term "self-administration," as used herein, refers to the situation where a subject has the ability to administer a composition to him or herself without requiring medical supervision. In some embodiments, self-administration may be performed outside of a clinical setting. To give but one example, in some embodiments, a facial cosmetic cream may be administered by a subject in one's own home.

*Shear force:* As used herein, the term "shear force" refers to a force that is parallel or tangential to the face of a material, as opposed to a force that is perpendicular to the face of a material. In some embodiments, a composition is exposed to high shear forces in order to produce a uniform nanoparticle composition. Any method known in the art can be used to generate high shear forces. In some embodiments, cavitation is used to generate high shear forces. In some embodiments, high pressure homogenization is used to generate high shear forces. Alternatively or additionally, high shear force may be administered by exposure to high pressure, for example about 15,000 psi. In some embodiments, such high pressure is within the range of about 18,000 psi to about 26,000 psi; in some embodiments, it is within the range of about 20,000 psi to about 25,000 psi. In some embodiments, and to give but one example, a Microfluidizer® Processor (Microfluidics Corporation/MFIC Corporation) or other like device is used to generate high shear force. Microfluidizer® Processors provide high pressure and a resultant high shear rate by accelerating a composition through microchannels (typically having dimensions on the order of 75 microns) at a high velocity (typically in the range of 50 m/s - 300 m/s) for size reduction to the nanoscale range. As the fluid exits the microchannels it forms jets which collide with jets from opposing microchannels. In the channels the fluid experiences high shear (up to 10⁷ 1/s) which is orders of magnitude higher than that of conventional technologies. Jet collisions result in mixing at submicron levels. Therefore, in such devices, high shear and/or impact can achieve particle size reduction and mixing of multiphase. In some embodiments, a sample is exposed to high shear forces for a period of time less than about 10 minutes. In some embodiments, the period of time is less than about 9 minutes, about 8 minutes, about 7 minutes, about 6 minutes, about 5 minutes, about 4 minutes, about 3 minutes, about 2 minutes, or about 1 minute. In some embodiments, the period of time is within the range of about 1 minute to about 2 minutes; in some embodiments, the period of time is less than about 1 minute; in some embodiments, the period of time is about 30 seconds. In some embodiments, a sample is "microfluidized" through a single exposure to high shear forces; such embodiments are referred to herein as "single pass" microfluidization.

*Small Molecule:* In general, a "small molecule" is a molecule that is less than about 5 kilodaltons (kD) in size. In some embodiments, the small molecule is less than about 4 kD, 3 kD, about 2 kD, or about 1 kD. In some embodiments, the small molecule is less than about 800 daltons (D), about 600 D, about 500 D, about 400 D, about 300 D, about 200 D, or about 100 D. In some embodiments, a small molecule is less than about 2000 g/mol, less than about 1500 g/mol, less than about 1000 g/mol, less than about 800 g/mol, or less than about 500 g/mol. In some embodiments, small molecules are non-polymeric. In some embodiments, in accordance with the present invention, small molecules are not proteins, polypeptides, oligopeptides, peptides, polynucleotides, oligonucleotides, polysaccharides, glycoproteins, proteoglycans, *etc.*

*Stable:* The term "stable," when applied to nanoparticle compositions herein, means that the compositions maintain one or more aspects of their physical structure (*e.g.,* size range and/or distribution of particles) over a period of time. In some embodiments, a stable nanoparticle composition is one for which the average particle size, the maximum particle size, the range of particle sizes, and/or the distribution of particle sizes (*i.e.,* the percentage of particles above a designated size and/or outside a designated range of sizes) is maintained for a period of time. In some embodiments, the period of time is at least about one hour; in some embodiments the period of time is about 5 hours, about 10 hours, about one (1) day, about one (1) week, about two (2) weeks, about one (1) month, about two (2) months, about three (3) months, about four (4) months, about five (5) months, about six (6) months, about eight (8) months, about ten (10) months, about twelve (12) months, about twenty-four (24) months, about thirty-six (36) months, or longer. In some embodiments, the period of time is within the range of about one (1) day to about twenty-four (24) months, about two (2) weeks to about twelve (12) months, about two (2) months to about five (5) months, *etc.* For example, if a nanoparticle composition is subjected to prolonged storage, temperature changes, and/or pH changes and a majority of the nanoparticles in the composition maintains a diameter within a stated range (for example, between approximately 10 nm and approximately 120 nm), the nanoparticle composition is stable. For some such populations, a majority is more than about 50%, about 60%, about 70%, about 80%, about 90%, about 95%, about 96%, about 97%, about 98%, about 99%, about 99.5%, about 99.6%, about 99.7%, about 99.8%, about 99.9% or more. In some embodiments, where a nanoparticle composition comprises at least one biologically active agent, the nanoparticle composition is considered stable if the concentration of biologically active agent (*e.g.,* botulinum toxin) is maintained in the composition over the designated period of time under a designated set of conditions.

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Substantially free of:* A nanoparticle composition is said to be "substantially free of' particles whose diameter is outside of a stated range when no more than about 50% of the particles in that composition have diameters outside of the range. In some embodiments, no more than 25% of the particles are outside of the range. In some embodiments, no more than 20%, 19%, 18%, 17%, 16%, 15%, 14%, 13%, 12%, 10%, 9%, 8%, 7%, 6%, 5%, 4%, 3%, 2%, 1%, 0.5% or less of the particles have diameters outside of the stated range.

*Suffering from:* An individual who is "suffering from" a disease, disorder, or condition (*e.g.,* a condition associated with sweat glands or sebaceous glands, such as acne; hyperhidrosis; bromhidrosis; chromhidrosis; hair loss; psoriasis; actinic keratosis; dermal infection; eczematous dermatitis (*e.g.,* atopic dermatitis, *etc*.); excess sebum-producing disorder; Raynaud's phenomenon; lupus erthythematosus; hyperpigmentation disorder; hypopigmentation disorder; skin cancer; *etc*.) has been diagnosed with or exhibits symptoms of the disease, disorder, or condition.

*Symptoms are reduced:* According to the present invention, "symptoms are reduced" when one or more symptoms of a particular disease, disorder or condition is reduced in magnitude (*e.g.,* intensity) or frequency. For purposes of clarity, a delay in the onset of a particular symptom is considered one form of reducing the frequency of that symptom. To give but a few examples, where the condition in question is acne, symptoms of that condition are reduced when the size and/or severity of one or more blemishes in the selected area is reduced, and/or when the number of total blemishes is reduced (*e.g.,* on a subject's face, back, *etc*.). Where the condition in question is hyperhidrosis, symptoms are reduced when the subject produces less sweat. It is not intended that the present invention be limited only to cases where the symptoms are eliminated. The present invention specifically contemplates treatment such that one or more symptoms is/are reduced (and the condition of the subject is thereby "improved"), albeit not completely eliminated.

*Therapeutically effective amount:* As used herein, the term "therapeutically effective amount" means an amount that is sufficient, when administered to an individual suffering from or susceptible to a disease, disorder, and/or condition, to treat the disease, disorder, and/or condition. Those of ordinary skill in the art will appreciate that the term "therapeutically effective amount" does not in fact require successful treatment be achieved in a particular individual. Rather, a therapeutically effective amount may be that amount that provides a particular desired pharmacological response in a significant number of subjects when administered or delivered to patients in need of such treatment. It is specifically understood that particular subjects may, in fact, be "refractory" to a "therapeutically effective amount." To give but one example, a refractory subject may have a low bioavailability such that clinical efficacy is not obtainable. In some embodiments, reference to a therapeutically effective amount may be a reference to an amount as measured in one or more specific tissues.

*Therapeutic agent:* As used herein, the phrase "therapeutic agent" refers to any agent that has a therapeutic effect and/or elicits a desired biological and/or pharmacological effect, when administered to a subject.

*Toxic solvent:* As used herein, the term "toxic solvent" refers to any substance that may alter, disrupt, remove, or destroy an animal's tissue. As would be understood by one of ordinary skill in the art, an animal's tissue can include living cells, dead cells, extracellular matrix, cellular junctions, biological molecules, *etc.* To give but a few examples, toxic solvents include dimethyl sulfoxide, dimethyl acetimide, dimethyl formamide, chloroform, tetramethyl formamide, acetone, acetates, and alkanes.

*Treatment:* As used herein, the term "treatment" (also "treat" or "treating") refers to any administration of a biologically active agent that partially or completely alleviates, ameliorates, relives, inhibits, delays onset of, reduces severity of and/or reduces incidence of one or more symptoms or features of a particular disease, disorder, and/or condition. Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition.

*Uniform:* The term "uniform," when used herein in reference to a nanoparticle composition, refers to a nanoparticle composition in which the individual nanoparticles have a specified range of particle diameter sizes. For example, in some embodiments, a uniform nanoparticle composition is one in which the difference between the minimum diameter and maximum diameter does not exceed about 600 nm, about 550 nm, about 500 nm, about 450 nm, about 400 nm, about 350 nm, about 300 nm, about 250 nm, about 200 nm, about 150 nm, about 100 nm, about 90 nm, about 80 nm, about 70 nm, about 60 nm, about 50 nm, or fewer nm. In some embodiments, particles (*e.g.,* botulinum toxin-containing particles) within uniform nanoparticle compositions in accordance with the invention have diameters that are smaller than about 600 nm, about 550 nm, about 500 nm, about 450 nm, about 400 nm, about 350 nm, about 300 nm, about 250 nm, about 200 nm, about 150 nm, about 130 nm, about 120 nm, about 115 nm, about 110 nm, about 100 nm, about 90 nm, about 80 nm, or less. In some embodiments, particles (e.g., particles containing one or more therapeutic agents) within uniform nanoparticle compositions in accordance with the invention have diameters within the range of about 10 nm and about 600 nm. In some embodiments, particles within uniform nanoparticle compositions in accordance with the invention have diameters within the range of about 10 nm and about 300 nm, about 10 nm and about 200 nm, about 10 nm and about 150 nm, about 10 nm and about 130 nm, about 10 nm and about 120 nm, about 10 nm and about 115 nm, about 10 nm and about 110 nm, about 10 nm and about 100 nm, or about 10 nm and about 90 nm. In some embodiments, particles within nanoparticle compositions in accordance with the invention have an average particle size that is under about 300 nm, about 250 nm, about 200 nm, about 150 nm, about 130 nm, about 120 nm, about 115 nm, about 110 nm, about 100 nm, or about 90 nm. In some embodiments, the average particle size is within the range of about 10 nm and about 300 nm, about 50 nm and about 250 nm, about 60 nm and about 200 nm, about 65 nm and about 150 nm, about 70 nm and about 130 nm. In some embodiments, the average particle size is between about 80 nm and about 110 nm. In some embodiments, the average particle size is about 90 nm to about 100 nm. In some embodiments, a majority of the particles within uniform nanoparticle compositions in accordance with the invention have diameters below a specified size or within a specified range. In some embodiments, the majority is more than 50%, 60%, 70%, 75%, 80%, 85%, 90%, 95%, 96%, 97%, 98%, 99%, 99.5%, 99.6%, 99.7%, 99.8%, 99.9% or more of the particles in the composition. In some embodiments, a uniform nanoparticle composition is achieved by microfluidization of a sample. In some embodiments, a uniform nanoparticle composition is prepared by exposure to high shear force, *e.g.,* by microfluidization.

*Unwanted side effects:* As used herein, the term "unwanted side effects" refers to effects and/or symptoms associated with administration of a therapeutic agent to a patient that are not the desired and/or intended effect. Exemplary unwanted side effects include pain; bruising; ecchymosis; hematoma; botulism poisoning; unwanted systemic effects; undesirable blood levels of a substance (*e.g.,* the therapeutic agent, a metabolite of the therapeutic agent, *etc*.); damage to underlying nervous tissue (*e.g.,* neuronal paralysis); unwanted effects on muscles (*e.g.,* muscle paralysis); flu-like symptoms; morbidity; mortality; alteration in body weight; alteration in enzyme levels; pathological changes detected at the microscopic, macroscopic, and/or physiological levels; infection; hemorrhage; inflammation; scarring; loss of function; changes in local blood flow; fever; malaise; teratogenesis; pulmonary hypertension; stroke; heart disease; heart attack; neuropathy; nausea; vomiting; dizziness; diarrhea; headache; dermatitis; dry mouth; addiction; miscarriage; abortion; uterine hemhorrage; birth defects; bleeding; cardiovascular disease; deafness; kidney damage and/or failure; liver damage and/or failure; dementia; depression; diabetes; erectile dysfunction; glaucoma; hair loss; anaemia; insomnia; lactic acidosis; melasma; thrombosis; priapism; rhabdomyolysis; seizures; drowsiness; increase in appetite; decrease in appetite; increase in libido; decrease in libido; tardive dyskinesia; non-axillary sweating; injection site pain and hemorrhage; pharyngitis; neck pain; back pain; pruritus; anxiety; follicular obstruction; and/or combinations thereof. In some embodiments, topical administration of a therapeutic agent reduces unwanted side effects by about 50%, about 60%, about 70%, about 80%, about 90%, about 95%, about 98%, about 99%, or about 100% relative to non-topical administration (*e.g.,* injection, oral administration, *etc*.) of the same therapeutic agent.

### Brief Description of the Drawing

*Figure 1* shows one embodiment of a particle diameter distribution of a microfluidized botulinum toxin nanoemulsion.
*Figure 2* shows one embodiment of a particle diameter distribution of homogenized botulinum toxin microemulsion.
*Figure 3* shows a patient attempting maximal brow elevation prior to (Panel A) and two weeks after (Panel B) topical administration of a composition comprising a botulinum nanoparticle composition.
*Figure 4a* illustrates a subject prior to treatment with a botulinum nanoemulsion. Darkened skin areas and sweat demonstrate profuse sweating at rest.
*Figure 4b* illustrates a subject to weeks following treatment with a botulinum nanoemulsion, demonstrating a profound diminishment of sweating at rest as demonstrated by the minor punctuate areas of skin darkening.

### Description of Certain Preferred Embodiments

The present invention relates to the treatment of certain disorders or conditions associated with the dermal layer of the skin (*e.g.,* conditions associated with sebaceous glands such as excess sebum producing disorder (*e.g.* acne) or rosecea) through the transdermal application of nanoemulsion compositions comprising at least one therapeutic agent. In some embodiments, the present invention provides treatments for acne. In some embodiments, the present invention provides treatments for rosacea.

### Nanoparticle Compositions

As described herein, the present invention provides novel nanoparticle compositions (*e.g.* nanoemulsions) that contain at least one therapeutic agent (*e.g.,* botulinum toxin). The present invention provides novel uses for such nanoparticle compositions. In some embodiments, the invention provides use of nanoparticle compositions for treatment of disorders or conditions associated with the dermal layer of the skin, such as dermal gland disorders such as sebaceous glands (such as excess sebum producing disorders (*e.g.,* acne)). In some embodiments, the invention provides use of nanoparticle compositions for treatment of rosacea. In some embodiments, the present invention provides use of nanoparticle compositions for treatment of excess sebum-producing disorders (*e.g.,* seborrhea, seborrheic dermatitis, *etc*.).

In general, a nanoparticle composition is any composition that includes at least one nanoparticle. In some embodiments, nanoparticle compositions comprise at least one therapeutic agent (*e.g.,* botulinum toxin). A therapeutic agent may be encapsulated or completely surrounded by one or more nanoparticles; associated with the nanoparticle interface; and/or adsorbed to the outer surface of one or more nanoparticles. A therapeutic agent may or may not be covalently linked to the nanoparticles and/or nanoparticle compositions; a therapeutic agent may or may not be attached to nanoparticles and/or nanoparticle compositions by adsorption forces.

In some embodiments, nanoparticle compositions (*e.g.* nanoemulsions) in accordance with the invention are stable. In some embodiments, nanoparticle compositions in accordance with the invention are uniform. For example, in some embodiments, the difference between the minimum diameter and maximum diameter of the nanoparticles in a nanoparticle composition does not exceed approximately 600 nm, approximately 550 nm, approximately 500 nm, approximately 450 nm, approximately 400 nm, approximately 350 nm, approximately 300 nm, approximately 250 nm, approximately 200 nm, approximately 150 nm, or approximately 100 nm, approximately 90 nm, approximately 80 nm, approximately 70 nm, approximately 60 nm, approximately 50 nm, or fewer nm.

In some embodiments, particles within nanoparticle compositions (*e.g.* nanoemulsions) have diameters that are smaller than about 1000 nm, about 600 nm, about 550 nm, about 500 nm, about 450 nm, about 400 nm, about 350 nm, about 300 nm, about 250 nm, about 200 nm, about 150 nm, about 130 nm, about 120 nm, about 115 nm, about 110 nm, about 100 nm, about 90 nm, about 80 nm, about 50 nm, or less.

In some embodiments, particles within nanoparticle compositions (*e.g.* nanoemulsions) have diameters within the range of about 10 nm and about 600 nm. In some embodiments, particles within nanoparticle compositions have diameters within the range of about 10 nm to about 300 nm, about 10 nm to about 200 nm, about 10 nm to about 150 nm, about 10 nm to about 130 nm, about 10 nm to about 120 nm, about 10 nm to about 115 nm, about 10 nm to about 110 nm, about 10 nm to about 100 nm, or about 10 nm to about 90 nm. In some embodiments, particles within the nanoparticle compositions have diameters within the range of 1 nm to 1000 nm, 1 nm to 600 nm, 1 nm to 500 nm, 1 nm to 400 nm, 1 nm to 300 nm, 1 nm to 200 nm, 1 nm to 150 nm, 1 nm to 120 nm, 1 nm to 100 nm, 1 nm to 75 nm, 1 nm to 50 nm, or 1 nm to 25 nm. In some embodiments, particles within the nanoparticle compositions have diameters of 1 nm to 15 nm, 15 nm to 200 nm, 25 nm to 200 nm, 50 nm to 200 nm, or 75 nm to 200 nm.

In some embodiments, the total particle distribution is encompassed within the specified range of particle diameter size. In some embodiments, less than 50%, 25%, 10%, 5%, or 1% of the total particle distribution is outside of the specified range of particle diameter sizes. In some embodiments, less than 1% of the total particle distribution is outside of the specified range of particle diameter sizes. In certain embodiments, the nanoparticle composition is substantially free of particles having a diameter larger than 300 nm, 250 nm, 200 nm, 150 nm, 120 nm, 100 nm, 75 nm, 50 nm, or 25 nm.

In some embodiments, particles within nanoparticle compositions (*e.g.* nanoemulsions) have an average particle size that is under about 300 nm, about 250 nm, about 200 nm, about 150 nm, about 130 nm, about 120 nm, about 115 nm, about 110 nm, about 100 nm, about 90 nm, or about 50 nm. In some embodiments, the average particle size is within the range of about 10 nm and about 300 nm, about 50 nm and about 250, about 60 nm and about 200 nm, about 65 nm and about 150 nm, or about 70 nm and about 130 nm. In some embodiments, the average particle size is about 80 nm and about 110 nm. In some embodiments, the average particle size is about 90 nm and about 100 nm.

In some embodiments, a majority of the particles within nanoparticle compositions (*e.g.* nanoemulsions) have diameters below a specified size or within a specified range. In some embodiments, the majority is more than 50%, 60%, 70%, 75%, 80%, 85%, 90%, 95%, 96%, 97%, 98%, 99%, 99.5%, 99.6%, 99.7%, 99.8%, 99.9% or more of the particles in the composition.

In some embodiments, nanoparticle compositions (*e.g.* nanoemulsions) are substantially free of particles having a diameter in excess of 300 nm. Specifically, in some embodiments, fewer than 50%, of the nanoparticles in nanoparticle compositions have a diameter in excess of 120 nm. In some embodiments, fewer than 25% of the particles have a diameter in excess of 120 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 120 nm. Furthermore, in some embodiments, the nanoparticles in nanoparticle compositions have diameters within the range of 10 nm and 300 nm.

In some embodiments, nanoparticle compositions (*e.g.* nanoemulsions) are substantially free of particles having a diameter in excess of 200 nm. Specifically, in some embodiments, fewer than 50%, of the nanoparticles in nanoparticle compositions have a diameter in excess of 120 nm. In some embodiments, fewer than 25% of the particles have a diameter in excess of 120 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 120 nm. Furthermore, in some embodiments, the nanoparticles in nanoparticle compositions have diameters within the range of 10 nm and 200 nm.

In some embodiments, nanoparticle compositions (*e.g.* nanoemulsions) are substantially free of particles having a diameter in excess of 120 nm. Specifically, in some embodiments, fewer than 50%, of the nanoparticles in nanoparticle compositions have a diameter in excess of 120 nm. In some embodiments, fewer than 25% of the particles have a diameter in excess of 120 nm. In some embodiments, fewer than 20%, 19%, 18%, 17%, 16%, 15%, 14%, 13%, 12%, 10%, 9%, 8%, 7%, 6%, 5%, 4%, 3%, 2%, 1%, 0.5% or less of the particles have a diameter in excess of 120 nm. Furthermore, in some embodiments, the nanoparticles in nanoparticle compositions have diameters within the range of 10 nm and 120 nm.

In some embodiments, a majority of nanoparticles in a nanoparticle composition (*e.g.* nanoemulsion) have diameters between 10 nm and 120 nm. In some embodiments, a majority of nanoparticles in a nanoparticle composition have diameters between 20 nm and 120 nm. In some embodiments, a majority of nanoparticles in a nanoparticle composition have diameters between 20 nm and 110 nm. In some embodiments, a majority of nanoparticles in a nanoparticle composition have diameters between 20 nm and 100 nm. In some embodiments, a majority of nanoparticles in a nanoparticle composition have diameters between 20 nm and 90 nm. In some embodiments, a majority of nanoparticles in a nanoparticle composition have diameters between 20 nm and 80 nm. In some embodiments, a majority of nanoparticles in a nanoparticle composition have diameters between 20 nm and 70 nm. In some embodiments, a majority of nanoparticles in a nanoparticle composition have diameters between 20 nm and 60 nm. In some embodiments, a majority of nanoparticles in a nanoparticle composition have diameters between 20 nm and 50 nm. In some embodiments, a majority of nanoparticles in a nanoparticle composition have diameters between 20 nm and 40 nm. In some embodiments, a majority of nanoparticles in a nanoparticle composition have diameters between 20 nm and 30 nm.

In certain embodiments, about 50% of nanoparticles in a nanoparticle composition (*e.g.* nanoemulsion) have diameters between 10 nm and 40 nm. In certain embodiments, about 90% of nanoparticles in a nanoparticle composition have diameters between 10 nm and 80 nm. In certain embodiments, about 90% of nanoparticles in a nanoparticle composition have diameters between 10 nm and 90 nm. In certain embodiments, about 95% of nanoparticles in a nanoparticle composition have diameters between 10 nm and 110 nm. In certain embodiments, about 95% of nanoparticles in a nanoparticle composition have diameters between 10 nm and 120 nm.

In certain embodiments, about 50% of the aggregate volume of all nanoparticles in a nanoparticle composition (*e.g.* nanoemulsion) comprises or consists of nanoparticles having diameters between 10 nm and 40 nm. In certain embodiments, about 90% of the aggregate volume of all nanoparticles in a nanoparticle composition comprises or consists of nanoparticles having diameters between 10 nm and 80 nm. In certain embodiments, about 95% of the aggregate volume of all nanoparticles in a nanoparticle composition comprises or consists of nanoparticles having diameters between 10 nm and 110 nm. In certain embodiments, about 95% of the aggregate volume of all nanoparticles in a nanoparticle composition comprises or consists of nanoparticles having diameters between 10 nm and 120 nm.

Zeta potential is a measurement of the electric potential at a shear plane. A shear plane is an imaginary surface separating a thin layer of liquid bound to a solid surface (*e.g.,* nanoparticle surface) and showing elastic behavior from the rest of liquid (*e.g.,* liquid dispersion medium) showing normal viscous behavior. In some embodiments, nanoparticles have a zeta potential ranging between -80 mV and +80 mV. In some embodiments, nanoparticles have a zeta potential ranging between -50 mV and +50 mV. In some embodiments, nanoparticles have a zeta potential ranging between -25 mV and +25 mV. In some embodiments, nanoparticles have a zeta potential ranging betwee n -10 mV and +10 mV. In some embodiments, nanoparticles have a zeta potential of about -80 mV, about -70 mV, about -60 mV, about 50 mV, about -40 mV, about -30 mV, about -25 mV, about -20 mV, about -15 mV, about -10 mV, or about -5 mV. In some embodiments, nanoparticles have a zeta potential of about +50 mV, about +40 mV, about +30 mV, about +25 mV, about +20 mV, about +15 mV, about +10 mV, or about +5 mV. In some embodiments, nanoparticles have a zeta potential that is about 0 mV.

In some embodiments, nanoparticles have a zeta potential that is about -5 mV to about -80 mV. In some embodiments, nanoparticles have a zeta potential that is about -5 mV to about -70 mV. In some embodiments, nanoparticles have a zeta potential that is about -5 mV to about -60 mV. In some embodiments, nanoparticles have a zeta potential that is about -5 mV to about -50 mV. In some embodiments, nanoparticles have a zeta potential that is about -5 mV to about -40 mV. In some embodiments, nanoparticles have a zeta potential that is about -5 mV to about -30 mV. In some embodiments, nanoparticles have a zeta potential that is about -5 mV to about -20 mV.

In some embodiments, nanoparticles have a zeta potential that is about -10 mV to about -15 mV. In some embodiments, nanoparticles have a zeta potential that is about -10 mV to about -80 mV. In some embodiments, nanoparticles have a zeta potential that is about -10 mV to about -70 mV. In some embodiments, nanoparticles have a zeta potential that is about -10 mV to about -60 mV. In some embodiments, nanoparticles have a zeta potential that is about -10 mV to about -50 mV. In some embodiments, nanoparticles have a zeta potential that is about -10 mV to about -40 mV. In some embodiments, nanoparticles have a zeta potential that is about -10 mV to about -30 mV. In some embodiments, nanoparticles have a zeta potential that is about -10 mV to about -20 mV.

In some embodiments, nanoparticles have a zeta potential that is about -80 mV to about -70 mV. In some embodiments, nanoparticles have a zeta potential that is about -70 mV to about -60 mV. In some embodiments, nanoparticles have a zeta potential that is about -60 mV to about -50 mV. In some embodiments, nanoparticles have a zeta potential that is about -50 mV to about -40 mV. In some embodiments, nanoparticles have a zeta potential that is about -40 mV to about -30 mV. In some embodiments, nanoparticles have a zeta potential that is about -30 mV to about -20 mV. In some embodiments, nanoparticles have a zeta potential that is about -20 mV to about -10 mV. In some embodiments, nanoparticles have a zeta potential that is about -10 mV to about 0 mV.

In some embodiments, nanoparticles have a zeta potential that is about -15 mV to about -20 mV. In some embodiments, nanoparticles have a zeta potential that is about -5 mV, about -6 mV, about -7 mV, about -8 mV, about -9 mV, -10 mV, about -11 mV, about - 12 mV, about -13 mV, about -14 mV, about -15 mV, about 16 mV, about -17 mV, about -18 mV, about -19 mV, or about -20 mV.

Nanoparticle compositions are typically emulsions or dispersions. In some embodiments, the compositions are "oil-in-water" dispersions (*i.e.,* dispersions in which oily particles are dispersed within an aqueous dispersion medium); in some embodiments, the compositions are "water-in-oil" dispersions (*i.e.,* dispersions in which aqueous particles are dispersed within an oily dispersion medium).

In some embodiments, nanoparticle compositions do not require toxic solvents. By contrast, many conventional strategies for inducing formation of nanoparticles in a composition utilize toxic (typically organic) solvents. In some embodiments, nanoparticle compositions do not require polymers. By contrast, many conventional strategies for preparing compositions that contain nanoparticle structures require polymers.

In some embodiments, nanoparticle compositions have better tissue absorption and/or better biocompatibility than other nanoparticle compositions. For example, in some embodiments, nanoparticle compositions have better tissue absorption and/or better biocompatibility than nanoparticle compositions that are not uniform, that utilize one or more toxic (*e.g.,* organic) solvents, and/or that utilize one or more polymers.

In some embodiments, nanoparticle compositions are stable. In some embodiments, a stable nanoparticle composition is one for which the average particle size, the maximum particle size, the range of particle sizes, and/or the distribution of particle sizes (*i.e.,* the percentage of particles above a designated size and/or outside a designated range of sizes) is maintained for a period of time. In some embodiments, the period of time is at least about one hour; in some embodiments the period of time is about 5 hours, about 10 hours, about one (1) day, about one (1) week, about two (2) weeks, about one (1) month, about two (2) months, about three (3) months, about four (4) months, about five (5) months, about six (6) months, about eight (8) months, about ten (10) months, about twelve (12) months, about twenty-four (24) months, or longer. In some embodiments, the period of time is within the range of about one (1) day to about twenty-four (24) months, about two (2) weeks to about twelve (12) months, about two (2) months to about five (5) months, *etc..* For example, if a population of nanoemulsion particles is subjected to prolonged storage, temperature changes, and/or pH changes and a majority of the nanoparticles in the population maintain a diameter within a stated range (*i.e.,* for example, between approximately 10 nm and about 120 nm), the nanoparticle composition is stable. For some such populations, a majority is more than about 50%, about 60%, about 70%, about 80%, about 90%, about 95%, about 96%, about 97%, about 98%, about 99%, about 99.5%, about 99.6%, about 99.7%, about 99.8%, about 99.9%, or more than about 99.9% pure. In some embodiments, where a nanoparticle composition comprises at least one biologically active agent, the nanoparticle composition is considered stable if the concentration of biologically active agent (*e.g.,* botulinum toxin) is maintained in the composition over the designated period of time under a designated set of conditions.

As described herein, nanoparticle compositions are useful in various cosmetic and/or medical applications. Such compositions may be delivered to a subject by transdermal delivery. In some embodiments, such compositions comprise botulinum toxin. It should be noted that botulinum nanoparticle compositions are readily distinguishable from other botulinum-toxin-containing compositions that have been described. For example, Donovan has described a preparation in which botulinum toxin has been incorporated into a lipid vesicle for transdermal delivery (U.S. Patent Publication 2004/0009180). Such vesicles also require the incorporation of an enhancing agent, such as an alcohol, to facilitate the absorption of botulinum toxin through the skin. Donovan also describes a neurotoxin that is incorporated into a transfersome, which are deformable carriers containing lipids and membrane softeners (Hofer et al., 2000, World J. Surg., 24:1187; and U.S. Patent 6,165,500). Donovan specifically describes the preparation of phosphatidyl choline + sodium cholate liposomes incorporating botulinum toxin.

Suvanprakorn *et al.* have also described suspensions of liposome-encapsulated materials in discrete macro-beads; one of the literally hundreds of compounds that is said to be amendable to encapsulation is "BOTOX®" (U.S. Patent Publication 2004/0224012). Included in contemplated methods of making these multi-lamellar vesicular liposomes are lyophilization/rehydration and organic solution dehydration/aqueous rehydration. These conventional methods of producing liposomes would be expected to produce microparticle-sized vesicles.

### Methods of Making Nanoparticle Compositions

In general, nanoparticle compositions may be prepared by any available method. In some embodiments, nanoparticle compositions are prepared by chemical means. However, chemical means often require toxic (typically organic) solvents; in some embodiments, nanoparticle compositions are prepared in accordance with the present invention without utilizing such solvents.

### High Shear Force

In some embodiments, nanoparticle compositions in accordance with the invention self-assemble from a collection of combined components. In some embodiments, nanoparticle compositions are prepared by subjecting a combination of components (*i.e.,* a "premix") to high shear force. As used herein, the term "shear force" refers to a force that is parallel or tangential to the face of a material, as opposed to a force that is perpendicular to the face of a material. In some embodiments, high shear force is applied by high pressure, by cavitation, by homogenization, and/or by microfluidization. In some embodiments, combined nanoparticle-forming components are agitated, stirred, or otherwise mixed. In some such embodiments, the components are subjected to high shear force after having been mixed. In some specific embodiments, mixing may be performed for a period of time such as, for example, about 1 minute, about 3 minutes, about 5 minutes, about 10 minutes, about 15 minutes, about 30 minutes, about 45 minutes, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, or about 15 hours. In some specific embodiments, mixing may be performed for a period of time such as, for example, more than 15 minutes, more than 30 minutes, more than 45 minutes, more than 1 hour, more than 2 hours, more than 3 hours, more than 4 hours, more than 5 hours, more than 6 hours, more than 7 hours, more than 8 hours, more than 9 hours, more than 10 hours, more than 11 hours, more than 12 hours, more than 13 hours, more than 14 hours, or more than 15 hours. In some specific embodiments, mixing may be performed for a period of time such as, for example, less than 15 minutes, less than 30 minutes, less than 45 minutes, less than 1 hour, less than 2 hours, less than 3 hours, less than 4 hours, less than 5 hours, less than 6 hours, less than 7 hours, less than 8 hours, less than 9 hours, less than 10 hours, less than 11 hours, less than 12 hours, less than 13 hours, less than 14 hours, or less than 15 hours. In some embodiments, solubilization is achieved.

Any method known in the art can be used to generate high shear forces. In some embodiments, cavitation is used to generate high shear forces. According to the present invention, the use of mechanical energy (*i.e.,* high shear forces) can replace or minimize any requirement to use costly and/or toxic chemical solvents; can increase the speed at which nanoparticles assemble, can increase the yield of nanoparticles generated in a particular mix of components, and/or can greatly reduce the overall cost of preparing nanoemulsion compositions. Furthermore, in those embodiments in which an agent such as a biologically active agent (*e.g.,* botulinum toxin) is incorporated into nanoparticle compositions, the use of high shear force can increase the loading capacity of the nanoparticle as compared to traditional methods of forming nanoparticles. In traditional methods, loading of agents within or on the surface of nanoparticles typically relies on diffusion of the agent to the interior and/or to the surface of the nanoparticle. According to the present invention, the use of high shear force can allow for the manufacture of smaller particles (*e.g.,* on average) and/or a more narrow distribution of particle sizes in a nanoparticle composition.

In some embodiments, high shear forces are achieved by exposure to high pressure, for example by continuous turbulent flow at high pressure, for example about 15,000 psi. In some embodiments, such high pressure is within the range of about 18,000 psi to about 26,000 psi; in some embodiments, it is within the range of about 20,000 psi to about 25,000 psi; in some embodiments, it is within the range of about 25,000 psi to about 30,000 psi; in some embodiments, it is within the range of about 30,000 psi to about 35,000 psi; in some embodiments, it is within the range of about 30,000 psi to about 40,000 psi; in some embodiments, it is within the range of about 40,000 psi to about 50,000 psi.

In some embodiments, high shear force or high pressure may be administered by cavitation or high pressure homogenization.

In some embodiments, high shear force may be administered by passage through an instrument such as, for example, a Microfluidizer® Processor (Microfluidics Corporation/MFIC Corporation) or other like device. Microfluidizer® Processors provide high pressure and a resultant high shear rate by accelerating the product through microchannels to a high velocity for size reduction to the nanoscale range. The fluid is split in two and is pushed through microchannels with typical dimensions in the order of 75 microns at high velocities (in the range of 50 m/s to 300 m/s). As the fluid exits the microchannels it forms jets which collide with jets from opposing microchannels. In the channels the fluid experiences high shear (up to 10⁷ 1/s) which is orders of magnitude higher than that of conventional technologies. Jet collisions result in mixing in submicron level. Therefore, high shear and impact are responsible for particle size reduction and mixing of multiphase fluids in the Microfluidizer® technology.

More generally, a microfluidizer may be any device that powers a single acting intensifier pump. The intensifier pump amplifies the hydraulic pressure to a selected level which, in turn, imparts that pressure to the product stream. As the pump travels through its pressure stroke, it drives the product at constant pressure through the interaction chamber. Within the interaction chamber are specially designed fixed-geometry microchannels through which the product stream will accelerate to high velocities, creating high shear and impact forces that can generate a uniform nanoparticle composition (*e.g.,* nanoemulsion) as the high velocity product stream impinges on itself and on wear-resistant surfaces.

As the intensifier pump completes its pressure stroke, it reverses direction and draws in a new volume of product. At the end of the intake stroke, it again reverses direction and drives the product at constant pressures, thereby repeating the process.

Upon exiting the interaction chamber, the product flows through an onboard heat exchanger which regulates the product to a desired temperature. At this point, the product may be recirculated through the system for further processing or directed externally to the next step in the process (U.S. Patents 4,533,254; and 4,908,154).

In some embodiments, a sample is "microfluidized" through exposure to high shear forces for a period of time less than about 10 minutes. In some embodiments, the period of time is less than about 9, about 8, about 7, about 6, about 5, about 4, about 3, about 2, or about 1 minute(s). In some embodiments, the period of time is within the range of about 1 to about 2 minutes or less; in some embodiments, the period of time is about 30 seconds.

In some embodiments, a sample is "microfluidized" through a single exposure to high shear forces; such embodiments are referred to herein as "single pass" microfluidization.

### Premix Composition

The present invention encompasses the recognition that subjecting a premix to high shear forces can generate a nanoparticle composition (*e.g.* a nanoemulsion), and in particular can generate a uniform nanoparticle composition.

In general, the premix from which nanoparticle compositions are prepared through the application of high shear force is expected to contain at least two immiscible materials, one of which will constitute the dispersion medium (*i.e.,* the liquid medium in which particles (*e.g.,* nanoparticles) are dispersed in the ultimate nanoparticle composition). An "oil-in-water" dispersion is one in which oily particles are dispersed within an aqueous dispersion medium. A "water-in-oil" dispersion is one in which aqueous particles are dispersed within an oily dispersion medium. Those of ordinary skill in the art will appreciate that a dispersion can be formed from any two immiscible media and is not limited strictly to combinations of aqueous and oily media. The term "dispersion medium" therefore applies broadly to *any* dispersion medium notwithstanding that it is common to refer to "aqueous" and "oily" categories.

Thus, in some embodiments, a premix will contain an aqueous dispersion medium and an oily medium that becomes dispersed in nanoparticle form in the dispersion medium; in some embodiments, a premix contains an oily dispersion medium and an aqueous medium that becomes dispersed in nanoparticle form in the oily dispersion medium.

Those of ordinary skill in the art will be well aware of suitable aqueous media that can be used as dispersion media or as media to be dispersed in accordance with the present invention. Representative such aqueous media include, for example, water, saline solutions (including phosphate buffered saline), water for injection, short chain alcohols, 5% dextrose, Ringer's solutions (lactated Ringer's injection, lactated Ringer's plus 5% dextrose injection, acylated Ringer's injection), Normosol-M, Isolyte E, and the like, and combinations thereof.

Those of ordinary skill in the art will also be well aware of suitable oily media that can be used as dispersion media or as media to be dispersed in accordance with the present invention. In some embodiments, the oil may comprise one or more fatty acid groups or salts thereof. In some embodiments, the fatty acid group may comprise digestible, long chain (*e.g.,* C₈₋C₅₀), substituted or unsubstituted hydrocarbons. In some embodiments, the fatty acid group may be a C₁₀-C₂₀ fatty acid or salt thereof. In some embodiments, the fatty acid group may be a C₁₅-C₂₀ fatty acid or salt thereof. In some embodiments, the fatty acid group may be a C₁₅-C₂₅ fatty acid or salt thereof. In some embodiments, the fatty acid group may be a medium chain triglyceride. In some embodiments, the fatty acid group may be unsaturated. In some embodiments, the fatty acid group may be monounsaturated. In some embodiments, the fatty acid group may be polyunsaturated. In some embodiments, a double bond of an unsaturated fatty acid group may be in the *cis* conformation. In some embodiments, a double bond of an unsaturated fatty acid may be in the *trans* conformation.

In some embodiments, the fatty acid group may be one or more of butyric, caproic, caprylic, capric, lauric, myristic, palmitic, stearic, arachidic, behenic, or lignoceric acid. In some embodiments, the fatty acid group may be one or more of palmitoleic, oleic, vaccenic, linoleic, alpha-linolenic, gamma-linoleic, arachidonic, gadoleic, arachidonic, eicosapentaenoic, docosahexaenoic, or erucic acid.

In some embodiments, the oil is a liquid triglyceride. In some embodiments, the oil is a medium chain triglyceride. In general, medium chain triglycerides are fatty acids containing 6-12 carbons atoms (*e.g.,* caprylic acid, octanoic acid, capric acid, decanoic acid, lauric acid, *etc*.) and may be obtained from coconut oil or palm kernel oil. In some embodiments 1349 oil is a medium-chain triglyceride that can be utilized in accordance with the invention.

Representative such oily media include, for example, saturated and unsaturated almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, jojoba, kukui nut, lavandin, lavender, lemon, litsea cubeba, macadamia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils; butyl stearate; caprylic triglyceride; capric triglyceride; cyclomethicone; diethyl sebacate; dimethicone 360; isopropyl myristate; mineral oil; octyldodecanol; oleyl alcohol; silicone oil; medium-chain triglyceride oils; 1349 oil; and combinations thereof.

In addition to the two immiscible media, a premix according to the present invention may include, for example, one or more surfactants or emulsifying agents. Suitable such surfactants or emulsifying agents include, but are not limited to, phosphoglycerides; phosphatidylcholines; dipalmitoyl phosphatidylcholine (DPPC); dioleylphosphatidyl ethanolamine (DOPE); dioleyloxypropyltriethylammonium (DOTMA); dioleoylphosphatidylcholine; cholesterol; cholesterol ester; diacylglycerol; diacylglycerolsuccinate; diphosphatidyl glycerol (DPPG); hexanedecanol; fatty alcohols such as polyethylene glycol (PEG); polyoxyethylene-9-lauryl ether; a surface active fatty acid, such as palmitic acid or oleic acid; fatty acids; fatty acid monoglycerides; fatty acid diglycerides; fatty acid amides; sorbitan trioleate (SPAN®85) glycocholate; sorbitan monolaurate (SPAN®20); polysorbate 20 (TWEEN®20); polysorbate 60 (TWEEN®60); polysorbate 65 (TWEEN®65); polysorbate 80 (TWEEN®80); polysorbate 85 (TWEEN®85); super-refined polysorbate 20 (SR TWEEN®20); super-refined polysorbate 60 (SR TWEEN®60); super-refined polysorbate 65 (SR TWEEN®65); super-refined polysorbate 80 (SR TWEEN®80); super-refined polysorbate 85 (SR TWEEN®85); polyoxyethylene monostearate; surfactin; a poloxomer; a sorbitan fatty acid ester such as sorbitan trioleate; lecithin; lysolecithin; phosphatidylserine; phosphatidylinositol; sphingomyelin; phosphatidylethanolamine (cephalin); cardiolipin; phosphatidic acid; cerebrosides; dicetylphosphate; dipalmitoylphosphatidylglycerol; stearylamine; dodecylamine; hexadecyl-amine; acetyl palmitate; glycerol ricinoleate; hexadecyl stearate; isopropyl myristate; tyloxapol; poly(ethylene glycol)5000-phosphatidylethanolamine; poly(ethylene glycol)400-monostearate; phospholipids; synthetic and/or natural detergents having high surfactant properties; deoxycholates; cyclodextrins; chaotropic salts; ion pairing agents; and combinations thereof. The surfactant component may be a mixture of different surfactants. These surfactants may be extracted and purified from a natural source or may be prepared synthetically in a laboratory. In some embodiments, the surfactants are commercially available.

In some embodiments, all of the components present in the final nanoparticle composition (*e.g.* nanoemulsion) are present in the premix and are subjected to high shear force to produce the nanoparticle composition. In some embodiments, one or more of the components that are present in the final nanoparticle composition is/are missing from the premix or is/are present in the premix in a smaller amount than in the final nanoparticle composition. That is, in some embodiments, one or more materials are added to the nanoparticle composition after the premix is subjected to high shear force.

In certain embodiments, the premix is prepared as a solution prior to application of high shear force. In particular, for nanoparticle compositions that include at least one therapeutic agent (*e.g.,* botulinum toxin), it is often desirable for the therapeutic agent to be dissolved in the premix before the high shear force is applied. Thus, in many embodiments, the therapeutic agent is soluble in at least one of the media (or in a combination of media utilized in the premix). In some embodiments, such dissolution requires heating; in other embodiments it does not.

In some embodiments, the premix components may assemble into particles before the application of high shear force. At least some of such particles may be microparticles or even nanoparticles. In some embodiments, a nanoparticle composition is prepared from a premix, wherein the premix is selected from the group comprising a suspension or a microemulsion. In some embodiments, however, particle structures do not form in the premix before application of high shear force.

In certain embodiments, relative amount of premix components are selected or adjusted to generate nanoparticles having desired characteristics. In some embodiments, the premix comprises oil and surfactant at a ratio ranging between 0.5 - 10. In some embodiments, the ratio of oil to surfactant is approximately 0.5:1, approximately 1:1, approximately 2:1, approximately 3:1, approximately 4:1, approximately 5:1, approximately 6:1, approximately 7:1, approximately 8:1, approximately 9:1 or approximately 10:1. In some embodiments, the ratio of surfactant to oil is approximately 0.5:1, approximately 1:1, approximately 2:1, approximately 3:1, approximately 4:1, approximately 5:1, approximately 6:1, approximately 7:1, approximately 8:1, approximately 9:1 or approximately 10:1.

In some embodiments, oil and surfactant are utilized at a ratio ranging between 0.5 and 2. In certain embodiments, the ratio of oil to surfactant is approximately 0.5:1, approximately 1:1, or approximately 2:1. In certain embodiments, the ratio of surfactant to oil is approximately 0.5:1, approximately 1:1, or approximately 2:1. In certain specific embodiments, the ratio of oil to surfactant is approximately 1:1.

In some embodiments, the water and surfactant are utilized at a ratio ranging between 0.5 and 10. In some embodiments, the ratio of water to surfactant is approximately 0.5:1, approximately 1:1, approximately 2:1, approximately 3:1, approximately 4:1, approximately 5:1, approximately 6:1, approximately 7:1, approximately 8:1, approximately 9:1 or approximately 10:1. In some embodiments, the ratio of surfactant to water is approximately 0.5:1, approximately 1:1, approximately 2:1, approximately 3:1, approximately 4:1, approximately 5:1, approximately 6:1, approximately 7:1, approximately 8:1, approximately 9:1 or approximately 10:1. In some embodiments, water and surfactant are utilized at a ratio ranging between 0.5 and 2. In certain embodiments, the ratio of water to surfactant is approximately 0.5:1, approximately 1:1, or approximately 2:1. In certain embodiments, the ratio of surfactant to water is approximately 0.5:1, approximately 1:1, or approximately 2:1. In certain specific embodiments, the ratio of water to surfactant is approximately 1:1. In some embodiments, compositions utilizing such ratios of water to surfactant comprise water-in-oil emulsions.

In some embodiments, the percent of oil in the premix ranges between 0% and 30%. In some embodiments, the percent of oil in the premix ranges between 0% and 5%, between 5% and 10%, between 10% and 15%, between 15% and 20%, between 20% and 25%, or between 25% and 30%. In some embodiments, the percent of oil in the premix ranges between 0% and 10%, between 0% and 20%, or between 0% and 30%. In some embodiments, the percent of oil in the premix ranges between 10% and 20% or between 10% and 30%. In some embodiments, the percent of oil in the premix ranges between 20% and 30%.

In some embodiments the percent of oil in the premix is approximately 1%, approximately 2%, approximately 3%, approximately 4%, approximately 5%, approximately 6%, approximately 7%, approximately 9%, approximately 10%, approximately 11%, approximately 12%, approximately 13%, approximately 14%, approximately 15%, approximately 16%, approximately 17%, approximately 18%, approximately 19%, approximately 20%, approximately 21%, approximately 22%, approximately 23%, approximately 24%, approximately 25%, approximately 26%, approximately 27%, approximately 28%, approximately 29% or approximately 30%. In some embodiments the percent of oil is approximately 10%. In some embodiments the percent of oil is approximately 9%. In some embodiments the percent of oil is approximately 8%. In some embodiments the percent of oil is approximately 7%. In some embodiments the percent of oil is approximately 6%. In some embodiments the percent of oil is approximately 5%. In some embodiments the percent of oil is approximately 4%. In some embodiments the percent of oil is approximately 3%. In some embodiments the percent of oil is approximately 2%. In some embodiments the percent of oil is approximately 1%.

The percent of water in the premix can range from 0% to 99%, from 10% to 99%, from 25% to 99%, from 50% to 99%, or from 75% to 99%. In some embodiments, the percent of water in the premix can range from 0% to 75%, from 0% to 50%, from 0% to 25%, or from 0% to 10%. In some embodiments, the percent of water in the premix ranges between 0% and 30%. In some embodiments the percent of water is approximately 1%, approximately 2%, approximately 3%, approximately 4%, approximately 5%, approximately 6%, approximately 7%, approximately 9%, approximately 10%, approximately 11%, approximately 12%, approximately 13%, approximately 14%, approximately 15%, approximately 16%, approximately 17%, approximately 18%, approximately 19%, approximately 20%, approximately 21%, approximately 22%, approximately 23%, approximately 24%, approximately 25%, approximately 26%, approximately 27%, approximately 28%, approximately 29%, approximately 30%, approximately 35%, approximately 40%, approximately 45%, approximately 50%, approximately 55%, approximately 60%, approximately 65%, approximately 70%, approximately 71%, approximately 72%, approximately 73%, approximately 74%, approximately 75%, approximately 76%, approximately 77%, approximately 78%, approximately 79%, approximately 80%, approximately 81%, approximately 82%, approximately 83%, approximately 84%, approximately 85%, approximately 86%, approximately 87%, approximately 88%, approximately 89%, approximately 90%, approximately 91%, approximately 92%, approximately 93%, approximately 94%, approximately 95%, approximately 96%, approximately 97%, approximately 98%, or approximately 99%,. In some embodiments the percent of water is approximately 83%. In some embodiments the percent of water is approximately 9%. In some embodiments the percent of water is approximately 5%.

In some embodiments, the percent of surfactant in the premix ranges between 0% -30%. In some embodiments the percent of surfactant in the premix is about 1%, about 2%, about 3%, about 4%, about 5%, about 6%, about 7%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, or about 30%. In some embodiments the percent of surfactant is approximately 10%. In some embodiments the percent of surfactant is approximately 9%. In some embodiments the percent of surfactant is approximately 8%. In some embodiments the percent of surfactant is approximately 7%. In some embodiments the percent of surfactant is approximately 6%. In some embodiments the percent of surfactant is approximately 5%.

In some embodiments, a nanoparticle composition does not contain more than one oil. In some embodiments, a nanoparticle composition may comprise two or more oils. In some embodiments, a nanoparticle composition does not contain more than one surfactant. In some embodiments, a nanoparticle composition may comprise two or more surfactants.

In some embodiments, a nanoparticle composition consists essentially of water, an oil, a surfactant, and a therapeutic agent (*e.g.* botulinum toxin). In some embodiments, a nanoparticle composition consists essentially of water, an oil, a surfactant, at least one therapeutic agent (*e.g.,* botulinum toxin), and at least one substance used to produce and/or preserve the nanoparticle composition (*e.g.,* proteins, salts, *etc*.).

In some embodiments, a nanoparticle composition consists of water, an oil, a surfactant, and a therapeutic agent (*e.g.,* botulinum toxin). In some embodiments, a nanoparticle composition consists of water, an oil, a surfactant, at least one therapeutic agent (*e.g.,* botulinum toxin), and at least one substance used to produce and/or preserve the nanoparticle composition (*e.g.,* proteins, salts, *etc*.).

### Therapeutic Agents

In some embodiments, a nanoparticle composition may comprise one or more therapeutic agents. In some embodiments, one or more therapeutic agents may be incorporated into a premix which is subjected to high shear force to generate nanoparticle compositions. In some embodiments, one or more therapeutic agents may be mixed with nanoparticle compositions when preparing a pharmaceutical composition.

### Botulinum Toxin

Botulinum toxin (BTX) BTX is produced in nature by the anaerobic, gram positive bacillus *Clostridium botulinum* and is a potent polypeptide neurotoxin. Most notably, BTX causes a neuroparalytic illness in humans and animals referred to as botulism. BTX can apparently pass through the lining of the gut and attack peripheral motor neurons. Symptoms of botulinum toxin intoxication can progress from difficulty walking, swallowing, and speaking to paralysis of the respiratory muscles, and death.

BTX-A is the most lethal natural biological agent known to man. The LD₅₀ in female Swiss Webster mice (18 g - 20 g) for commercially available BTX-A is about 50 picograms; this amount is defined as 1 Unit of BTX-A. On a molar basis, BTX-A is about 1.8 billion times more lethal than diphtheria, about 600 million times more lethal than sodium cyanide, about 30 million times more lethal than cobra toxin and about 12 million times more lethal than cholera (Singh, et al., ed., "Critical Aspects of Bacterial Protein Toxins" Natural Toxins II, pp. 63-84, Plenum Press, New York, 1996).

The different serotypes of botulinum toxin vary in the animal species that they affect and in the severity and duration of the paralysis they evoke. For example, it has been determined that BTX-A is 500 times more potent than is BTX-B, as measured by the rate of paralysis produced in the rat. Additionally, BTX-B has been determined to be non-toxic in primates at a dose of 480 U/kg, which is about 12 times the primate LD₅₀ for BTX-A. Furthermore, it is known that botulinum toxin type B has, upon intramuscular injection, a shorter duration of activity and is also less potent than BTX-A at the same dose level.

Botulinum toxin apparently binds with high affinity to cholinergic motor neurons, is translocated into the neuron and blocks the release of acetylcholine and other preformed mediators and transmitters. For example, *in vitro* studies performed on neurons other than motor neurons revealed that botulinum toxin not only blocks acetylcholine release, but can also prevent liberation of other neurotransmitters (*e.g.,* neurotransmitters stored in vesicles), including small organic molecules and neuropeptides (*e.g.,* adrenaline; noradrenaline; dopamine; glutamate; aspartate; glycine; GABA; ATP that is co-stored with neurotransmitters such as acetylcholine and/or glutamate; substance P; and/or CGRP) (Poulain, 2008, Botulinum J., 1:14).

Botulinum toxins have been used in clinical settings for the treatment of certain neuromuscular disorders. In particular, BTX-A has been approved by the U.S. Food and Drug Administration for the treatment of cervical dystonia in adults to decrease the severity of abnormal head position and neck pain associated with cervical dystonia; the treatment of severe primary axillary hyperhidrosis that is inadequately managed with topical agents; the treatment of strabismus and blepharospasm associated with dystonia, including benign essential blepharospasm or VII nerve disorders in patients 12 years of age and above; and for the temporary improvement in the appearance of moderate to severe glabellar lines associated with corrugator and/or procerus muscle activity in adult patients ≤ 65 years of age.

Clinical effects of peripheral intramuscular BTX-A are usually seen within one week of injection. The typical duration of symptomatic relief from a single intramuscular injection of BTX-A averages about three months.

Although all the botulinum toxins serotypes apparently inhibit release of the neurotransmitter acetylcholine at the neuromuscular junction, they do so by affecting different neurosecretory proteins and/or cleaving these proteins at different sites. For example, botulinum types A and E both cleave the 25 kilodalton (kD) synaptosomal associated protein (SNAP-25), but they target different amino acid sequences within this protein. Botulinum toxin types B, D, F and G act on vesicle-associated membrane protein (VAMP, also called synaptobrevin), with each serotype cleaving the protein at a different site. Finally, botulinum toxin type C₁ has been shown to cleave both syntaxin and SNAP-25. These differences in mechanism of action may affect the relative potency and/or duration of action of the various botulinum toxin serotypes. The cytosol of pancreatic islet B cells contains at least SNAP-25 (Gonelle-Gispert et al., 1999, Biochem. J., 339 (pt 1): 159-65), and synaptobrevin (1995, Mov. Disord., 10: 376).

The molecular weight of a botulinum toxin protein molecule, for all seven of the known botulinum toxin serotypes, is about 150 kD. Botulinum toxins are released by the *Clostridium* bacterium as complexes comprising the 150 kD botulinum toxin protein molecule along with associated non-toxin proteins. Thus, the BTX-A complex can be produced by the *Clostridium* bacterium as 900 kD, 500 kD and 360 kD forms. Botulinum toxin types B and C₁ are apparently produced as only a 500 kD complex. Botulinum toxin type D is produced as both 300 kD and 500 kD complexes. Finally, botulinum toxin types E and F are produced as only approximately 300 kD complexes.

BTX complexes (*i.e.,* those compositions having molecular weights greater than about 150 kD) are believed to contain a non-toxin hemagglutinin protein and a non-toxin and non-toxic non-hemagglutinin protein. These two non-toxin proteins (which along with the botulinum toxin molecule comprise the relevant neurotoxin complex) may act to provide stability against denaturation to the botulinum toxin molecule and protection against digestive acids when toxin is ingested.

Either BTX proteins or BTX complexes may be utilized in accordance with the present invention. Indeed, it will be appreciated by those of ordinary skill in the art that any portion or fragment of a BTX protein or complex that retains the appropriate activity may be utilized as described herein.

*In vitro* studies have indicated that botulinum toxin inhibits potassium cation induced release of both acetylcholine and norepinephrine from primary cell cultures of brainstem tissue. Additionally, it has been reported that botulinum toxin inhibits the evoked release of both glycine and glutamate in primary cultures of spinal cord neurons and that in brain synaptosome preparations botulinum toxin inhibits the release of each of the neurotransmitters acetylcholine, dopamine, norepinephrine, CGRP and glutamate.

As noted above, the source of botulinum toxin is not critical to the present invention. For purposes of completeness, however, we note that a variety of sources, including commercial sources, for certain botulinum toxin preparations are readily available.

For example, BTX or BTX complex can be obtained by establishing and growing cultures of *Clostridium botulinum* in a fermenter and then harvesting and purifying the fermented mixture in accordance with known procedures. All the botulinum toxin serotypes are initially synthesized as inactive single chain proteins which must be cleaved or nicked by proteases to become neuroactive. The bacterial strains that make botulinum toxin serotypes A and G possess endogenous proteases. Therefore, serotypes A and G can be recovered from bacterial cultures in predominantly their active form. In contrast, botulinum toxin serotypes C₁, D and E are synthesized by nonproteolytic strains and are therefore typically unactivated when recovered from culture. Serotypes B and F are produced by both proteolytic and nonproteolytic strains and therefore can be recovered in either the active or inactive form. However, even the proteolytic strains that produce, for example, the BTX-A serotype typically only cleave a portion of the toxin produced. The exact proportion of nicked to unnicked molecules can depend on the length of incubation and the temperature of the culture. Therefore, a certain percentage of any preparation of, for example BTX-A, is likely to be inactive. The presence of inactive botulinum toxin molecules in a clinical preparation will contribute to the overall protein load of the preparation, which has been linked in some commercially available botulinum toxin preparations to increased antigenicity, without contributing to its clinical efficacy.

High quality crystalline botulinum toxin type A can be produced from the Hall A strain of *Clostridium botulinum* with characteristics of ≥ 3 × 10⁷ U/mg, an A₂₆₀/A₂₇₈ of less than 0.60 and a distinct pattern of banding on gel electrophoresis. The known Schantz process can be used to obtain crystalline botulinum toxin including type A (Shantz et al., 1992, Microbiol. Rev., 56:80).

Generally, the botulinum toxin complex can be isolated and purified from an anaerobic fermentation by cultivating *Clostridium botulinum* (*e.g.,* type A) in a suitable medium. The known process can be used, upon separation out of the non-toxin proteins, to obtain pure botulinum toxins, such as for example: purified botulinum toxin type A with an approximately 150 kD molecular weight with a specific potency of 1-2 × 10⁸ LD₅₀ U/mg or greater; purified botulinum toxin type B with an approximately 156 kD molecular weight with a specific potency of 1-2 × 10⁸ LD₅₀ U/mg or greater, and; purified botulinum toxin type F with an approximately 155 kD molecular weight with a specific potency of 1-2 × 10⁷ LD₅₀ U/mg or greater.

Alternatively or additionally, already prepared and purified botulinum toxins and toxin complexes can be obtained from, for example, List Biological Laboratories, Inc., Campbell, CA; the Centre for Applied Microbiology and Research, Porton Down, U.K.; Wako (Osaka, Japan) as well as from Sigma Chemicals of St. Louis, MO.

Pure botulinum toxin, when administered as a free solution, is so labile that it is generally not used to prepare a pharmaceutical composition. Furthermore, the botulinum toxin complexes, such the toxin type A complex can also be susceptible to denaturation due to surface denaturation, heat, and alkaline conditions. In some cases, inactivated toxin forms toxoid proteins which may be immunogenic. Resulting antibodies can render a patient refractory to toxin injection.

In some embodiments, the present invention provides botulinum toxin nanoparticle compositions (*e.g.,* nanoemulsions) in which the botulinum toxin has improved stability when compared to currently administered free solutions. That is, in some embodiments, botulinum toxin present in a nanoparticle composition is protected, at least in part, from at least one adverse condition such as heat, alkaline conditions, acidic conditions, degradative enzymes, host organism antibodies, *etc.* Alternatively or additionally, botulinum toxin present in nanoparticle compositions may show less surface denaturation than an otherwise comparable preparation of botulinum toxin in free solution. Surface denaturation refers to protein degradation that results from interactions of proteins with surfaces (*e.g.,* walls of a container in which proteins are stored) or with air (*e.g.,* at the interface between a nanoparticle composition and air).

Indeed, one surprising aspect of the present invention encompasses the recognition that botulinum toxin may be stabilized by incorporation into a nanoparticle composition. Those of ordinary skill in the art will readily appreciate that a nanoparticle composition according to this aspect of the present invention may be prepared by any available means. In some embodiments, the present invention allows use of isolated botulinum toxin rather than botulinum toxin complex, at least in part due to the additional stability imparted by incorporation into a nanoparticle composition.

The present invention further provides botulinum toxin nanoparticle compositions (*e.g.,* nanoemulsions) in which the botulinum toxin has improved ability to permeate skin when compared to currently administered free solutions. In some embodiments, the minimal time between administration and intracellular accumulation results in a method of administration having improved efficacy and decreased side effects.

Moreover, as demonstrated herein, the present invention provides botulinum toxin nanoparticle compositions (*e.g.* nanoemulsions) from which botulinum toxin can cross the skin without requiring alteration or disruption of skin structures. For example, commercially available technologies for transdermal administration of biologically active agents traditionally require chemical, physical, electrical or other disruption of at least the outer layer of skin. Such disruption can cause irritation, undesirable medical side-effects, and/or unwanted aesthetic outcomes. The present invention provides botulinum toxin nanoparticle compositions that, when administered to skin, do not significantly or noticeably irritate the skin and/or erode the stratum corneum, and yet allow botulinum toxin to permeate the skin to have its biological effects.

As with proteins generally, the biological activities of the botulinum toxins (which are intracellular peptidases) can be affected by changes in three dimensional conformation. Thus, botulinum toxin type A can be detoxified by heat, various chemicals, surface stretching and surface drying. Additionally, it is known that dilution of the toxin complex obtained by the known culturing, fermentation and purification to the much, much lower toxin concentrations used for pharmaceutical composition formulation results in rapid detoxification of the toxin unless a suitable stabilizing agent is present. Dilution of the toxin from milligram quantities to a solution containing nanograms per milliliter presents significant difficulties because of the rapid loss of specific toxicity upon such great dilution. Since the toxin may be used months or years after the toxin containing pharmaceutical composition is formulated, solution preparations of the toxin may be formulated with a stabilizing agent, such as albumin.

As noted above, the present invention may provide stabilized preparations of botulinum toxin. Notwithstanding the additional stability that may be imparted by the formulation itself, in some embodiments, use of additional stabilizers is contemplated. For example, in some embodiments, at least one additional protein is used together with the botulinum toxin. In some embodiments, this additional protein comprises albumin. In some embodiments, this additional protein comprises one or more of the proteins naturally found in a botulinum toxin complex. Indeed, in some embodiments, a complete botulinum toxin complex is employed. In some such embodiments, albumin is also utilized. Thus, in some embodiments, the present invention provides a botulinum nanoemulsion (*e.g.,* microfluidized nanoemulsion) comprising albumin.

In some embodiments, the botulinum toxin utilized is BOTOX® (Allergan, Inc.). BOTOX® consists of a purified botulinum toxin type A complex, albumin and sodium chloride packaged in sterile, vacuum-dried form.

The botulinum toxin type A present in BOTOX® is made from a culture of the Hall strain of *Clostridium botulinum* grown in a medium containing N-Z amine and yeast extract. The botulinum toxin type A complex is purified from the culture solution by a series of acid precipitations to a crystalline complex (see, *e.g.,* Shantz et al., 1992, Microbiol. Rev., 56:80) consisting of the active high molecular weight toxin protein and at least one associated hemagglutinin protein. The crystalline complex is re-dissolved in a solution containing saline and albumin and sterile filtered (0.2 microns) prior to vacuum-drying. BOTOX® can be reconstituted with sterile, non-preserved saline prior to intramuscular injection. Each vial of BOTOX® contains about 100 units (U) of *Clostridium botulinum* toxin type A purified neurotoxin complex, 0.5 milligrams of human serum albumin, and 0.9 milligrams of sodium chloride in a sterile, vacuum-dried form without a preservative.

Currently, BOTOX® is typically reconstituted with 0.9% sodium chloride for administration by injection. Since there is a concern that BOTOX® can be denatured by bubbling or similar violent agitation, it is recommended that the diluent be gently injected into the vial. BOTOX®, as a free solution, is recommended to be administered within four hours after reconstitution. Further, between reconstitution and injection, it is further recommended that reconstituted BOTOX® be stored in a refrigerator (*i.e.,* for example, between 2° to 8° C). Reconstituted BOTOX® is clear, colorless and free of particulate matter.

It has been reported that BOTOX° has been used in clinical settings as follows (for a review, see, *e.g.,* Poulain, 2008, Botulinum J., 1:14):
(1) about 75 U - 125 U of BOTOX° per intramuscular injection (multiple muscles) to treat cervical dystonia;
(2) 5 U - 10 U of BOTOX° per intramuscular injection to treat glabellar lines (brow furrows) (5 units injected intramuscularly into the procerus muscle and 10 units injected intramuscularly into each corrugator supercilli muscle);
(3) about 30 U - 80 U of BOTOX® to treat constipation by intrasphincter injection of the puborectalis muscle;
(4) about 1 U - 5 U per muscle of intramuscularly injected BOTOX® to treat blepharospasm by injecting the lateral pre-tarsal orbicularis oculi muscle of the upper lid and the lateral pre-tarsal orbicularis oculi of the lower lid.
(5) to treat strabismus, extraocular muscles have be in injected intramuscularly with between about 1 U -5 U of BOTOX®, the amount injected varying based upon both the size of the muscle to be injected and the extent of muscle paralysis desired (*i.e.,* amount of diopter correction desired).
(6) to treat upper limb spasticity following stroke by intramuscular injections of BOTOX° into five different upper limb flexor muscles, as follows:
   (a) flexor digitorum profundus: 7.5 U to 30 U
   (b) flexor digitorum sublimus: 7.5 U to 30 U
   (c) flexor carpi ulnaris: 10 U to 40 U
   (d) flexor carpi radialis: 15 U to 60 U
   (e) biceps brachii: 50 U to 200 U
   Each of the five indicated muscles has been injected at the same treatment session, so that the patient receives from 90 U to 360 U of upper limb flexor muscle BOTOX® by intramuscular injection at each treatment session.
(7) to treat migraine, pericranial injected (injected symmetrically into glabellar, frontalis and temporalis muscles) injection of 25 U of BOTOX® has showed significant benefit as a prophylactic treatment of migraine compared to vehicle as measured by decreased measures of migraine frequency, maximal severity, associated vomiting and acute medication use over the three month period following the 25 U injection.

The present invention demonstrates (see, for example, Examples 4 and 5) that a botulinum nanoparticle composition, when incorporated into a cream that is applied to the skin for transdermal delivery of the toxin, achieves biological results (*i.e.,* reduction of wrinkles) comparable to those historically observed with injection of a botulinum toxin solution containing approximately the same amount of BOTOX®.

The positive clinical responses of botulinum toxin type A has led to interest in other botulinum toxin serotypes. A study of two commercially available botulinum type A preparations (BOTOX® and DYSPORT®) and preparations of botulinum toxins type B and F (both obtained from Wako Chemicals, Japan) has been carried out to determine local muscle weakening efficacy, safety and antigenic potential in mice. Botulinum toxin preparations were injected into the head of the right gastrocnemius muscle (0.5 to 200.0 U/kg) and muscle weakness was assessed using the mouse digit abduction scoring assay (DAS). ED₅₀ values were calculated from dose response curves.

Additional mice were given intramuscular or peritoneal injections to determine LD₅₀ doses. The therapeutic index was calculated as LD₅₀ /ED₅₀. Separate groups of mice received hind limb injections of BOTOX® (5.0 to 10.0 U/kg) or botulinum toxin type B (50.0 to 400.0 U/kg), and were tested for muscle weakness and increased water consumption, the later being a putative model for dry mouth. Peak muscle weakness and duration were dose related for all serotypes.

DAS ED₅₀ values (U/kg) were as follows: BOTOX®: 6.7, DYSPORT®: 24.7, botulinum toxin type B: 27.0 to 244.0, botulinum toxin type F: 4.3. BOTOX® had a longer duration of action than botulinum toxin type B or botulinum toxin type F. Therapeutic index values were as follows: BOTOX®: 10.5, DYSPORT®: 6.3, botulinum toxin type B: 3.2. Water consumption was greater in mice injected with botulinum toxin type B than with BOTOX®, although botulinum toxin type B was less effective at weakening muscles. DAS results indicate relative peak potencies of botulinum toxin type A being equal to botulinum toxin type F, and botulinum toxin type F being greater than botulinum toxin type B. With regard to duration of effect, botulinum toxin type A was greater than botulinum toxin type B, and botulinum toxin type B duration of effect was greater than botulinum toxin type F. As shown by the therapeutic index values, the two commercial preparations of botulinum toxin type A (BOTOX® and DYSPORT ®) are different. The increased water consumption behavior observed following hind limb injection of botulinum toxin type B indicates that clinically significant amounts of this serotype entered the murine systemic circulation. The results also indicate that in order to achieve efficacy comparable to botulinum toxin type A, it may be necessary to increase doses of the other serotypes examined. Increased dosage, however, can compromise safety.

Antigenic potential was assessed by monthly intramuscular injections in rabbits (1.5 or 6.5 ng/kg for botulinum toxin type B or 0.15 ng/kg for BOTOX®). After four months of injections, 2 of 4 rabbits treated with 1.5 ng/kg and 4 of 4 animals treated with 6.5 ng/kg developed antibodies against botulinum toxin type B. In a separate study, 0 of 9 BOTOX® treated rabbits demonstrated antibodies against botulinum toxin type A. Therefore, in rabbits, botulinum toxin type B was more antigenic than was BOTOX®, possibly because of the higher protein load injected to achieve an effective dose of botulinum toxin type B (Aoki, 1999, Eur. J. Neurol., 6:S3-S10).

As indicated herein, the present invention contemplates use of botulinum toxin of any serotype. Those of ordinary skill in the art will readily be able to assess the appropriateness of a particular serotype for a particular use and, according to the teachings herein, will be able to prepare nanoparticle compositions containing such botulinum toxin. Thus, the present invention provides nanoparticle compositions containing botulinum toxin of any serotype, including compositions containing only botulinum toxin proteins and compositions containing one or more other proteins. In some embodiments, such other proteins comprise or consist of albumin; in some embodiments, botulinum toxin complexes are employed.

Commercially available sources of botulinum toxin that may be utilized in accordance with the present invention include, but are not limited to, BOTOX®, DYSPORT® (*Clostridium botulinum* type A toxin hemagglutinin complex with human serum albumin and lactose; Ispen Limited, Berkshire U.K.), Xeomin®, PurTox®, Medy-Tox, NT-201 (Merz Pharmaceuticals), and/or MYOBLOC® (an injectable solution consisting of botulinum toxin type B, human serum albumin, sodium succinate, and sodium chloride, pH 5.6, Elan Pharmaceuticals, Dublin, Ireland), *etc.*

### Therapeutic Agents Useful for Acne Treatment

In some embodiments, a therapeutic agent is useful for treating acne. In accordance with the present invention, the therapeutic agent that is useful for treating acne is botulinum toxin. In some embodiments, a therapeutic agent useful for treatment of acne can be a topical bactericidal, for example, benzoyl peroxide, triclosan, and/or chlorhexidine gluconate. In addition to its therapeutic effect as a keratolytic (*i.e.,* a substance that dissolves keratin plugging pores), benzoyl peroxide can prevent new lesions by killing *P. acnes.* Benzoyl peroxide is also thought to decrease the presence of free fatty acids, resulting in a decrease in inflammation and follicular obstruction. In one study, roughly 70% of participants using a 10% benzoyl peroxide solution experienced a reduction in acne lesions after 6 weeks (Dogra et al., 1993, Indian J. Dermatol. Venereol. Leprol., 59:243-6). Benzoyl peroxide routinely causes dryness, local irritation, and redness. Nanoemulsions in accordance with the present invention may be prepared with ingredients (*e.g.,* oil, surfactant, aqueous medium, excipients, *etc*.) that can help reduce or ameliorate one or more of these side effects. In some embodiments, a topical bactericidal is present in a premix at a concentration of about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, a topical bactericidal is present in a nanoparticle composition at a concentration of about 0.1%, about 0.5%, about 0.75%, 1%, about 2%, about 2.5%, about 5%, about 7.5%, about 10%, about 15%, about 20%, or about 25%. In some embodiments, a topical bactericidal is present in a premix and/or in a nanoparticle composition at a concentration ranging between about 0.1% to about 50%, about 0.1% to about 25%, about 0.1% to about 10%, or about 0.1% to about 5%. In some embodiments, a topical bactericidal is present in a premix and/or in a nanoparticle composition at a concentration ranging between about 0.1% to about 50%, about 5% to about 50%, about 10% to about 50%, or about 25% to about 50%.

In some embodiments, a therapeutic agent useful for treatment of acne can be a topical antibiotic, for example, erythromycin, clindamycin, Stiemycin, doxycycline, and/or tetracycline. Topical antibiotics typically aim to kill the bacteria (*e.g., P. acnes*) that are harbored in follicles. While topical use of antibiotics can be equally as effective as oral use, topical administration may avoid side effects including upset stomach and drug interactions (*e.g.,* it will not affect use of the oral contraceptive pill). In some embodiments, a topical antibiotic is present in a premix at a concentration of about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, a topical antibiotic is present in a nanoparticle composition at a concentration of about 0.1%, about 0.5%, about 0.75%, 1%, about 2%, about 2.5%, about 5%, about 7.5%, about 10%, about 15%, about 20%, or about 25%. In some embodiments, a topical antibiotic is present in a premix and/or in a nanoparticle composition at a concentration ranging between about 0.1% to about 50%, about 0.1% to about 25%, about 0.1% to about 10%, or about 0.1% to about 5%. In some embodiments, a topical antibiotic is present in a premix and/or in a nanoparticle composition at a concentration ranging between about 0.1% to about 50%, about 5% to about 50%, about 10% to about 50%, or about 25% to about 50%.

In some embodiments, a therapeutic agent useful for treatment of acne can be a hormone, for example, cortisone. Injected cortisone has the effect of flattening a pimple, thereby making it less conspicuous, and can also aid in the healing process. Side effects of injection may include a temporary whitening of the skin around the injection point, formation of a small depression, and/or scarring. In some embodiments, a hormone such as cortisone is present in a premix at a concentration of about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, a hormone such as cortisone is present in a nanoparticle composition at a concentration of about 0.1%, about 0.5%, about 0.75%, 1%, about 2%, about 2.5%, about 5%, about 7.5%, about 10%, about 15%, about 20%, or about 25%. In some embodiments, a hormone such as cortisone is present in a premix and/or in a nanoparticle composition at a concentration ranging between about 0.1 % to about 50%, about 0.1% to about 25%, about 0.1% to about 10%, or about 0.1% to about 5%. In some embodiments, a hormone such as cortisone is present in a premix and/or in a nanoparticle composition at a concentration ranging between about 0.1% to about 50%, about 5% to about 50%, about 10% to about 50%, or about 25% to about 50%.

In some embodiments, a therapeutic agent useful for treatment of acne can be a topical retinoid, for example, tretinoin (RETIN-A®), adapalene (DIFFERIN®), and tazarotene (TAZORAC®), retinol, *etc.* Topical retinoids may function by influencing cell creation and cell death in the follicle lining, thereby preventing hyperkeratinization of these cells. Topical retinoids can cause significant irritation of the skin, and they often cause an initial flare up of acne and facial flushing. In some embodiments, a topical retinoid is present in a premix at a concentration of about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, a topical retinoid is present in a nanoparticle composition at a concentration of about 0.1%, about 0.5%, about 0.75%, 1%, about 2%, about 2.5%, about 5%, about 7.5%, about 10%, about 15%, about 20%, or about 25%. In some embodiments, a topical retinoid is present in a premix and/or in a nanoparticle composition at a concentration ranging between about 0.1% to about 50%, about 0.1% to about 25%, about 0.1% to about 10%, or about 0.1% to about 5%. In some embodiments, a topical retinoid is present in a premix and/or in a nanoparticle composition at a concentration ranging between about 0.1% to about 50%, about 5% to about 50%, about 10% to about 50%, or about 25% to about 50%.

In some embodiments, a therapeutic agent useful for treatment of acne can be a natural product with anti-acne activity, for example, aloe vera, aruna, haldi (*i.e.,* turmeric), papaya, *etc.* (Mantle et al., 2001, Adverse Drug Reactions and Toxicological Reviews, 20:89-103). In some embodiments, a natural product with anti-acne activity is present in a premix at a concentration of about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, a natural product with anti-acne activity is present in a nanoparticle composition at a concentration of about 0.1%, about 0.5%, about 0.75%, 1%, about 2%, about 2.5%, about 5%, about 7.5%, about 10%, about 15%, about 20%, or about 25%.

In some embodiments, a therapeutic agent useful for treatment of acne can be azelaic acid (brand names AZELEX™, FINACEA®, FINEVIN®, SKINOREN, *etc*.). In some embodiments, azelaic acid is present in a premix at a concentration of about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, azelaic acid is present in a nanoparticle composition at a concentration of about 0.1%, about 0.5%, about 0.75%, 1%, about 2%, about 2.5%, about 5%, about 7.5%, about 10%, about 15%, about 20%, or about 25%. In some embodiments, azelaic acid is present in a premix and/or in a nanoparticle composition at a concentration ranging between about 0.1 to about 50%, about 0.1% to about 25%, about 0.1% to about 10%, or about 0.1% to about 5%. In some embodiments, azelaic acid is present in a premix and/or in a nanoparticle composition at a concentration ranging between about 0.1% to about 50%, about 5% to about 50%, about 10% to about 50%, or about 25% to about 50%.

In some embodiments, a therapeutic agent useful for treatment of acne can be nicotinamide (*i.e.,* vitamin B3) (Shalita et al., 1995, Int. J. Dermatol., 34:434-7). Topical nicotinamide is thought to have anti-inflammatory activity and/or to result in increased synthesis of collagen, keratin, involucrin and/or flaggrin. In some embodiments, nicotinamide is present in a premix at a concentration of about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, nicotinamide is present in a nanoparticle composition at a concentration of about 0.1%, about 0.5%, about 0.75%, 1%, about 2%, about 2.5%, about 5%, about 7.5%, about 10%, about 15%, about 20%, or about 25%. In some embodiments, nicotinamide is present in a premix and/or in a nanoparticle composition at a concentration ranging between about 0.1% to about 50%, about 0.1% to about 25%, about 0.1% to about 10%, or about 0.1% to about 5%. In some embodiments, nicotinamide is present in a premix and/or in a nanoparticle composition at a concentration ranging between about 0.1% to about 50%, about 5% to about 50%, about 10% to about 50%, or about 25% to about 50%.

In some embodiments, a therapeutic agent useful for treatment of acne can be tea tree oil (melaleuca oil). Tea tree oil has been shown to be an effective anti-inflammatory in skin infections (Mantle et al., 2001, Adverse Drug Reactions and Toxicological Reviews, 20:89-103; Koh et al., 2002, Br. J. Dermatol., 147:1212-7; and Khalil et al., 2004, J. Invest. Dermatol., 123:683-90). In some embodiments, tea tree oil is present in a premix at a concentration of about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, tea tree oil is present in a nanoparticle composition at a concentration of about 0.1%, about 0.5%, about 0.75%, 1%, about 2%, about 2.5%, about 5%, about 7.5%, about 10%, about 15%, about 20%, or about 25%. In some embodiments, tea tree oil is present in a premix and/or in a nanoparticle composition at a concentration ranging between about 0.1% to about 50%, about 0.1% to about 25%, about 0.1% to about 10%, or about 0.1% to about 5%. In some embodiments, tea tree oil is present in a premix and/or in a nanoparticle composition at a concentration ranging between about 0.1% to about 50%, about 5% to about 50%, about 10% to about 50%, or about 25% to about 50%.

In some embodiments, a therapeutic agent useful for treatment of acne can be aminolevulinic acid, azithromycin, methylaminolevuninate, nadifloxacine, PRK124, talarozole, zileuton, and/or combinations thereof. In some embodiments, such agents are present in a premix at a concentration of about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, such agents are present in a nanoparticle composition at a concentration of about 0.1%, about 0.5%, about 0.75%, 1%, about 2%, about 2.5%, about 5%, about 7.5%, about 10%, about 15%, about 20%, or about 25%. In some embodiments, such agents are present in a premix and/or in a nanoparticle composition at a concentration ranging between about 0.1% to about 50%, about 0.1% to about 25%, about 0.1% to about 10%, or about 0.1% to about 5%. In some embodiments, such agents are present in a premix and/or in a nanoparticle composition at a concentration ranging between about 0.1% to about 50%, about 5% to about 50%, about 10% to about 50%, or about 25% to about 50%.

Various acne treatments are described, for example, in Krowchuk (2000, Pediatric Dermatology, 47:841-857); and Johnson et al. (2000, American Family Physician, 62:1823-1830 and 1835-1836).

Any of the therapeutic agents described herein may be incorporated in nanoparticle compositions to be used for treatment of acne (*e.g.,* may be present in a premix and/or in a nanoparticle composition at any of the concentrations specified above). Any of the therapeutic agents useful for treatment of acne can be utilized in any combination with one another. Such agents, when used in combination, may be present in the same nanoparticle composition, or they may be present in different nanoparticle compositions. In some embodiments, botulinum toxin is utilized in combination with one or more of the acne therapeutics described herein. In some embodiments, botulinum toxin is not utilized in combination with any of the acne therapeutics described herein. Additional considerations for combination therapies are described in further detail below, in the section entitled "Treatment Applications of Nanoparticle Compositions."

### Therapeutic Agents Useful for Treatment of Rosacea

In some embodiments, a therapeutic agent is useful for treating rosacea. In accordance with the present invention, a therapeutic agent that is useful for treating rosacea is botulinum toxin. In some embodiments, such a therapeutic agent can be an oral antibiotic, for example, tetracycline, doxycycline, minocycline, metronidazole, macrolide antibiotic, and/or combinations thereof. In some embodiments, an additional therapeutic agent can be oral isotretinoin. In some embodiments, such a therapeutic agent can be a topical antibiotic (*e.g.,* metronidazole, clindamycin, erythromycin, *etc*.). In some embodiments, such a therapeutic agent is a topical azelaic acid (*e.g.,* FINACEA, AZELEX™, FINEVIN®, SKINOREN, *etc*.); topical sulfacetamide; topical sulfur; topical calcineurin inhibitor (*e.g.,* tacrolimus, pimecrolimus, *etc*.); topical benzoyl peroxide; topical permethrin; a combination of plant-sourced Methylsulfonylmethane (MSM) and Silymarin; and/or combinations thereof. In some embodiments, such a therapeutic agent can be brimonidine, dapsone, IDP-115, PRK124, SR-01, tretinoin, zinc sulfate, and/or combinations thereof.

In some embodiments, one or more of the therapeutic agents described above is present in a premix at a concentration of about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, one or more of the therapeutic agents described above is present in a nanoparticle composition at a concentration of about 0.1%, about 0.5%, about 0.75%, 1%, about 2%, about 2.5%, about 5%, about 7.5%, about 10%, about 15%, about 20%, or about 25%.

Any of the therapeutic agents described herein may be incorporated in nanoparticle compositions to be used for treatment of rosacea (*e.g.,* may be present in a premix and/or in a nanoparticle composition at any of the concentrations specified above). Any of the therapeutic agents useful for treatment of rosacea can be utilized in any combination with one another. Such agents, when used in combination, may be present in the same nanoparticle composition, or they may be present in different nanoparticle compositions. In some embodiments, botulinum toxin is utilized in combination with one or more of the rosacea therapeutics described herein. In some embodiments, botulinum toxin is not utilized in combination with any of the rosacea therapeutics described herein. Additional considerations for combination therapies are described in further detail below, in the section entitled "Treatment Applications of Nanoparticle Compositions."

### Therapeutic Agents Useful for Treatment of Excess Sebum-Producing Disorders

In some embodiments, a therapeutic agent is useful for treating excess sebum-producing disorders (*e.g.,* seborrhea, seborrheic dermatitis, *etc*.). In accordance with the present invention, a therapeutic agent that is useful for treating excess sebum-producing disorders is botulinum toxin. In some embodiments, therapeutic agents useful for treatment of excess sebum-producing disorders (*e.g.,* seborrhea, seborrheic dermatitis, *etc*.) include, but are not limited to, salicylic acid, azelaic acid, selnium sulfide, imidazoles (*e.g.,* ketoconazole, miconazole, fluconazole, econazole, bifonazole, climazole, ciclopirox, ciclopiroxolamine, *etc*.), itraconazole, terbinafine, zinc pyrithione, benzoyl peroxide, coal tar, juniper tar, glucocorticosteroids (*e.g.,* hydrocortisone, *etc*.), metronidazole, lithium, calcineurin inhibitors (*e.g.,* tacrolimus, pimecrolimus, *etc*.), Vitamin D3, isotretinoin, and/or combinations thereof.

In some embodiments, one or more of the therapeutic agents described above is present in a premix at a concentration of about 0.01%, about 0.1%, about 0.5%, about 0.75%, 1%, about 2%, about 2.5%, about 5%, about 7.5%, about 10%, about 15%, about 20%, about 25%, about 30%, about 35%, about 40%, or about 50%. In some embodiments, one or more of the therapeutic agents described above is present in a nanoparticle composition at a concentration of about 0.1%, about 0.5%, about 0.75%, 1%, about 2%, about 2.5%, about 5%, about 7.5%, about 10%, about 15%, about 20%, or about 25%.

Any of the therapeutic agents described herein may be incorporated in nanoparticle compositions to be used for treatment of excess sebum-producing disorders (*e.g.,* seborrhea, seborrheic dermatitis, *etc*.) (*e.g.,* may be present in a premix and/or in a nanoparticle composition at any of the concentrations specified above). Any of the therapeutic agents useful for treatment of excess sebum-producing disorders can be utilized in any combination with one another. Such agents, when used in combination, may be present in the same nanoparticle composition, or they may be present in different nanoparticle compositions. In some embodiments, botulinum toxin is utilized in combination with one or more of the excess sebum-producing disorder therapeutics described herein. In some embodiments, botulinum toxin is not utilized in combination with any of the excess sebum-producing disorder therapeutics described herein. Additional considerations for combination therapies are described in further detail below, in the section entitled "Treatment Applications of Nanoparticle Compositions."

### Administration

The present invention provides nanoemulsions for use in methods of delivering nanoparticle compositions for various applications including, for example, cosmetic and/or medical applications. Such nanoparticle compositions may include one or more biologically active agents. In certain embodiments, nanoparticle compositions include botulinum toxin.

In some embodiments, the present invention contemplates nanoemulsions for use in methods of delivering nanoparticle compositions including, but not limited to transdermal, topical, or intradermal, routes of administration. These routes of administration are particularly favored for formulations (*e.g.,* certain nanoparticle compositions comprising particular therapeutic agents) that are intended to have a localized effect. Subsequent tissue absorption of the formulation's ingredients, however, is not always predictable.

In some embodiments, nanoparticle compositions in accordance with the invention may be encapsulated for example using lipid-based carriers, *e.g.,* to facilitate entry into cells. Lipid-based carrier efficacies, however, may be dependent upon; i) lipid composition (*i.e.,* for example, molecular size and charge); ii) the structure (*e.g.,* molecular size and pH ionization) of any biologically active agent or other entity included in the composition; and iii) the overall health of the subject. The present invention contemplates compositions and methods related to uniform nanoemulsions (*e.g.,* microfluidized nanoemulsions) comprising lipid-based carriers thereby improving the bioavailability of cosmeceuticals.

The present invention specifically provides nanoemulsions for use in methods of administering therapeutic agents, and particularly of administering nanoparticle compositions comprising therapeutic agents, for the treatment of disorders or conditions associated with the dermal level of the skin, such as acne, rosacea, excess sebum-producing disorders (*e.g.,* seborrhea, seborrheic dermatitis, *etc*).

The therapeutic agent is botulinum toxin. Clinical effects of topically applied transdermal administration of botulinum toxins may be seen within one week, similar to botulinum toxins administered by injection. The typical duration of symptomatic relief (*i.e.,* for example, flaccid muscle paralysis) from a single intramuscular injection of botulinum toxin type A can be present for up to four months or longer; durations of clinical effect following transdermal administration of botulinum toxins according to the present invention can be present for up to four months or longer, depending on the characteristics of the individual subject and/or one the specific formulation of botulinum nanoparticle preparation.

It will be appreciated by those of ordinary skill in the art that botulinum toxin is currently administered almost exclusively by injection, and in particular by injection of a liquid saline solution, usually reconstituted from a lyophilized preparation.

As has already been discussed, BOTOX® (a purified *Clostridium botulinum* toxin type A complex, human serum albumin, and sodium chloride packaged in a sterile vacuum-dried form) is currently reconstituted for injection using sterile normal saline without a preservative (0.9% sodium chloride, injection grade). Specifically, standard injection protocols involve drawing up the proper amount of diluent in the appropriate size syringe. Since BOTOX® is denatured by bubbling or similar violent agitation, the diluent is gently injected into a vial containing a designated amount of lyophilized BOTOX®_{.} Standard injection protocols involve administering aqueous BOTOX® solutions within four hours after reconstitution.

Although problems with the available botulinum toxin preparations (including stability issues, sterility issues, *etc.)* have been well known, few improved formulations have been developed. Furthermore, injection remains the standard approach for delivering botulinum toxin, notwithstanding the undesirability of invasive techniques in general, patient discomfort, *etc.*

The present invention provides nanoemulsions for use in methods for treating acne, and/or rosacea utilizing topical administration of nanoparticle compositions comprising one or more therapeutic agents (including, but not limited to, botulinum toxin).

In certain embodiments, the present invention provides nanoemulsions for use in methods of administering a therapeutic agent (*e.g*., botulinum toxin) transdermally. Human skin comprises the dermis and the epidermis. The epidermis has several layers of tissue, namely, stratum corneum, stratum lucidum, stratum granulosum, stratum spinosum, and stratum basale (identified in order from the outer surface of the skin inward).

The stratum corneum presents the most significant hurdle in transdermal delivery of medications. The stratum corneum is typically about 10 µm - 15 µm thick, and it comprises flattened, keratised cells (corneocytes) arranged in several layers. The intercellular space between the corneocytes is filled with lipidic structures, and may play a role in the permeation of substances through skin (Bauerova et al., 2001, Eur. J. Drug Metabolism Pharmacokinetics, 26:85).

The rest of the epidermis below the stratum corneum is approximately 150 µm thick. The dermis is about 1 mm - 2 mm thick and is located below the epidermis. The dermis is supported by various capillaries as well as neuronal processes.

Transdermal administration of pharmaceuticals generally has been the subject of research in an attempt to provide an alternative route of administration of medications without undesirable consequences associated with injections and oral delivery. For example, needles often cause localized pain, bleeding and bruising, and potentially expose patients to transmissible diseases. Oral administration often suffers from poor bioavailability of medications due to the extremely acidic environment of the patient's stomach.

Efforts have been made to develop transdermal administration techniques for certain pharmaceuticals in an attempt to overcome these shortcomings by providing noninvasive administration. It is generally desirable with transdermal administration to reduce damage to a patient's skin. Thus, transdermal administration of medication may reduce or eliminate pain associated with injections and/or reduce the likelihood of infection.

Traditionally, attempts at transdermal administration of medication have been focused on increasing the permeability of the stratum corneum. Some attempts have included using chemical penetration enhancing agents that increase the permeability of molecules through the skin. Some attempts have included using mechanical apparatus to bypass or ablate portions of the stratum corneum. In addition, attempts have included use of ultrasound or iontophoresis to facilitate the permeation of pharmaceuticals through the skin. In most cases, the goal has been to deliver a pharmaceutical agent, typically a small molecule, through the skin so that an agent may pass to the capillary bed in the dermis where the agent may be systemically incorporated into the subject to achieve a therapeutic effect.

Although small molecules have been a major focus of transdermal administration techniques, it is important to note that it appears that large molecules, such as polypeptides, and protein complexes, are also amenable to transdermal administration. Erythropoietin, which is about 48 kD, has also been successfully transdermally administered with the assistance of ultrasound (Mitragotri et al., 1995, Science, 269:850; and U.S. Patents 5,814,599 and 6,002,961).

The present invention provides, among other things, nanoemulsions for use in methods of treating acne, and/or rosacea utilizing topical application of therapeutic agents (*e.g*., botulinum toxin) that does not require use of abrasive or other disrupting agents (whether chemical, mechanical, electrical, magnetic, *etc.*). The inventors have surprisingly found that therapeutic agents (*e.g*., botulinum toxin) incorporated into nanoparticle compositions are effectively delivered transdermally without further steps to permeabilize or disrupt the stratum corneum. Use of such agents or steps with nanoparticle compositions is not necessarily precluded in all embodiments, but is also not required.

In some embodiments, a nanoparticle composition (*e.g*. a nanoemulsion) is applied directly to the skin and for absorption through the epidermal layers. In some embodiments, the nanoparticle composition can penetrate the top layer of the skin, including the stratum corneum, dermal pores, and/or dermal glands, without the use of chemical or mechanical skin permeation enhancers or other agents that cause abrasion.

It will be appreciated by those of ordinary skill in the art that compositions for topical administration may have a cosmetic formulation such as skin softener, nutritional lotion type emulsion, cleansing lotion, cleansing cream, skin milk, emollient lotion, massage cream, emollient cream, make-up base, lipstick, facial pack or facial gel, cleaner formulation such as shampoos, rinses, body cleanser, hair-tonics, or soaps, or dermatological composition such as lotions, ointments, gels, creams, patches, deodorants, or sprays.

A composition for topical administration in accordance with the invention may be formulated and/or administered such that an amount of therapeutic agent between about 10⁻³ U/kg and 10 U/kg passes through a patient's skin *(i.e.,* reaches the dermal layer). In some embodiments, a composition is formulated and/or administered so that between about 10⁻² U/kg and about 1 U/kg pass through a patient's skin. In some embodiments, a composition is formulated and/or administered so that between about 10⁻¹ U/kg and about 1 U/kg pass through a patient's skin. In some embodiments, a composition is formulated and/or administered so that between about 1 U/kg and about 3 U/kg pass through a patient's skin. In some embodiments, a composition is formulated and/or administered so that between about 3 U/kg and about 5 U/kg pass through a patient's skin. In some embodiments, a composition is formulated and/or administered so that between about 5 U/kg and about 10 U/kg pass through a patient's skin. In some embodiments, a composition is formulated and/or administered so that between about 10 U/kg and about 50 U/kg pass through a patient's skin. In some embodiments, a composition is formulated and/or administered so that between about 0.1 Units and about 5 Units pass through a patient's skin. In some embodiments, a composition is formulated and/or administered at about 1U /ng, 10 U/ng, 100 U/ng, about 250 U/ng, about 500 U/ng, about 750 U/ng, about 0.1 U/pg, about 0.25 U/pg, about 0.5 U/pg, about 1.0 U/pg, about 10 U/pg, or about 100 U/pg. In some embodiments, between about 1 U and about 500 U, about 5 U and about 400 U, about 10 U and about 300 U, about 50 U and about 200 U, or about 100 U and about 150 U botulinum toxin will be administered to a patient treatment area. In some embodiments, about 1 U, about 2 U, about 3 U, about 4 U, about 5 U, about 6 U, about 7 U, about 8 U, about 9 U, about 10 U, about 11 U, about 12 U, about 13 U, about 14 U, about 15 U, about 16 U, about 17 U, about 18 U, about 19 U, about 20 U, about 30 U, about 40 U, about 50 U, about 75 U, about 100 U, about 200 U, about 300 U, about 400 U, or about 500 U botulinum toxin will be administered to a patient treatment area.

Those of ordinary skill in the art will appreciate that units herein relate to Units that are biologically equivalent or bioactively equivalent to Units defined by commercial manufacturers of any given therapeutic agent.

Therapeutic effects of a therapeutic agent administered according to the present invention may persist as long as do the effects of an injected solution. To give but one example, the effects of such injected botulinum toxin solution can persist for up to about 4 months, about 6 months, about 9 months, about 12 months, or longer. Furthermore, use of a synthetic polymer carrier that can retain the therapeutic agent so that it is released slowly may prolong the effects for up to about five years (U.S. Patent 6,312,708).

In certain embodiments, the present invention provides novel uses for topical formulations of a therapeutic agent (*e.g*., botulinum toxin) that avoid potential complications. To give but a few examples, such complications might include systemic toxicity or botulism poisoning. In some embodiments, dosages of a therapeutic agent (*e.g*., botulinum toxin, including types A, B, C, D, E, F, or G) can range from as low as about 1 unit to as high as about 20,000 units, with minimal risk of adverse side effects. The particular dosages may vary depending on the condition being treated and therapeutic regime being utilized. For example, if a therapeutic agent is botulinum toxin, treatment of subdermal, hyperactive muscles may require high transdermal dosages (*e.g*., 10 units to 20,000 units) of botulinum toxin. In comparison, treatment of neurogenic inflammation or hyperactive sweat glands may require relatively small transdermal dosages (*e.g*., about 1 unit to about 1,000 units) of botulinum toxin.

The exact amount required will vary from subject to subject, depending on the species, age, and general condition of the subject, the severity of the condition, the particular composition, its mode of administration, its mode of activity, and the like. Compositions in accordance with the invention are typically formulated in dosage unit form for ease of administration and uniformity of dosage. It will be understood, however, that the total daily usage of the compositions in accordance with the invention will be decided by the attending physician within the scope of sound medical judgment. The specific therapeutically effective dose level for any particular patient or organism will depend upon a variety of factors including the disorder being treated and the severity of the disorder; the activity of the specific composition employed; the age, body weight, general health, sex and diet of the patient; the time of administration, route of administration, and rate of clearance of the specific composition; the duration of the treatment; therapeutic agents and/or procedures used in combination or coincidental with the specific compositions employed; and like factors well known in the medical arts.

In certain embodiments, compositions in accordance with the invention may be administered at dosage levels sufficient to deliver from about 0.001 ng/kg to about 100 ng/kg, from about 0.01 ng/kg to about 50 ng/kg, from about 0.1 ng/kg to about 40 ng/kg, from about 0.5 ng/kg to about 30 ng/kg, from about 0.01 ng/kg to about 10 ng/kg, from about 0.1 ng/kg to about 10 ng/kg, or from about 1 ng/kg to about 25 ng/kg, or from about 25 ng/kg to about 50 ng/kg of subject body weight per day, one or more times a day, to obtain the desired therapeutic effect. In certain embodiments, compositions in accordance with the invention may be administered at dosage levels sufficient to deliver from about 0.01 U/kg to about 100 U/kg, from about 0.1 U/kg to about 50 U/kg, from about 0.2 U/kg to about 20 U/kg, from about 0.5 U/kg to about 15 U/kg, from about 0.1 U/kg to about 10 U/kg, or from about 0.5 U/kg to about 5 U/kg of subject body weight per day, one or more times a day, to obtain the desired therapeutic effect.

The desired dosage may be delivered three times a day, two times a day, once a day, every other day, every third day, every week, every two weeks, every three weeks, every four weeks, every six weeks, every 2 months, every 3 months, every 4 months, every 6 months, every 9 months, once a year, or longer. In certain embodiments, the desired dosage may be delivered using multiple administrations (e.g., two, three, four, five, six, seven, eight, nine, ten, eleven, twelve, thirteen, fourteen, or more administrations).

### Pharmaceutical Compositions

In some embodiments, the present invention provides pharmaceutical compositions comprising at least one therapeutic agent (e.g., botulinum toxin) for transdermal delivery into a human patient. A composition can comprise between about 1 unit to about 20,000 units of therapeutic agent, and a composition can comprise an amount of therapeutic agent sufficient to achieve a therapeutic effect lasting between 1 month and 5 years.

The present invention further provides pharmaceutical compositions comprising one or more nanoparticle compositions (e.g. nanoemulsions), together with one or more pharmaceutically acceptable excipients. In accordance with some embodiments, a method of administering pharmaceutical compositions comprising nanoparticle compositions to a subject in need thereof is provided. In some embodiments, compositions are administered to humans. For the purposes of the present disclosure, the phrase "active ingredient" generally refers to nanoparticle compositions as described herein.

In some embodiments, the present invention provides topical formulations of therapeutic agents (e.g., botulinum toxin) that allow the therapeutic agent to permeate through a subject's skin without permeating in significant amount through a blood vessel. For example, in some embodiments, less than about 25%, less than 20%, less than 15%, less than 10%, less than 10%, or even less than about 5%, of the therapeutic agent present in a pharmaceutical composition permeates into a blood vessel upon application of a topical and/or transdermal preparation.

In some embodiments, the present invention provides topical formulations of therapeutic agents (*e.g.*, botulinum toxin) that allow the therapeutic agent to permeate to the dermal level of the skin without permeating in significant amount to the subdermal level. For example, in some embodiments, less than about 35%, about 25%, less than 20%, less than 15%, less than 10%, less than 10%, less 5%, or even less than about 1%, of the therapeutic agent present in a pharmaceutical composition permeates into the subdermal level of the skin upon application of a topical and/or transdermal preparation.

Formulations of pharmaceutical compositions described herein may be prepared by any method known or hereafter developed in the art of pharmacology. In general, such preparatory methods include the step of bringing the active ingredient into association with an excipient and/or one or more other accessory ingredients, and then, if necessary and/or desirable, shaping and/or packaging the product into a desired single- or multi-dose unit.

A pharmaceutical composition in accordance with the invention may be prepared, packaged, and/or sold in bulk, as a single unit dose, and/or as a plurality of single unit doses. As used herein, a "unit dose" is a discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of active ingredient, pharmaceutically acceptable excipient, and/or any additional ingredients in a pharmaceutical composition in accordance with the invention will vary, depending upon the identity, size, and/or condition of the subject treated and further depending upon the route by which the composition is to be administered. By way of example, a composition may comprise between 0.1% and 100% (w/w) active ingredient.

Pharmaceutical formulations may additionally comprise at least one pharmaceutically acceptable excipient, which, as used herein, includes any and all solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro (Lippincott, Williams & Wilkins, Baltimore, MD, 2005) discloses various excipients used in formulating pharmaceutical compositions and known techniques for the preparation thereof. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

In some embodiments, a pharmaceutically acceptable excipient is at least 95%, at least 96%, at least 97%, at least 98%, at least 99%, or 100% pure. In some embodiments, an excipient is approved by United States Food and Drug Administration. In some embodiments, an excipient is pharmaceutical grade. In some embodiments, an excipient meets the standards of the United States Pharmacopoeia (USP), the European Pharmacopoeia (EP), the British Pharmacopoeia, and/or other International Pharmacopoeia.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in pharmaceutical formulations. Excipients such as cocoa butter and suppository waxes, coloring agents, coating agents, sweetening, flavoring, and/or perfuming agents can be present in the composition, according to the judgment of the formulator.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, *etc.,* and/or combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked poly(vinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (VEEGUM®), *etc.,* and/or combinations thereof.

Exemplary surface active agents and/or emulsifiers include, but are not limited to, natural emulsifiers (e.g., acacia, agar, alginic acid, sodium alginate, tragacanth, chondrux, cholesterol, xanthan, pectin, gelatin, egg yolk, casein, wool fat, cholesterol, wax, and lecithin), colloidal clays (*e.g*., bentonite [aluminum silicate] and VEEGUM® [magnesium aluminum silicate]), long chain amino acid derivatives, high molecular weight alcohols (*e.g.,* stearyl alcohol, cetyl alcohol, oleyl alcohol, triacetin monostearate, ethylene glycol distearate, glyceryl monostearate, and propylene glycol monostearate, polyvinyl alcohol), carbomers (*e.g*., carboxy polymethylene, polyacrylic acid, acrylic acid polymer, and carboxyvinyl polymer), carrageenan, cellulosic derivatives (*e.g*., carboxymethylcellulose sodium, powdered cellulose, hydroxymethyl cellulose, hydroxypropyl cellulose, hydroxypropyl methylcellulose, methylcellulose), sorbitan fatty acid esters (*e.g*., polyoxyethylene sorbitan monolaurate [TWEEN®20], polyoxyethylene sorbitan [TWEEN®60], polyoxyethylene sorbitan monooleate [TWEEN®80], sorbitan monopalmitate [SPAN®40], sorbitan monostearate [SPAN®60], sorbitan tristearate [SPAN®65], glyceryl monooleate, sorbitan monooleate [SPAN®80]), polyoxyethylene esters (e.g., polyoxyethylene monostearate [MYRJ®45], polyoxyethylene hydrogenated castor oil, polyethoxylated castor oil, polyoxymethylene stearate, and SOLUTOL®), sucrose fatty acid esters, polyethylene glycol fatty acid esters (*e.g.,* CREMOPHOR®), polyoxyethylene ethers, *(e.g.,* polyoxyethylene lauryl ether [BRIJ®30]), poly(vinyl-pyrrolidone), diethylene glycol monolaurate, triethanolamine oleate, sodium oleate, potassium oleate, ethyl oleate, oleic acid, ethyl laurate, sodium lauryl sulfate, PLURONIC®F 68, POLOXAMER®188, cetrimonium bromide, cetylpyridinium chloride, benzalkonium chloride, docusate sodium, *etc.* and/or combinations thereof.

Exemplary binding agents include, but are not limited to, starch *(e.g.,* cornstarch and starch paste); gelatin; sugars (*e.g*., sucrose, glucose, dextrose, dextrin, molasses, lactose, lactitol, mannitol,); natural and synthetic gums (*e.g*., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, poly(vinyl-pyrrolidone), magnesium aluminum silicate (VEEGUM®), and larch arabogalactan); alginates; polyethylene oxide; polyethylene glycol; inorganic calcium salts; silicic acid; polymethacrylates; waxes; water; alcohol; *etc.*; and combinations thereof.

Exemplary preservatives may include, but are not limited to, antioxidants, chelating agents, antimicrobial preservatives, antifungal preservatives, alcohol preservatives, acidic preservatives, and/or other preservatives. Exemplary antioxidants include, but are not limited to, alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and/or sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and/or trisodium edetate. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and/or thimerosal. Exemplary antifungal preservatives include, but are not limited to, butyl paraben, methyl paraben, ethyl paraben, propyl paraben, benzoic acid, hydroxybenzoic acid, potassium benzoate, potassium sorbate, sodium benzoate, sodium propionate, and/or sorbic acid. Exemplary alcohol preservatives include, but are not limited to, ethanol, polyethylene glycol, phenol, phenolic compounds, bisphenol, chlorobutanol, hydroxybenzoate, and/or phenylethyl alcohol. Exemplary acidic preservatives include, but are not limited to, vitamin A, vitamin C, vitamin E, beta-carotene, citric acid, acetic acid, dehydroacetic acid, ascorbic acid, sorbic acid, and/or phytic acid. Other preservatives include, but are not limited to, tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, GLYDANT PLUS ®, PHENONIP®, methylparaben, GERMALL®115, GERMABEN®II, NEOLONE™, KATHON™, and/or EUXYL®.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, *etc.,* and/or combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, *etc.,* and combinations thereof.

Exemplary oils include, but are not limited to, almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macadamia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, medium-chain triglycerides (e.g., 1349 oil), and/or combinations thereof.

Dosage forms for topical and/or transdermal administration of a composition may include ointments, pastes, creams, lotions, gels, powders, solutions, sprays, inhalants, deodorants, and/or patches. Generally, an active ingredient is admixed (e.g., under sterile conditions) with at least one pharmaceutically acceptable excipient and/or any needed preservatives and/or buffers as may be required. Additionally, the present invention contemplates the use of transdermal patches, which often have the added advantage of providing controlled delivery of a compound to the body. Such dosage forms may be prepared, for example, by dissolving and/or dispensing the compound in the proper medium. Alternatively or additionally, the rate may be controlled by either providing a rate controlling membrane and/or by dispersing the compound in a polymer matrix and/or gel.

Those of ordinary skill in the art will appreciate that compositions in accordance with the invention that achieve transdermal administration of a therapeutic agent (e.g., botulinum toxin) may be incorporated into a device such as, for example, a patch. A variety of transdermal patch structures are known in the art; those of ordinary skill will appreciate that nanoparticle compositions may readily be incorporated into any of a variety of such structures. In some embodiments, a transdermal patch may further comprise a plurality of needles extending from one side of the patch that is applied to the skin, wherein needles extend from the patch to project through the stratum corneum of the skin. In some embodiments, needles do not rupture a blood vessel.

In some embodiments, a transdermal patch includes an adhesive. Some examples of adhesive patches are well known (for example, see U.S. Design Patent 296,006; and U.S. Patents 6,010,715; 5,591,767; 5,008,110; 5,683,712; 5,948,433; and 5,965,154). Adhesive patches are generally characterized as having an adhesive layer, which will be applied to a patient's skin, a depot or reservoir for holding a pharmaceutical agent, and an exterior surface that prevents leakage of the pharmaceutical from the depot. The exterior surface of a patch is typically non-adhesive.

In accordance with the present invention, a therapeutic agent (e.g., botulinum toxin) is incorporated into the patch so that it remains stable for extended periods of time. For example, a therapeutic agent may be present in a nanoparticle composition. Alternatively or additionally, a therapeutic agent may be incorporated into a polymeric matrix that stabilizes the agent, and permits the agent to diffuse from the matrix and the patch. A therapeutic agent may also be incorporated into the adhesive layer of the patch so that once the patch is applied to the skin, the agent may diffuse through the skin. In some embodiments, an adhesive layer may be heat-activated where temperatures of about 37 °C cause the adhesive to slowly liquefy so that the agent diffuses through the skin. The adhesive may remain tacky when stored at less than 37 °C, and once applied to the skin, the adhesive loses its tackiness as it liquefies. Administration of the therapeutic agent is complete once the patch no longer adheres to the skin.

In some embodiments, a therapeutic agent (e.g., botulinum toxin) can be provided in a depot in the patch so that pressure applied to the patch causes the therapeutic agent to be directed out of the patch (optionally through needles) and through the stratum corneum.

Suitable devices for use in delivering intradermal pharmaceutical compositions described herein include short needle devices such as those described in U.S. Patents 4,886,499; 5,190,521; 5,328,483; 5,527,288; 4,270,537; 5,015,235; 5,141,496; and 5,417,662. Intradermal compositions may be administered by devices which limit the effective penetration length of a needle into the skin, such as those described in PCT publication WO 99/34850 and functional equivalents thereof. Jet injection devices which deliver liquid vaccines to the dermis via a liquid jet injector and/or via a needle which pierces the stratum corneum and produces a jet which reaches the dermis are suitable. Jet injection devices are described, for example, in U.S. Patents 5,480,381; 5,599,302; 5,334,144; 5,993,412; 5,649,912; 5,569,189; 5,704,911; 5,383,851; 5,893,397; 5,466,220; 5,339,163; 5,312,335; 5,503,627; 5,064,413; 5,520,639; 4,596,556; 4,790,824; 4,941,880; 4,940,460; and PCT publications WO 97/37705 and WO 97/13537. Ballistic powder/particle delivery devices which use compressed gas to accelerate vaccine in powder form through the outer layers of the skin to the dermis are suitable. Alternatively or additionally, conventional syringes may be used in the classical mantoux method of intradermal administration.

Formulations suitable for topical administration include, but are not limited to, liquid and/or semi liquid preparations such as liniments, lotions, oil in water and/or water in oil emulsions such as creams, ointments and/or pastes, and/or solutions and/or suspensions. Topically-administrable formulations may, for example, comprise from about 1% to about 10% (w/w) active ingredient, although the concentration of the active ingredient may be as high as the solubility limit of the active ingredient in the solvent. Formulations for topical administration may further comprise one or more of the additional ingredients described herein.

Those of ordinary skill in the art will appreciate that a transdermal patch is but one example of a device with which nanoparticle compositions may be administered. To give but a few other examples, a device may be employed that allows the composition to be applied without first applying the composition to one's fingers, which may lead to undesirable paralysis of the fingers. Suitable devices include gloves, spatulas, swabs, syringes without needles, and adhesive patches. Use of spatulas, swabs, or the like may require the device to be inserted into a container containing the composition. Using syringes may be accomplished by filling the syringe with the composition. A composition may then be topically spread by spatulas or swabs, or may be expelled from the syringes onto the patient's skin.

In many embodiments, it may be desirable to limit delivery of a therapeutic agent (e.g., botulinum toxin) to only an intended delivery area. In some embodiments, such limited delivery may be accomplished by utilizing a nanoparticle composition in an application device that permits application of the composition to a target site on the skin without applying the composition to non-target site areas of the skin. Clearly, a transdermal patch may be utilized to this end. Alternatively or additionally, if a therapeutic agent is to be applied topically to only a selected area, other areas may be covered or pre-treated or otherwise protected from exposure.

General considerations in the formulation and/or manufacture of pharmaceutical agents may be found, for example, in Remington: The Science and Practice of Pharmacy 21st ed., Lippincott Williams & Wilkins, 2005.

The scope of the invention is defined by the claims. Any references in the description to methods of treatment refer to the compounds, pharmaceutical compositions and medicaments of the present invention for use in a method for treatment of the human (or animal) body by therapy (or for diagnosis).

### Treatment Applications of Nanoparticle Compositions

As described herein, the present invention provides treatment of conditions or disorders associated with the dermal level of the skin, including, but not limited to, acne, rosacea, excess sebum-producing disorders (*e.g.,* seborrhea, seborrheic dermatitis, *etc.*), via transdermal administration of one or more therapeutic agents (*e.g*., botulinum toxin) to a subject in the context of a nanoparticle composition. Such delivery is useful in a variety of contexts, including in particular certain cosmetic and medical applications. Certain such applications are discussed in more detail below.

### The Dermis

In some embodiments, the present invention provides methods of treating conditions, diseases, and/or disorders that involve the layer of the skin called the "dermis." In general, the dermis is the layer of skin beneath the epidermis that contains connective tissue and cushions the body from stress and strain. The dermis is tightly connected to the epidermis by a basement membrane and harbors nerve endings that provide the sense of touch and heat. The dermis contains hair follicles, sweat glands, sebaceous glands, apocrine glands, lymphatic vessels and blood vessels. Blood vessels in the dermis provide nourishment and waste removal to its own cells as well as to the Stratum basale of the epidermis.

The dermis is structurally divided into two areas: a superficial area adjacent to the epidermis, called the papillary region, and a deep thicker area known as the reticular region. The papillary region comprises loose areolar connective tissue. It is named for its fingerlike projections (called papillae) that extend toward the epidermis. Papillae provide the dermis with a "bumpy" surface that interdigitates with the epidermis, strengthening the connection between the two layers of skin. Also located within the reticular region are the roots of the hair, sebaceous glands, sweat glands, receptors, nails, and blood vessels.

The dermis comprises sebaceous glands, which secrete an oily substance called sebum that comprises lipids and debris of dead fat-producing cells. Sebum typically comprises about 25% wax monoesters, about 41% triglycerides, about 16% free fatty acids, and about 12% squalene. In sebaceous glands, sebum is produced within specialized cells and is released as these cells burst; sebaceous glands are thus classified as holocrine glands. Sebum acts to protect and waterproof hair and skin, and keeps them from becoming dry, brittle, and cracked. It can also inhibit the growth of microorganisms on skin. Sebum itself is odorless, but its bacterial breakdown can produce odors. Sebum is one cause of some people experiencing "oily" hair or skin if not washed for several days. Sebum is also found in earwax.

The dermis comprises two different types of sweat glands: apocrine sweat glands and merocrine sweat glands. Both gland types contain myoepithelial cells, specialized epithelial cells located between the gland cells and the underlying basal lamina. Myoepithelial cell contractions squeeze the gland and discharge any accumulated secretions. The secretory activities of gland cells and the contractions of myoepithelial cells are controlled by both the autonomic nervous system and by circulating hormones.

Apocrine sweat glands develop during the early- to mid-puberty ages within the age range of 13 to 15, and release more than normal amounts of sweat for approximately a month, regulating and releasing normal amounts of sweat after a certain period of time. These glands produce sweat that contains organic molecules (lipids and proteins) and pheromones. Mainly present on the face, in the armpits, and around the genital area, their activity is the main cause of sweat odor, due to the bacteria that break down the organic compounds in the sweat.

The name apocrine sweat gland is archaic; these glands are no longer believed to secrete their products by an apocrine mechanism in which the apical portion of the cell is sloughed off with secretory products inside. Rather, apocrine sweat glands secrete in an merocrine fashion: membrane-bound vesicles bind to the plasma membrane of secretory cells and release products by exocytosis with no net loss of the plasma membrane. These glands are still called apocrine sweat glands to distinguish them from the merocrine (eccrine) sweat glands.

Merocrine sweat glands (eccrine sweat glands) are far more numerous and widely distributed than apocrine sweat glands is the merocrine sweat glands. The adult integument contains around 3 million merocrine glands. They are smaller than apocrine sweat glands, and they do not extend as far into the dermis. Palms and soles have the highest numbers; estimates are that the palm of the hand has about 500 glands per square centimeter (3000 glands per square inch). Merocrine sweat glands are coiled tubular glands that discharge their secretions directly onto the surface of the skin.

The dermis comprises hair follicles, which are attached to sebaceous glands. Also attached to the follicle is a tiny bundle of muscle fiber ("arrector pili") that is responsible for causing the follicle lissis to become more perpendicular to the surface of the skin, and causing the follicle to protrude slightly above the surrounding skin (piloerection), resulting in goose bumps.

Hair follicles are structures that support hair growth by packing old cells together. At the base of a hair follicle is a large structure that is called the papilla, which is made up mainly of connective tissue and a capillary loop. Around the papilla is the hair matrix, a collection of epithelial cells often interspersed with melanocytes. Cell division in the hair matrix is responsible for the cells that will form the major structures of the hair fiber and the inner root sheath. The papilla is usually ovoid or pear shaped with the matrix wrapped completely around it except for a short stalk-like connection to the surrounding connective tissue that provides access for the capillary. The root sheath is composed of an external root sheath ("Henle's layer"), a middle layer ("Huxley's layer"), and an internal cuticle that is continuous with the outermost layer of the hair fiber. The hair fiber is composed of a cuticle that is continuous with the root sheath, an intermediate cortex, and an inner medulla.

Hair grows in cycles of various phases: anagen (growth phase), catagen (involuting or regressing phase), and telogen (quiescent phase). Each phase has several morphologically and histologically distinguishable sub-phases. Prior to the start of cycling is a phase of follicular morphogenesis, and there is also a shedding phase ("exogen") in which a hair exits the follicle. Typically up to 90% of the hair follicles are in anagen phase while, 10-14% are in telogen and 1-2% in catagen. The cycle's length varies on different parts of the body. For example, the cycle for eyebrows takes around 4 months, while the cycle for the scalp takes 3 to 4 years to finish. Growth cycles are controlled by a chemical signal like epidermal growth factor

Botulinum toxin A (BTXA) has become a widely used drug in cosmetic dermatology. Adverse effects of BTXA observed with cosmetic use can have a significant impact on patient health and appearance and can deter patient use and/or repeat use. Currently, BTXA is administered by medical personnel and in a clinical setting both because BTXA is administered by injection, which requires trained personnel, and because the major tools for preventing adverse effects from BTXA are knowledge and skill. Use of correct injection techniques is mandatory since most unwanted effects are caused by incorrect technique. Knowledge of human anatomy, (*i.e.,* for example, facial and extrafacial muscles, location and depth of glands, *etc.*), is important for physicians to select the optimal dose, time and technique.

The most common adverse effects of current procedures for administering BTXA are pain and hematoma. When BTXA solution is administered by injection to the periocular region, eyelid and brow ptosis are common adverse effects. Adverse effects such as pain, hematoma, ecchymosis, and bruising may also occur in the upper and lower face and at extrafacial sites. Other possible adverse effects include, but are not limited to, headache and possible interaction with concomitant medications. Suggestions have been made to avoid the most unwanted adverse effects by implementing the proper techniques of dilution, storage, and injection, as well as the careful exclusion of patients with any contraindications. Pain, hematoma, ecchymosis, and bruising can be prevented by cooling the skin before and after BTXA injection. Upper lid ptosis may be partly corrected using apraclonidine or phenylephrine eyedrops (Wollina et al., 2005, Am. J. Clin. Dermatol., 6:141). However, significant adverse effects remain with current strategies.

By contrast, the present invention provides methods and compositions for safely and effectively administering therapeutic agents, such as botulinum toxins, in a manner that minimizes adverse side effects. In some embodiments, topical administration of a therapeutic agent reduces unwanted side effects by about 50%, about 60%, about 70%, about 80%, about 90%, about 95%, about 98%, about 99%, or about 100% relative to non-topical administration (*e.g.,* injection, oral administration, *etc.*) of the same therapeutic agent. For example, in some embodiments, topical administration of a therapeutic agent (*e.g*., botulinum toxin) reduces pain, bruising, ecchymosis, and/or hematoma by about 50%, about 60%, about 70%, about 80%, about 90%, about 95%, about 98%, about 99%, or about 100% relative to injection of the same therapeutic agent.

In some embodiments, topical administration of a therapeutic agent reduces unwanted systemic effects by about 50%, about 60%, about 70%, about 80%, about 90%, about 95%, about 98%, about 99%, or about 100% relative to non-topical administration (*e.g.,* injection, oral administration, *etc.*) of the same therapeutic agent. In some embodiments, topical administration of a therapeutic agent reduces undesirable blood levels by about 50%, about 60%, about 70%, about 80%, about 90%, about 95%, about 98%, about 99%, or about 100% relative to non-topical administration (*e.g*., injection, oral administration, *etc.*) of the same therapeutic agent. For example, in specific embodiments, topical administration of botulinum toxin reduces the incidence and/or severity of botulism by about 50%, about 60%, about 70%, about 80%, about 90%, about 95%, about 98%, about 99%, or about 100% relative to injection of botulinum toxin. In specific embodiments, topical administration of botulinum toxin reduces delivery of the therapeutic agent to the bloodstream by about 50%, about 60%, about 70%, about 80%, about 90%, about 95%, about 98%, about 99%, or about 100% relative to injection of botulinum toxin.

In some embodiments, topical administration of a therapeutic agent reduces damage to underlying nervous tissue (e.g., neuronal paralysis) by about 50%, about 60%, about 70%, about 80%, about 90%, about 95%, about 98%, about 99%, or about 100% relative to non-topical administration *(e.g.,* injection, oral administration, *etc.)* of the same therapeutic agent. In some embodiments, topical administration of a therapeutic agent reduces unwanted effects on muscles (*e.g*., muscle paralysis) by about 50%, about 60%, about 70%, about 80%, about 90%, about 95%, about 98%, about 99%, or about 100% relative to non-topical administration (*e.g.,* injection, oral administration, *etc.*) of the same therapeutic agent.

In some embodiments, the present invention provides methods and compositions for specific delivery of therapeutic agents to dermal structures. In some embodiments, therapeutic agents are specifically delivered to dermal structures without significant delivery to subdermal structures. In some embodiments, greater than about 50%, greater than about 60%, greater than about 70%, greater than about 80%, greater than about 85%, greater than about 90%, greater than about 95%, greater than about 96%, greater than about 97%, greater than about 98%, greater than about 99%, greater than about 99.5%, or about 100% of a therapeutic agent administered to the skin of a subject is delivered specifically to the dermis. In some embodiments, less than about 50%, less than about 40%, less than about 30%, less than about 20%, less than about 10%, less than about 5%, less than about 4%, less than about 3%, less than about 2%, less than about 1%, less than about 0.5%, or less than about 0.1% of a therapeutic agent administered to the skin of a subject is delivered to subdermal structures.

In some embodiments, specific delivery to dermal structures is achieved through application of a dose of therapeutically active agent that is lower than a dose per area used to achieve delivery to subdermal structures. For example, in some embodiments, a volume of nanoparticle composition is applied to a larger surface area; in some embodiments, a nanoparticle composition containing a reduced amount of therapeutic agent per unit volume of composition is utilized; in some embodiments, penetration of the therapeutic agent and/or the nanoparticle composition into the skin is reduced (*e.g*., through combination with penetration inhibitors and/or adjustment of nanoparticle composition characteristics such as particle size, component ratios, component identity, *etc.,* and combinations thereof). In some embodiments, the lower dose is at least about 2-fold, about 3-fold, about 4-fold, about 5-fold, about 10-fold, about 20-fold, about 30-fold, about 40-fold, about 50-fold, about 100-fold, or greater than about 100-fold lower than a dose per area used to achieve delivery to subdermal structures.

In some embodiments, the present invention contemplates method of administration of therapeutic agents as a topically and/or locally delivered composition comprising a nanoparticle composition such as a nanoemulsion (*e.g*., microfluidized nanoemulsion). In some embodiments, the composition is formulated as a cream, ointment, oil, foam, spray, lotion, liquid, powder, thickening lotion, or gel. Formulations comprising nanoparticle compositions can contain water and also any cosmetically acceptable solvent, in particular, monoalcohols, such as alkanols having 1 to 8 carbon atoms (like ethanol, isopropanol, benzyl alcohol and phenylethyl alcohol), polyalcohols, such as alkylene glycols (like glycerine, ethylene glycol and propylene glycol), and glycol ethers, such as mono-, di-, and tri-ethylene glycol monoalkyl ethers, for example, ethylene glycol monomethyl ether and diethylene glycol monomethyl ether, used singly or in a mixture. Such components can be present, for example, in proportions of up to as much as 70% by weight, relative to the weight of the total composition.

Formulations including nanoparticle compositions may contain at least one filler, for example, in order to obtain a matte product, which may be especially desired for individuals with greasy skin. The term "filler" means any particle that is solid at room temperature and atmospheric pressure, used alone or in combination, which does not react chemically with the various ingredients of the composition and which are insoluble in these ingredients, even when these ingredients are brought to a temperature above room temperature and especially to their softening point or to their melting point. Such inert fillers typically have melting points at least higher than 170 °C, higher than 180 °C, higher than 190 °C, or higher than 200 °C.

Fillers may be absorbent or nonabsorbent, *i.e.,* capable in particular of absorbing the oils of the composition and also the biological substances secreted by the skin. In some embodiments, fillers are particulate and have an apparent diameter ranging from 0.01 µm to 150 µm, from 0.5 µm to 120 µm, or from 1 µm to 80 µm. An apparent diameter corresponds to the diameter of the circle in which the elementary particle is inscribed along its smallest dimension (thickness for lamellae).

Pharmaceutical compositions are described in further detail in the section entitled "Pharmaceutical Compositions."

### Acne Vulgaris

In some embodiments, nanoparticle compositions may comprise at least one therapeutic agent that is useful for treating acne vulgaris (commonly referred to as "acne"), a skin disease caused by changes in the pilosebaceous units (*i.e.,* skin structures comprising a hair follicle and its associated sebaceous gland). In some embodiments, acne is inflammatory. In some embodiments, acne is noninflammatory. While not life-threatening, acne vulgaris can cause significant problems for affected individuals. Depending on its severity and other factors, recalcitrant acne can be psychologically debilitating, and can impose significant financial and emotional costs on those whom it afflicts. Despite some recent successes in acne therapy, treatment failures are still common, especially in adult women. While many adults "outgrow" this disease, there are some who continue to be afflicted during much of adulthood, despite continued medical advances. Unfortunately, the most potent acne medication in current use is administered systemically via a treatment that is teratogenic, an important issue for many women. There is an unfilled need for a more localized and effective treatment for acne, one with minimal side effects.

In general, acne develops as a result of blockages in follicles. The pathology centers on the pilosebaceous units, comprising a sebaceous gland, a follicle (*i.e.,* pore), and a vellus hair. Among the first events leading to acne are hyperkeratinization and formation of a plug of keratin and sebum (a "microcomedo"), obstructing the upper region of a follicle. Enlargement of sebaceous glands and an increase in sebum production occur with increased androgen production at adrenarche. A microcomedo may enlarge to form an open comedo (a "blackhead") or closed comedo (a "whitehead"). In these conditions the naturally occurring largely commensual bacteria *Propionibacterium acnes* can cause inflammation, leading to inflammatory lesions (papules, infected pustules, or nodules) in the dermis around the microcomedo or comedo, which results in redness and may result in scarring or hyperpigmentation.

Adolescence is marked by an increase in levels of circulating androgens, particularly dehydroepiandrosterone sulfate (DHEAS). Increased androgen levels are thought to cause sebaceous glands to enlarge and to increase sebum production. While most acne patients have normal hormone levels, there are reasons to conclude that increased sebum production plays a role in acne. For example, there may be a correlation between the rate of sebum production and the severity of acne. In addition, acne patients typically produce sebum that is deficient in linoleic acid, which is a potential cause of abnormal keratinization and follicular obstruction.

In response to increased sebum levels, *Propionibacterium acnes,* a relatively slow growing, typically aerotolerant anaerobic gram positive, diphtheroid bacterium, often colonizes the sebaceous follicles. *P. acnes* exacerbates acne by acting as a chemo-attractant for neutrophils. Neutrophils ingest *P. acnes,* and in doing so release various hydrolytic enzymes that damage the follicular wall. Released follicular contents then invade the dermis and cause an inflammatory reaction, manifesting as pustules, erythematous papules, or nodules. In a separate route, *P. acnes* can hydrolyze triglycerides to free fatty acids, which also increase inflammation and follicular obstruction. *P. acnes* may also activate the complement components of the immune system, which can also lead to follicular obstruction.

Follicles are lined with squamous epithelium, a layer of cells that is contiguous with the skin surface. In an acne-prone individual, the shedding of cells from this lining is often impeded, perhaps due to an increased level of intercellular adhesion that promotes the retention of cells. Retained cells can obstruct follicles, resulting in comedones. Such inhibited shedding may be related to abnormalities in epidermal differentiation and/or to abnormal sebum composition (e.g., a deficiency in linoleic acid). It has also been demonstrated that increased sebum levels can irritate keratinocytes, causing the release of interleukin-1, which in turn can cause follicular hyperkeratinization. In general, each of these acne-causing routes, which are not mutually exclusive, are associated with follicular obstruction.

Several factors are known to be linked to acne, including, but not limited to, family and/or genetic history (see, *e.g.,* Ballanger et al., 2006, Dermatology, 212:145-149); hormonal activity (*e.g.,* menstrual cycles, puberty, *etc.*); stress (*e.g.,* through increased output of hormones from the adrenal glands); hyperactive sebaceous glands; accumulation of dead skin cells; bacteria in the pores (*e.g., P. acnes*); skin irritation or scratching; use of anabolic steroids; use of medications containing halogens (*e.g*., iodides, chlorides, bromides), lithium, barbiturates, or androgens; exposure to certain chemical compounds (*e.g*., dioxins such as chlorinated dioxins); exposure to testosterone, dihydrotestosterone (DHT), dehydroepiandrosterone sulfate (DHEAS), and/or insulin-like growth factor 1 (IGF-I); diet including milk and/or high levels of carbohydrate; low levels of vitamins A and/or E; poor hygiene; or any combinations thereof.

In some embodiments, acne treatments work via one or more of the following mechanisms: (1) normalizing shedding into the pore to prevent blockage; (2) killing *P. acnes;* (3) having antinflammatory activity; and/or (4) manipulating hormone levels.

The present invention provides methods of treating acne comprising topical administration of a nanoparticle composition comprising at least one therapeutic agent to a subject suffering from, susceptible to, and/or displaying symptoms of acne. In some embodiments, such a nanoparticle composition is administered locally to an affected site (*e.g.,* face, neck, back, arms, chest, *etc.*). In some embodiments, nanoparticle compositions for treatment of acne are formulated into a cream, lotion, gel, sunscreen, *etc.* Further considerations for formulation and administration are described in further detail in the sections entitled "Pharmaceutical Compositions" and "Administration."

In some embodiments, botulinum toxin (*e.g*., administered in the context of a nanoparticle composition) can be utilized to treat acne. Injected botulinum toxin has been reported to alleviate the appearance of acne. For example, see U.S. Patent 7,226,605. However, as discussed herein, there are numerous negative side effects associated with botulinum toxin injection. The present invention contemplates administration of a botulinum nanoparticle composition such as a botulinum toxin nanoemulsion (*e.g*., a microfluidized botulinum toxin nanoemulsion) to a patient exhibiting symptoms of acne.

In some embodiments, nanoparticle compositions may comprise any therapeutic agent that is useful for treatment of acne, including all therapeutic agents for acne listed in the section entitled *"Therapeutic Agents."* In some embodiments, such agents include, but are not limited to, cleansers or soaps; topical bactericidals (*e.g*., benzoyl peroxide, triclosan, chlorhexidine gluconate, *etc.*); topical antibiotics *(e.g.,* externally-applied erythromycin, clindamycin, tetracycline, *etc.*); oral antibiotics (*e.g.,* erythromycin, tetracycline, oxytetracycline, doxycycline, minocycline, lymecycline, trimethoprim, *etc.*); hormonal treatments (*e.g*., estrogen/progestogen oral contraceptives, low dose spironolactone, cortisone, *etc.*); topical retinoids (*e.g.,* tretinoin [RETIN-A®], adapalene [DIFFERIN®], tazarotene [TAZORAC®], retinol, isotretinoin, *etc.*); oral retinoids (*e.g.,* isotretinoin [ACCUTANE®, AMNESTEEM™, SOTRET™, CLARAVIS™]); herbs (*e.g.,* aloe vera; aruna, haldi [turmeric], papaya, *etc.*); azelaic acid; anti-inflammatory agents (*e.g.,* naproxen, ibuprofen, rofecoxib [Tehrani and Dharmalingam, 2004, Indian J. Dermatol. Venereol. Leprol., 70:345-348], *etc.*); nicotinamide [vitamin B3]; tea tree oil [melaleuca oil]; rofecoxib; zinc (Dreno et al., 1989, Acta Derm. Venereol., 69:541-3; and Dreno et al., 2001, Dermatology, 203:135-40; and/or combinations thereof; as described in further detail in the section entitled *"Therapeutic Agents."*

It will be appreciated that nanoparticle compositions in accordance with the present invention can be employed in combination therapies. In some embodiments, the present invention encompasses "therapeutic cocktails" comprising nanoparticle compositions. The particular combination of therapies (therapeutics or procedures) to employ in a combination regimen will take into account compatibility of the desired therapeutics and/or procedures and the desired therapeutic effect to be achieved. In some embodiments, pharmaceutical compositions comprising one or more nanoparticle compositions can be administered concurrently with, prior to, or subsequent to, one or more other desired pharmaceutical agents and/or procedures. In some embodiments, nanoparticle compositions themselves may comprise one or more additional therapeutic agents, as described herein.

The particular combination of therapies to employ in a combination regimen will generally take into account compatibility of the desired therapeutics and/or procedures and the desired therapeutic effect to be achieved. It will be appreciated that the therapies employed may achieve a desired effect for the same disorder (for example, a nanoparticle composition may be administered concurrently with another agent that is useful for treating a disorder associated with the dermal level of the skin, *etc.),* or they may achieve different effects (for example, a nanoparticle composition may be administered concurrently with another agent that is useful for alleviating any adverse side effects of the nanoparticle composition). In some embodiments, compositions in accordance with the invention are administered with a second therapeutic agent that is approved by the U.S. Food and Drug Administration (FDA).

By "in combination with," it is not intended to imply that the agents must be administered at the same time and/or formulated for delivery together, although these methods of delivery are within the scope of the invention. In some embodiments, therapeutic agents utilized in combination are administered together in a single composition. In some embodiments, therapeutic agents utilized in combination are administered separately in different compositions. Compositions can be administered concurrently with, prior to, or subsequent to, one or more other desired therapeutics or medical procedures. In general, each agent will be administered at a dose and/or on a time schedule determined for that agent. Additionally, the invention encompasses the delivery of nanoparticle compositions in combination with agents that may improve their bioavailability, reduce and/or modify their metabolism, inhibit their excretion, and/or modify their distribution within the body.

In general, it is expected that agents utilized in combination will be utilized at levels that do not exceed the levels at which they are utilized individually. In some embodiments, levels utilized in combination will be lower than those utilized individually.

Pharmaceutical compositions in accordance with the present invention may be administered alone and/or in combination with other agents that are used to treat the symptoms and/or causes of acne, such as the agents described above. Pharmaceutical compositions in accordance with the present invention may be administered alone and/or in combination with procedures that are used to treat the symptoms and/or causes of acne. In some embodiments, such procedures include, but are not limited to, phototherapy (e.g., alternating blue and red light); photodynamic therapy (e.g., intense blue/violet light); laser treatment (e.g., to burn away the follicle sac from which the hair grows; to burn away the sebaceous gland which produces the oil; and/or to induce formation of oxygen in the bacteria, killing them); local heating; and/or combinations thereof.

In some embodiments, nanoparticle compositions comprising therapeutic agents useful for treatment of acne may be administered to a subject in combination with phototherapy for treatment of acne. It is known in the art that short-term improvement of acne can be achieved with sunlight, but studies have shown that sunlight worsens acne long-term. More recently, visible light has been successfully employed to treat acne (*i.e.,* "phototherapy") - in particular, intense violet light (405 nm - 420 nm) generated by purpose-built fluorescent lighting, dichroic bulbs, LEDs, and/or lasers. Used twice weekly, this has been shown to reduce the number of acne lesions by about 64% (Kawada et al., 2002, J. Dermatol. Sci., 30:129-35) and is even more effective when applied daily. Without wishing to be bound by any one theory, a porphyrin (Coproporphyrin III) produced within *P. acnes* generates free radicals when irradiated by 420 nm and shorter wavelengths of light (Kjeldstad, 1984, Z. Naturforsch [C], 39:300-2). Particularly when applied over several days, these free radicals ultimately kill bacteria (Ashkenazi et al., 2003, FEMS Immunol. Med. Microbiol., 35:17-24). Since porphyrins are not otherwise present in skin, and no ultraviolet (UV) light is employed, it appears to be safe, and has been licensed by the U.S. FDA. The treatment apparently works even better if used with red visible light (about 660 nm), resulting in a 76% reduction of lesions after 3 months of daily treatment for 80% of the patients (Papageorgiou et al., 2000, Br. J. Dermatol., 142:973-8Unlike most of other treatments, few negative side effects are typically experienced, and development of bacterial resistance to the treatment seems very unlikely. After treatment, clearance can be longer lived than is typical with topical or oral antibiotic treatments (e.g., may be up to several months).

In some embodiments, nanoparticle compositions comprising therapeutic agents useful for treatment of acne may be administered to a subject in combination with photodynamic therapy for treatment of acne. There is some evidence that intense blue/violet light (405 nm - 425 nm) can decrease the number of inflammatory acne lesion by 60% - 70% in 4 weeks of therapy, particularly when *P. acnes* is pretreated with delta-aminolevulinic acid (ALA), which increases the production of porphyrins.

In some embodiments, nanoparticle compositions comprising therapeutic agents useful for treatment of acne may be administered to a subject in combination with laser treatment for treatment of acne. Laser surgery has been in use for some time to reduce the scars left behind by acne, but research has been done on lasers for prevention of acne formation itself. In general, laser is used to burn away the follicle sac from which the hair grows, to burn away the sebaceous gland which produces the oil, and/or to induce formation of oxygen in the bacteria, thereby killing them.

In some embodiments, nanoparticle compositions comprising therapeutic agents useful for treatment of acne may be administered to a subject in combination with local heating for treatment of acne. In some cases, local heating may be used to kill bacteria in a developing pimple, thereby expediting healing.

In some embodiments, the present invention involves administration of at least one therapeutic agent in a nanoparticle composition in an amount sufficient to achieve a reduction in the severity and/or prevalence of acne of at least about 25%; in some embodiments in an amount sufficient to achieve a reduction in the severity and/or prevalence of acne of at least about 30%; in some embodiments in an amount sufficient to achieve a reduction in the severity and/or prevalence of acne of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more.

### Rosacea

Rosacea is a condition that is estimated to affect over 45 million people worldwide. Rosacea affects both sexes, but is almost three times more common in women, and has a peak age of onset between 30 and 60. It begins as erythema (*i.e.,* flushing and redness) on the central face and across the cheeks, nose, and/or forehead but can also less commonly affect the neck and chest. As rosacea progresses, other symptoms can develop such as one or more of semi-permanent erythema, telangiectasia (*i.e.,* dilation of superficial blood vessels on the face), red domed papules and pustules, red gritty eyes, burning and stinging sensations, and/or rhinophyma (*i.e.,* a red lobulated nose).

There are four main subtypes of rosacea. "Erythematotelangiectatic rosacea" is characterized by permanent redness with a tendency to flush and blush easily. It is also common to have small blood vessels visible near the surface of the skin (*i.e.,* telangiectasias) and/or burning or itching sensations. "Papulopustular rosacea" is characterized by some permanent redness with papules and/or pustules, which typically last 1 to 4 days. This subtype is commonly confused with acne. "Phymatous rosacea" is most commonly associated with rhinophyma, an enlargement of the nose. Symptoms include thickening skin, irregular surface nodularities, and enlargement. Phymatous rosacea can also affect the chin (gnatophyma), forehead (metophyma), cheeks, eyelids (blepharophyma), and/or ears (otophyma) (see, *e.g.,* Jansen and Plewig, 1998, Facial Plast. Surg., 14:241). Small blood vessels visible near the surface of the skin (*i.e.,* telangiectasias) may be present. "Ocular rosacea" is characterized by red, dry, irritated eyes and/or eyelids. Other symptoms may include foreign body sensations, itching, and/or burning.

Rosacea can be triggered by a variety of stimuli. Triggers that cause episodes of flushing and blushing play a part in the development of rosacea, such as exposure to temperature extremes, strenuous exercise, heat from sunlight, severe sunburn, stress, anxiety, cold wind, and/or moving to a warm or hot environment from a cold one. Some foods and drinks can trigger flushing, such as alcohol, foods and beverages containing caffeine (*e.g.,* hot tea, coffee), foods high in histamines, and spicy foods. Certain medications and topical irritants can quickly progress rosacea (*e.g*., steroids, benzoyl peroxide, isotretinoin.)

The present invention provides methods of treating rosacea comprising topical administration of a nanoparticle composition comprising at least one therapeutic agent to a subject suffering from, susceptible to, and/or displaying symptoms of rosacea. In some embodiments, a therapeutic agent in the context of a nanoparticle composition is administered locally to an affected site *(e.g.,* cheeks, nose, forehead, ears, neck, chest, *etc.).* In some embodiments, nanoparticle compositions for treatment of rosacea are formulated into a cream, lotion, gel, sunscreen, *etc.* Further considerations for formulation and administration are described in further detail in the sections entitled "Pharmaceutical Compositions" and "Administration."

In some embodiments, different subtypes of rosacea are treated differently from other subtypes of rosacea (Cohen and Tiemstra, 2002, J. Am. Board Fam. Pract., 15:214). In some embodiments, different subtypes of rosacea are not treated differently from other subtypes of rosacea.

In some embodiments, nanoparticle compositions may comprise any therapeutic agent that is useful for treatment of rosacea, including all therapeutic agents for rosacea listed in the section entitled *"Therapeutic Agents."* In some embodiments, such agents include, but are not limited to, oral antibiotics (e.g., tetracycline, doxycycline, minocycline, metronidazole, macrolide antibiotics, *etc.).* In some embodiments, oral antibiotics may be administered at anti-inflammatory doses (e.g., about 40 mg/day) or at higher doses. In some embodiments, agents for combination therapy may include oral isotretinoin. In some embodiments, agents for combination therapy may include topical antibiotics (*e.g.,* metronidazole, clindamycin, erythromycin, *etc.*); topical azelaic acid (*e.g.,* FINACEA™, AZELEX™, FINEVIN®, SKINOREN, *etc.*); topical sulfacetamide; topical sulfur; topical calcineurin inhibitori (*e.g.,* tacrolimus, pimecrolimus, *etc.*); topical benzoyl peroxide; topical permethrin; a combination of plant-sourced methylsulfonylmethane (MSM) and Silymarin; and/or combinations thereof. Any combination of the foregoing may be utilized, and such therapeutic agents are described in further detail in the section entitled *"Therapeutic Agents."*

It will be appreciated that nanoparticle compositions in accordance with the present invention can be employed in combination therapies, as described above for the treatment of acne. Pharmaceutical compositions in accordance with the present invention may be administered alone and/or in combination with other agents that are used to treat the symptoms and/or causes of rosacea, such as those described above. Pharmaceutical compositions in accordance with the present invention may be administered alone and/or in combination with procedures that are used to treat the symptoms and/or causes of rosacea. In some embodiments, such procedures include, but are not limited to, use of a gentle skin cleansing regimen using non-irritating cleansers; protecting skin from the sun by covering skin with clothing; applying sunscreen to exposed skin; dermatological vascular laser (single wavelength); intense pulsed light (broad spectrum); carbon dioxide lasers; low level light therapies; and/or combinations thereof.

Rosacea may be treated via dermatological vascular laser (single wavelength) and/or intense pulsed light (broad spectrum) (Angermeier, 1999, J. Cutan. Laser Ther., 1:95). These methods use light to penetrate the epidermis to target the capillaries in the dermis. Light is absorbed by oxy-hemoglobin, thereby causing capillary walls to heat up to 70 °C, damaging them, which causes them to be absorbed by the body's natural defense mechanism. These methods may be successful for eliminating redness altogether, though additional periodic treatments might be necessary to remove newly-formed capillaries. Alternatively or additionally, a 595 nm long pulse-duration pulsed-dye laser may be useful for the treatment of rosacea (Kligman and Bernstein, 2008, Lasers Surg. Med., 40:233).

Alternatively or additionally, carbon dioxide lasers can be used to remove excess tissue caused by phymatous rosacea. Carbon dioxide lasers emit a wavelength that is absorbed directly by the skin. The laser beam can be focused into a thin beam and used as a scalpel or defocused and used to vaporize tissue.

In some embodiments, rosacea can be treated using low level light therapies.

In some embodiments, the present invention involves administration of at least one therapeutic agent (*e.g.,* botulinum toxin) in a nanoparticle composition, in an amount sufficient to achieve a reduction in the degree and/or prevalence of one or more symptoms of rosacea of at least about 25%; in some embodiments in an amount sufficient to achieve a reduction in the degree and/or prevalence of one or more symptoms of rosacea of at least about 30%; in some embodiments in an amount sufficient to achieve a reduction in the degree and/or prevalence of one or more symptoms of rosacea of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more.

### Excess Sebum-Producing Disorders

Excess sebum-producing disorders (*e.g.,* seborrhea, seborrheic dermatitis, *etc.*) are disorders affecting the areas of the skin that are rich in sebum glands, which typically include the scalp, face, and/or trunk. Patients with these conditions typically have scaly, flaky, erythematous, and often pruritic skin. Involvement of the scalp can result in hair loss. In some cases, the skin is also oily.

Pharmaceutical compositions in accordance with the present invention may be administered alone and/or in combination with other agents that are used to treat the symptoms and/or causes of excess sebum-producing disorders, as described above for the treatment of excess sebum-producing disorders. In some embodiments, such agents include botulinum toxin, salicylic acid, azelaic acid, selnium sulfide, imidazoles (*e.g.,* ketoconazole, miconazole, fluconazole, econazole, bifonazole, climazole, ciclopirox, ciclopiroxolamine, *etc.),* itraconazole, terbinafine, zinc pyrithione, benzoyl peroxide, coal tar, juniper tar, glucocorticosteroids (*e.g.,* hydrocortisone, *etc.*), metronidazole, lithium, calcineurin inhibitors (*e.g.,* tacrolimus, pimecrolimus, *etc.*), Vitamin D3, isotretinoin, and/or combinations thereof.

In some embodiments, the present invention involves administration of at least one therapeutic agent (*e.g*., botulinum toxin) in a nanoparticle composition, in an amount sufficient to achieve a reduction in the degree and/or prevalence of one or more symptoms of excess sebum production of at least about 25%; in some embodiments in an amount excess sebum production to achieve a reduction in the degree and/or prevalence of one or more symptoms of excess sebum production of at least about 30%; in some embodiments in an amount sufficient to achieve a reduction in the degree and/or prevalence of one or more symptoms of excess sebum production of at least about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more.

### Kits

In some embodiments, the present invention provides pharmaceutical packs or kits including nanoparticle compositions antigens according to the present invention. In certain embodiments, pharmaceutical packs or kits include preparations or pharmaceutical compositions containing nanoparticle compositions in one or more containers filled with optionally one or more additional ingredients of pharmaceutical compositions in accordance with the invention. In certain embodiments, the pharmaceutical pack or kit includes an additional approved therapeutic agent (*e.g.,* benzoyl peroxide for treatment of acne; aluminum compounds for treatment of hyperhidrosis; *etc.*) for use in combination therapies. Optionally associated with such container(s) can be a notice in the form prescribed by a governmental agency regulating the manufacture, use or sale of pharmaceutical products, which notice reflects approval by the agency of manufacture, use, or sale for human administration.

Kits are provided that include therapeutic reagents. As but one non-limiting example, nanoparticle compositions can be provided as topical formulations and administered as therapy. Pharmaceutical doses or instructions therefor may be provided in a kit for administration to an individual suffering from or at risk for conditions or disorders associated with the dermal level of the skin, including, but not limited to, acne, hyperhidrosis, bromhidrosis, chromhidrosis, rosacea, hair loss, actinic keratosis, psoriasis, eczematous dermatitis (*e.g.,* atopic dermatitis, *etc.*), excess sebum-producing disorders (*e.g.,* seborrhea, seborrheic dermatitis, *etc.*), Raynaud's phenomenon, lupus erthythematosus, hyperpigmentation disorders (*e.g.,* melasma, *etc.*), hypopigmentation disorders (*e.g.,* vitiligo, *etc.*), skin cancer (*e.g.,* squamous cell skin carcinoma, basal cell skin carcinoma, *etc.*) and/or dermal infection (*e.g*., fungal infection, herpes simplex virus infection, human papillomavirus infection, *etc.*).

In some embodiments, a kit may comprise (i) a nanoparticle composition (*e.g*. nanoemulsion); and (ii) at least one pharmaceutically acceptable excipient; and optionally (iii) at least one syringe, spatula, swab for administration to skin; and (iv) instructions for use.

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. The following examples contain information, exemplification and guidance, which can be adapted to the practice of this invention in its various embodiments and the equivalents thereof.

### Exemplification

The following examples are only intended to provide illustrations of specific embodiments contemplated by the present invention. The examples are not intended in any way to be limiting.

### Example 1: Botulinum Nanoemulsion Formulation

This example presents one embodiment of nanoemulsion prepared by microfluidization comprising botulinum toxin (*i.e.,* for example, botulinum toxin type A, whether complexed or isolated).

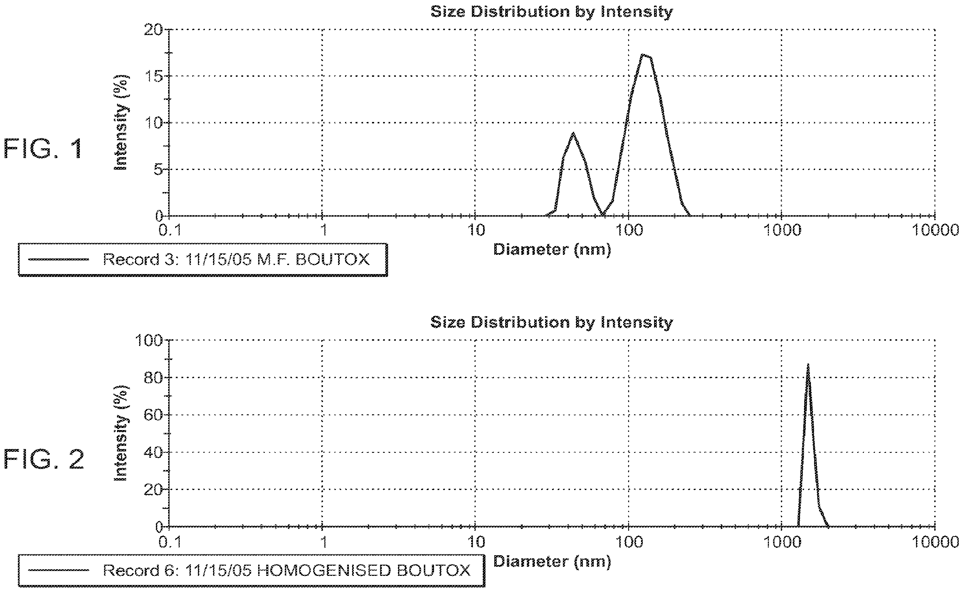

A preparation for micro fluidization was made as follows:
1. 5 g of soybean oil and 5 g of Tween 80 were mixed, heating as needed (typically not required) to emulsify the mixture.
2. 100 Units of botulinum toxin type A, was added to 100 mL of deionized/distilled water and stirred until evenly mixed.
3. Step 1 preparation was added to Step 2 preparation and stirred until evenly mixed.
4. Preparation was homogenized for 1 minute (see resulting particle distributions in Table 1 and Figure 1)
5. Single-pass micro fluidization procedure at 21,000 psi was performed using a Microfluidizer® Processor.

The resulting nanoemulsion was evaluated for particle size using the Malvern Nano S particle sizer capable of sizing particles between about 0.6 nm and 6,000 nm. The botulinum nanoemulsion preparation had two particle size peaks having an average particle size of about 95 nm (Table 2 and Figure 2).

**Table 1: Particle Size Distribution of a Homogenized Botulinum Microemulsion**

| | | **Diam. (nm)** | **% Intensity** | **Width (nm)** |
|---|---|---|---|---|
| **Z-Average:** 3391 | Peak 1 | 1512 | 100 | 76.6 |
| **PDI:** 0.341 | Peak 2 | 0 | 0 | 0 |
| **Intercept:** 0.5852 | Peak 3 | 0 | 0 | 0 |

**Table 2: Particle Size Distribution of a Microfluidized Botulinum Nanoemulsion**

| | | **Diam. (nm)** | **% Intensity** | **Width (nm)** |
|---|---|---|---|---|
| **Z-Average:** 95.33 | Peak 1 | 134.2 | 76.49 | 31.03 |
| **PDI:** 0.252 | Peak 2 | 44.97 | 23.51 | 6.34 |
| **Intercept:** 0.9659 | Peak 3 | 0 | 0 | 0 |

### Example 2: Muscle Relaxant Effect of Transdermal Botulinum Toxin Nanoemulsions

This example demonstrates the therapeutic efficacy of transdermally applied botulinum nanoemulsions (*i.e.,* for example, a nanoemulsion containing botulinum toxin type A).

A botulinum nanoemulsion (9.9 U/100 µl), prepared in accordance with methods similar to Example 1 (*e.g.,* as described in co-pending U.S. Patent Application U.S.S.N. 11/607,436, entitled "BOTULINUM NANOEMULSIONS," filed December 1, 2006), was topically administered to the hind leg gastrocnemius muscle of ten (10) Swiss Webster female mice. A control group of ten (10) Swiss Webster female mice received an identically prepared nanoemulsion except that botulinum toxin was omitted. During the eleven days following treatment, the Digital Abduction Score (DAS) assay was used to determine local muscle weakening efficacy (Aoki, 1999), The DAS values were assigned as follows: (0) flat foot, digit spread same as control leg; (1) flat foot, a difference in the width of digit abduction compared to the control leg or two digits touching and the rest spread completely; (2) flat foot, slight space open at tips of all digits or three digits touching; (3) five digits touching if foot is flat; four digits together if foot is curved; (4) curved foot, all five digits touching. DAS scores of 1-2 were observed for the botulinum toxin nanoemulsion treated group but not in the control group, *i.e.,* the control had a DAS score of 0. Aggravation of the skin (*e.g.,* irritation, redness, *etc.*) was not observed at any time after treatment. The data show that a botulinum toxin nanoemulsion is biologically active upon transdermal administration in a manner similar to conventionally administered botulinum toxin injections.

### REFERENCE Example 3 - Not an example of the invention: Administration of Botulinum Nanoparticle Composition to a Human Subject to Relieve Wrinkles

A topical botulinum nanoemulsion was prepared according to a method similar to Example 1 and applied to a person with significant forehead wrinkles to determine if it could be effective in relaxing the muscles in the forehead that generated those wrinkles (in much the same manner that would be expected from the injection of botulinum into those muscles).

### Methods

A botulinum nanoemulsion was made employing steps similar to Example 1 (see, for example, those described in co-pending U.S. Patent Application U.S.S.N. 11/607,436, entitled "BOTULINUM NANOEMULSIONS," filed December 1, 2006).

The nanoemulsion was added to an equal volume of skin cream (Base PCCA Vanishing Cream Light) and was vortexed into a uniform cream.

A patient who had significant horizontal wrinkles over his forehead, representing overactivity of his frontalis muscles, was selected for treatment. This patient had never been treated with a botulinum product or a dermal filler product. The patient was assessed prior to treatment by a board-certified plastic surgeon using a 4-point wrinkle scale, with a score of "1" equal to "no wrinkle" and a score of "4" equal to significant wrinkle. The patient was assessed using this scale when his face was "At Rest" and when he attempted to create maximal wrinkles by contracting his frontalis muscles which was achieved by attempting to maximally elevate his eyebrows ("Maximal Brow Elevation").

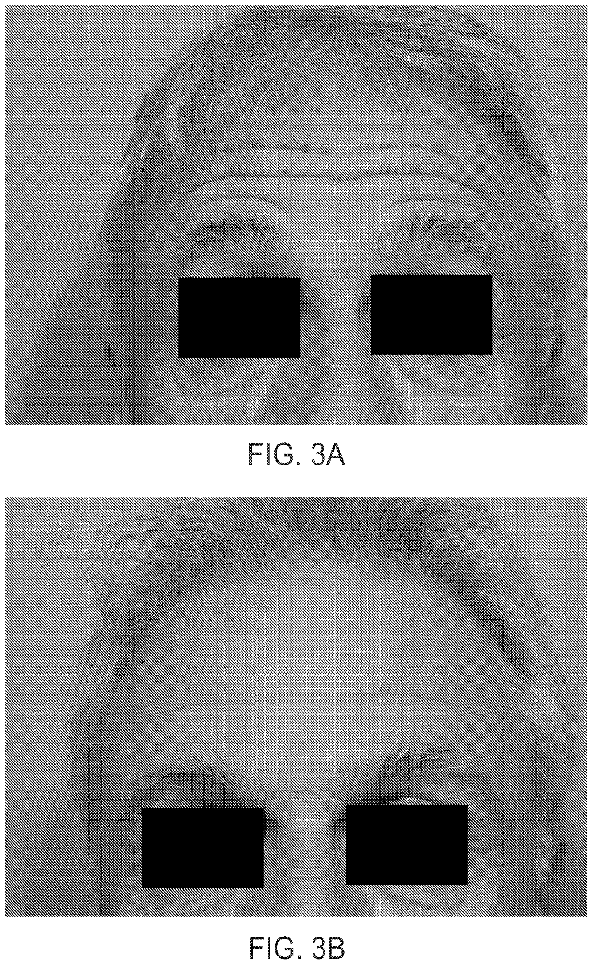

This patient had a score of 4 at rest and 4 on maximal brow elevation. He demonstrated excellent mobility of being able to contract the frontalis muscles. The patient was photographed using a digital SLR camera as well as digital video, both At Rest and when asked to perform a Maximal Brow Elevation (Figure 3A, maximal brow elevation prior to treatment).

The patient was asked not to use any facial make-up or sun-screen on the day of treatment but wash his face prior to coming to the office with Ivory Soap. When at the office, 0.6 cc of the nanoemulsion cream (as prepared in Example 1) was applied to the patient's forehead over the distribution of his frontalis muscles by the plastic surgeon. The cream was applied to the patient's forehead skin by a pipette and rubbed into the skin by the surgeon using his finger (covered by a plastic glove) until the cream was no longer visible to the surgeon. The patient was observed at the physician's office for three hours. He was asked not to touch his forehead for 12 hours and then to wash it off with Ivory Soap and water. The patient was then observed on follow-up after 1 day and then at 1, 2, 4, 8, and 12 weeks. On follow-up visits, the patient's wrinkles At Rest and at Maximal Brow Elevation were assessed by the physician. As well, the physician repeated standardized digital still photographs and video.

### Results

By the first week after treatment, the patient was unable to contract his forehead muscles as evidenced by an inability to lift his brow on requested Maximal Brow Elevation (Figure 3B). His wrinkle score was 2 At Rest and 2 on Maximal Brow Elevation. The physician's clinical assessment was that the treatment had induced a complete paralysis of the treated muscles that was equivalent to treatments he had performed on other patients using injections of botulinum toxin in a similar treatment area. The patient had a slight restoration of brow mobility by Week 8 but continued to have a significant reduction in his brow mobility at Week 12 of observation.

The patient was able to move his other facial muscles under areas of skin not treated and no side-effects were observed by the plastic surgeon, including no changes to the skin immediately after treatment or in any follow-up visit. Likewise, the patient reported no side-effects, including any changes to his skin (*e.g.,* irritation, redness, *etc.*) at any time after treatment.

### Conclusion

In sum, this experiment strongly suggests that the topical botulinum nanoemulsion preparation delivered a significant biological and clinical effect that was assessed by the plastic surgeon to be comparable in clinical efficacy to what would have been expected for following a standard treatment of injected botulinum (in a simple saline solution) for this patient.

### REFERENCE Example 4 - Not an example of the invention: Botulinum Nanoparticle Compositions for Treatment of Hyperhidrosis

As already discussed herein, botulinum toxin type A (BOTOX®) was approved by the FDA in 2004 for the treatment of severe underarm sweating (*i.e.,* primary axillary hyperhidrosis). Small doses of purified botulinum toxin injected into the underarm temporarily block the nerves that stimulate sweating. Side effects include injection-site pain and flu-like symptoms. BOTOX® used for sweating of the palms can cause mild, but temporary, weakness and intense pain during and after injection.

The present invention provides, among other things, improved botulinum toxin therapies for the treatment of hyperhidrosis. Moreover, in demonstrating effective and efficient transdermal delivery of botulinum toxin for the treatment of hyperhidrosis without unwanted clinical effects associated with such delivery (*e.g*., one or more of systemic side effects, damage to underlying nervous tissue [*e.g*., neuronal paralysis], unwanted effects on muscles [*e.g.,* muscle paralysis], undesirable blood levels, flu-like symptoms, *etc.*)., the present invention demonstrates the appropriateness of treating other disorders or conditions associated with sweat or sebaceous glands (*e.g.,* acne, bromhidrosis, chromhidrosis, *etc.*) with botulinum toxin nanoparticle compositions as described herein and/or in United States patent application serial number 11/607,436 (entitled "BOTULINUM NANOEMULSIONS," filed December 1, 2006) (see, for example, Example 5). Furthermore, the data presented herein demonstrate effective and efficient delivery of botulinum toxin to the dermis (which houses the sweat and sebaceous glands). The present invention therefore also demonstrates the usefulness of botulinum nanoparticle compositions as described herein and/or in United States patent application serial number 11/607,436 in the treatment of other disorders and conditions associated with the dermis, or defects therein. For example, as addressed below in Example 6, which describes use of botulinum nanoparticle compositions in the treatment of rosacea.

### Materials and Methods

### Botulinum toxin nanoparticle composition

A nanoemulsion containing botulinum toxin type A prepared as described in Example 1 or in co-pending U.S. Patent Application U.S.S.N. 11/607,436, entitled "BOTULINUM NANOEMULSIONS," filed December 1, 2006 was used in the treatment of hyperhidrosis as described in this Example.

### Selection of Subjects

Inclusion criteria include the following: a) diagnosis of primary axillary hyperhidrosis; b) a Hyperhidrosis Disease Severity Score ("HDSS") of 3 or 4; and c) 50 mg of sweat production per axilla in 5 minutes as measured gravimetrically.

### Experimental Design

Three subjects received a topical treatment containing 80 Units of an approved botulinum pharmaceutical formulated in a nanoparticle composition similar to the prior examples.

### Treatment Procedure

The clinical investigator wiped each axilla of the subject with an alcohol wipe and then wiped dry with cotton gauze. Using a latex-gloved finger, the investigator massaged the topical treatment into the skin of the axilla in the distribution area of the axillary sweat glands. This procedure was completed when there is no topical treatment visible on the surface of the skin. This procedure was employed for the right and left axillary regions.

### Study Duration

Subjects who received the drug treatment were evaluated prior to treatment (Week 0) and at Week 2 following treatment.

### Study Visits

During the first office visit and the Week 2 follow-up visit, the Hyperhidrosis Disease Severity Scale questionnaire ("HDSS questionnaire") was administered to the subjects. This questionnaire is a four point scale, where a score of 1 is the least severe sweating and 4 is most severe sweating. Each subject's sweat production for each axilla was measured using the Gravimetric Sweat Test by placing the subject in a room with relatively constant temperature and humidity, and a) having him or her sit down in a semi-reclining position with the axilla fully exposed and the arm resting comfortably above the head; b) drying the axilla gently with a cotton gauze square; c) using a forceps, placing one filter paper (90 mm diameter) on a balance sensitive to 0.01 mg and recording its weight; d) again using a forceps, placing the measured filter paper on the axilla, covering it with a plastic bag, and taping the edges of the bag against the subject's skin with hypoallergenic tape, forming a seal around the bag; and e) after 5 minutes, gently removing the tape and plastic bag from the subject's axilla and then, using forceps, immediately taking the filter paper from the axilla and placing it onto the scale to record its weight again.

Finally, the starch-iodine test ("Starch-Iodine Test") was administered to the subject by maintaining the subject in a semi-reclining position, with the axilla fully exposed and the arm resting comfortably above the head and a) drying the axilla with cotton gauze; (b) applying povidine solution with a swab to the axilla to create a thin coat and letting it dry; (c) sprinkling starch powder to the area coated with povidine; (d) having the subject rest in this position for 10 minutes; and (e) photographing the axilla from about 1 foot away, with minimal background in the frame.

### Results

### Gravimetric Sweat Test

### Baseline Sweat Production Levels

The three subjects whose results are described below had an average baseline gravimetric sweat production over a five minute period of 368 mg (entry criterion was 50 mg or greater).

**Table 3: Gravimetric Sweat Reduction @ 80 Unit/Axilla Dose**

| | At two weeks |
|---|---|
| Subject #1 | 71% |
| Subject #2 | 71% |
| Subject #3 | 68% |

### HDSS Assessment

**Table 4: HDSS Reduction @ 80 Unit/Axilla Dose**

| | At two weeks |
|---|---|
| Subject #1 | 2.0 points |
| Subject #2 | 2.0 point |
| Subject #3 | 1.0 point |

### Minor's Starch-Iodine Test

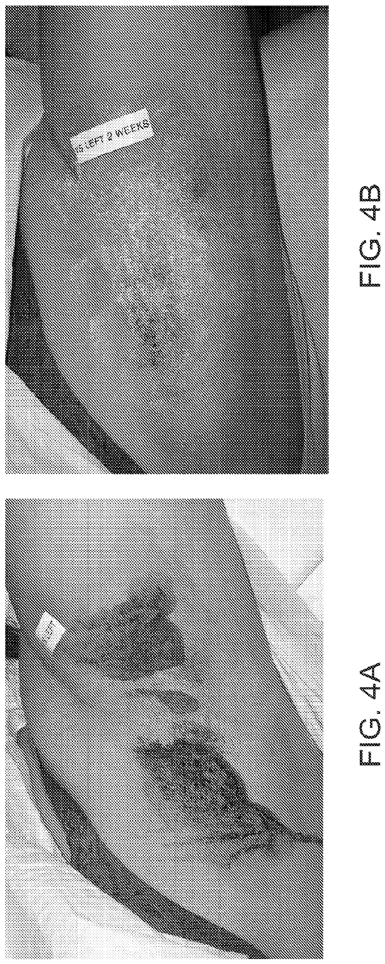

Generally, as reflected in the Minor's Starch-Iodine Test, subjects demonstrated a reduction in sweat production across the area of the axilla with the exception of punctate areas (darkened spots) where there was "break-through" sweating. Figure 4a illustrates a subject prior to treatment where the darkened skin areas and sweat demonstrate profuse sweating at rest; Fugure 4b illustrates a subject to weeks following treatment demonstrating a profound diminishment of sweating at rest as demonstrated by the minor punctuate areas of skin darkening.

### Discussion

The interim results of this study with three subjects studied suggest that botulinum nanoparticle compositions are effective in treating hyperhidrosis without any unwanted side-effects. In particular, the results show efficacy as evaluated by gravimetric sweat measurement, HDSS assessment, and Minor's starch iodine test. In this three-person study, the average maximal gravimetric sweat measurement reduction with a treatment of 80 Units/axilla was 70% at two weeks. By comparison, injected botulinum toxin (50 Units/axilla) achieved an average response rate of 83% in a large placebo-controlled trial (Naumann, 2001). Also by comparison, placebo rates in published controlled studies have an average of 15% - 25% reduction. The initial response observed herein far exceeded expected placebo rates, and therefore, demonstrated a true treatment response. By clinical observation and questioning of the subjects, none of the subjects had any side-effects, including muscular weakness and flu-like symptoms.

While the gravimetric sweat test demonstrated substantial objective reductions in sweat production, some subjects had some focal areas of the sweat gland distribution that had "break-through" sweating as reflected in the Minor's starch iodine test. By comparison, this same phenomenon is observed in standard botulinum injection treatment; often the first treatment round does not adequately treat all sweat glands, that then requires a second more focal treatment. The variable subjective response - as measured by the HDSS - is likely being modulated by the degree of the break-through sweat gland phenomenon in an individual subject. A few sub-optimally treated sweat glands may influence a subjective impression of an individual subject despite a substantial overall reduction in sweating.

In the present example, patients were administered 80 U of botulinum toxin per dose per axilla, and the nanoparticle composition comprising this dose was applied to a surface area that is estimated to be approximately 30 cm² to 40 cm² (as contrasted with, for example, a surface area of approximately 4 cm² for treatment of sub-dermal muscle structures which cause peri-orbital ["Crow's Feet"] wrinkles.)

In sum, the results of this study suggested that response rates for this topical botulinum treatment are promising when considered in their own right and when compared to either injectable treatments or placebo.

Moreover, the results of this study demonstrate effective and efficient delivery of botulinum toxin to areas of the skin including sebaceous glands using inventive nanoparticle compositions. The surprising ability to introduce a significant reduction in sweating without unwanted side-effects reveals a degree of success that demonstrates the applicability of this technology to other gland-based disorders and conditions. For example, these results well illustrate the usefulness of botulinum toxin nanoparticle compositions in accordance with the invention in the treatment of disorders or conditions such as acne, bromhidrosis, and/or chromhidrosis. Furthermore, these results demonstrate effective transdermal delivery of botulinum toxin to the dermis using nanoparticle compositions, and therefore demonstrate usefulness of such compositions in the treatment of disorders and conditions of the dermis such as rosacea.

### Example 5: Botulinum Nanoparticle Compositions for Treatment of Acne

### Materials and Methods

### Selection of Subjects

Inclusion criteria includes a diagnosis of acne.

### Experimental Design

A pre-determined number of subjects (e.g., 2, 4, 8, 10, 12, 14, 16, 18, 20, or more) receives a topical treatment containing a botulinum pharmaceutical in a preparation similar to the prior examples. If no significant adverse events are observed with a starting dose of treatment *(e.g.,* 20, 30, 40, 50, 60, 75, 80, or 100 Units) at a pre-determined endpoint *(e.g.,* 4, 6, 8, 10, or 12 weeks after treatment), a second group of different subjects of a similar size to the first group receives a topical treatment containing a higher dose (*e.g.,* 30, 40, 50, 60, 75, 80, 100, 120, 125, 150, 160, 175, 200, 240, 250, 300, 350, 400, 500, 600, 800, or 1,000 Units) of a botulinum pharmaceutical in a preparation similar to the prior examples. If no significant adverse events are observed with the second group of subjects, a third group of subjects of similar size is treated at a higher dose than the second group of subjects (e.g., 40, 50, 60, 75, 80, 100, 120, 125, 150, 160, 175, 200, 240, 250, 300, 350, 400, 500, 600, 800, or 1,000 Units) using a botulinum pharmaceutical in a preparation similar to the prior examples.

### Treatment Procedure

The clinical investigator wipes a region affected by acne with an alcohol wipe and then wipe dry with cotton gauze. Using a latex-gloved finger, the investigator massages the topical treatment into the skin. This procedure is completed when there is no topical treatment visible on the surface of the skin.

### Study Duration

Subjects are evaluated prior to treatment (Week 0) and 2, 4, 8, 12, and 16 weeks after treatment.

### Study Visits

During the first office visit and the follow-up office visits, the study investigator evaluates the treatment region for number of open comedomes, closed comedomes, raised lesions, papules, pustules, lesion with erythema, and cysts.

### Results

The study shows that the area of treatment is significantly improved on at least one of the follow-up office observation visits when compared to pre-treatment levels for at least some of the number of open comedomes, closed comedomes, raised lesions, papules, pustules, lesion with erythema, and cysts for treatment with at least one of the dose levels selected for study.

Based on these results, the investigator concludes that topical botulinum treatment in accordance with the invention is effective in treating acne.

### Example 6: Botulinum Nanoparticle Compositions for Treatment of Rosacea

### Materials and Methods

### Selection of Subjects

Inclusion criteria includes a diagnosis of rosacea.

### Experimental Design

A pre-determined number of subjects (e.g., 2, 4, 8, 10, 12, 14, 16, 18, 20, or more) receives a topical treatment containing a botulinum pharmaceutical in a preparation similar to the prior examples. If no significant adverse events are observed with a starting dose of treatment (*e.g.,* 20, 30, 40, 50, 60, 75, 80, or 100 Units) at a pre-determined endpoint (*e.g.,* 4, 6, 8, 10, or 12 weeks after treatment), a second group of different subjects of a similar size to the first group receives a topical treatment containing a higher dose (*e.g.,* 30, 40, 50, 60, 75, 80, 100, 120, 125, 150, 160, 175, 200, 240, 250, 300, 350, 400, 500, 600, 800, or 1,000 Units) of a botulinum pharmaceutical in a preparation similar to the prior examples. If no significant adverse events are observed with the second group of subjects, a third group of subjects of similar size is treated at a higher dose than the second group of subjects (e.g., 40, 50, 60, 75, 80, 100, 120, 125, 150, 160, 175, 200, 240, 250, 300, 350, 400, 500, 600, 800, or 1,000 Units) using a botulinum pharmaceutical in a preparation similar to the prior examples.

### Treatment Procedure

The clinical investigator wipes the surface of the affected skin area with an alcohol wipe and then wipes it dry with cotton gauze. Using a latex-gloved finger, the investigator massages the topical treatment into the skin. This procedure is completed when there is no topical treatment visible on the surface of the skin.

### Study Duration

Subjects are evaluated prior to treatment (Week 0) and 2, 4, 8, 12, and 16 weeks after treatment.

### Study Visits

During the first office visit and the four week follow-up office visit, the study investigator evaluates the treatment region in terms of Investigator Global Assessment (using, for example, a seven point scale with 0 = clear, 1 = minimal, 2 = mild to moderate, 4 = moderate, 5 = moderate to severe, and 6 = severe); Subject Global Self-Assessment (using, for example, a nine point scale from 100% worse to no change to 100% improved as measured in 25% increments); and erythema intensity and teleangiectasis intensity (each using, for example, a four point scale from 1 = none, 2 = mild, 3 = moderate, and 4 = severe).

### Results

The study shows that the area of treatment is significantly improved on at least one of the follow-up office observation visits when compared to pre-treatment levels for at least some of the number of Investigator Global Assessment, Subject Global Self-Assessment, erythema intensity or teleangiectasis intensity for treatment with one or more of the applied strengths of botulinum.

Based on these results, the investigator concludes that the inventive topical botulinum treatment was effective in treating rosacea.

### Example 7: Clindamycin Nanoparticle Compositions for Treatment of Acne

### Materials and Methods

### Selection of Subjects

Inclusion criteria includes a diagnosis of acne.

### Experimental Design

A pre-determined number of subjects, *e.g*., 2, 4, 8, 10, 12, 14,16,18, or 20, receives a topical treatment containing a clindamycin pharmaceutical in a preparation similar to the prior examples. If no significant adverse events are observed with a starting dose of, *e.g.,* 0.05%, 0.1%, 0.2%, 0.3%, 0.4%, 0.5%, 0.6%, 0.7%, 0.8%, 0.9%, or 1.0% treatment (applied in a pre-determined manner once, twice, or three times per day) at a pre-determined endpoint (*e.g.,* 4, 6, 8, 10, or 12 weeks after treatment), a second group of different subjects of a similar size to the first group receive a topical treatment containing a higher dose, *e.g.,* 0.1%, 0.2%, 0.3%, 0.4%, 0.5%, 0.6%, 0.7%, 0.8%, 0.9%, 1.0%, 1.5%, 2.0%, 2.5%, or 5% of a clindamycin pharmaceutical in a preparation similar to the prior examples applied wth the same frequency per day as the first group. If no significant adverse events are observed with the second group of subjects, a third group of subjects of similar size is treated at a higher dose than the second group of subjects (e.g., 0.2%, 0.3%, 0.4%, 0.5%, 0.6%, 0.7%, 0.8%, 0.9%, 1.0%, 1.5%, 2.0%, 2.5%, 5%) using a clindamycin pharmaceutical in a preparation similar to the prior examples applied with the same frequency per day as the first group.

### Treatment Procedure

After wiping a region affected by acne with soap and water and then drying, the subject massages the topical treatment into the skin at the pre-determined fequencu of once, twice or three times per day. This procedure is completed when there is no topical treatment visible on the surface of the skin.

### Study Duration

Subjects are evaluated prior to treatment (Week 0) and 2, 4, 8, 12, and 16 weeks after treatment.

### Study Visits

During the first office visit and the follow-up office visits, the study investigator evaluates the treatment region for number of open comedomes, closed comedomes, raised lesions, papules, pustules, lesion with erythema, and cysts.

### Results

The study shows that the area of treatment is significantly improved compared to pre-treatment levels for at least some of the number of open comedomes, closed comedomes, raised lesions, papules, pustules, lesion with erythema, and cysts on at least one of the follow-up examination office visits following treatment with one of the dose levels selected of the preparations.

Based on these results, the investigator concludes that the inventive topical clindamycin treatment was effective in treating acne.

### Example 8: Azeliac Acid Nanoparticle Compositions for Treatment of Rosacea

### Materials and Methods

### Selection of Subjects

Inclusion criteria includes a diagnosis of rosacea.

### Experimental Design

A pre-determined number of subjects, *e.g*., 2, 4, 8, 10, 12, 14,16,18, or 20, receives a topical treatment containing an azeliac acid pharmaceutical in a preparation similar to the prior examples. If no significant adverse events are observed with a starting dose of, *e.g.,* 1%, 2%, 3%, 4%, 5%, 6%, 7%, 8%, 9%, or 10% treatment (applied in a pre-determined manner once, twice, or three times perday) at a pre-determined endpoint (*e.g.,* 4, 6, 8, 10, or 12 weeks after treatment), a second group of different subjects of a similar size to the first group receive a topical treatment containing a higher dose, *e.g.,* 1%, 2%, 3%, 4%, 5%, 6%, 7%, 8%, 9%, 10%, 11%, 12%, 13% 14%, 15%, 20%, 25% of an azeliac acid pharmaceutical in a preparation similar to the prior examples applied wth the same frequency per day as the first group. If no significant adverse events are observed with the second group of subjects, a third group of subjects of similar size is treated at a higher dose than the second group of subjects (*e.g*., 0.3%, 0.4%, 0.5%, 0.6%, 0.7%, 0.8%, 0.9%, 1.0%, 1.5%, 2.0%, 2.5%, 5%) using an azeliac acid pharmaceutical in a preparation similar to the prior examples applied wth the same frequency per day as the first group.

### Treatment Procedure

After washing the treatment area with soap and water and then drying, the subject massages the topical treatment into the skin at a pre-determined frequency of once, twice or three times per day. This procedure is completed when there is no topical treatment visible on the surface of the skin.

### Study Duration

Subjects are evaluated prior to treatment (Week 0) and 2, 4, 8, 12, and 16 weeks after treatment.

### Study Visits

During the first office visit and the follow-up office visit, the study investigator evaluates the treatment region of the nose in terms of Investigator Global Assessment (using, for example, a seven point scale with 0 = clear, 1 = minimal, 2 = mild to moderate, 4 = moderate, 5 = moderate to severe, and 6 = severe); Subject Global Self-Assessment (using, for example, a nine point scale from 100% worse to no change to 100% improved as measured in 25% increments); and erythema intensity and teleangiectasis intensity (each using, for example, a four point scale from 1 = none, 2 = mild, 3 = moderate, and 4 = severe).

### Results

The study show that the area of treatment is significantly improved compared to pre-treatment levels for at least some of the number of Investigator Global Assessment, Subject Global Self-Assessment, erythema intensity or teleangiectasis intensity on at least one of the follow-up examination visits following treatment with one more of the selected dose levels.

Based on these results, the investigator concludes that the inventive topical azeliac acid treatment is effective in treating rosacea.

## Claims

1. A nanoemulsion comprising a population of particles, wherein the majority of particles have diameters between 10 and 300 nanometers and wherein the nanoemulsion comprises an oil, a surfactant and a botulinum toxin,
wherein the oil and surfactant are present in a ratio of ranging from 0.5-2.0,
wherein the nanoemulsion is for use in reducing at least one symptom of a disorder associated with the dermal level of the skin,
wherein the nanoemulsion can penetrate the top layer of the skin without the use of chemical or mechanical skin permeation enhancers or abrasives;
wherein the disorder associated with the dermal level of the skin is rosacea or an excess sebum-producing disorder; and optionally wherein the botulinum toxin is
a) encapsulated within the particles,
b) adsorbed on the surface of the particles,
c) associated with the particle interface,
d) selected from the group comprising type A, type B, type C1, type C2, type D, type E, type F and type G,
e) a botulinum toxin complex,
f) incorporated within an albumin matrix,
g) not incorporated within an albumin matrix,
h) a purified botulinum toxin protein or fragment thereof,
i) isolated, or substantially isolated, from other proteins,
j) isolated, or substantially isolated, from non-toxin proteins,
k) chemically synthesized,
l) produced recombinantly,
m) a fragment of a wild-type toxin, or
n) contains at least one mutation relative to the wild-type toxin.

2. The nanoemulsion for use as claimed in claim 1, wherein the oil is selected from the group consisting of almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macadamia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, wheat germ, and 1349 oils, and combinations thereof or is a medium-chain triglyceride, or is selected from the group consisting of butyl stearate, caprylic triglyceride, capric triglyceride, cyclomethicone, diethyl sebacate, dimethicone 360, isopropyl myristate, mineral oil, octyldodecanol, oleyl alcohol, silicone oil, and combinations thereof or wherein the surfactant is a nonionic detergent, or is selected from the group consisting of phosphoglycerides; phosphatidylcholines; dipalmitoyl phosphatidylcholine (DPPC); dioleylphosphatidyl ethanolamine (DOPE); dioleyloxypropyltriethylammonium (DOTMA); dioleoylphosphatidylcholine; cholesterol; cholesterol ester; diacylglycerol; diacylglycerolsuccinate; diphosphatidyl glycerol (DPPG); hexanedecanol; fatty alcohols such as polyethylene glycol (PEG); polyoxyethylene-9-lauryl ether; a surface active fatty acid, such as palmitic acid or oleic acid; fatty acids; fatty acid monoglycerides; fatty acid diglycerides; fatty acid amides; sorbitan trioleate (Span 85) glycocholate; sorbitan monolaurate (Span 20); polysorbate 20 (TWEEN®20); polysorbate 60 (TWEEN®60); polysorbate 65 (TWEEN®65); polysorbate 80 (TWEEN®80); polysorbate 85 (TWEEN®85); super-refined polysorbate 20 (SR TWEEN®20); super-refined polysorbate 60 (SR TWEEN®60); super-refined polysorbate 65 (SR TWEEN®65); super-refined polysorbate 80 (SR TWEEN®80); super-refined polysorbate 85 (SR TWEEN®85); polyoxyethylene monostearate; surfactin; a poloxomer; a sorbitan fatty acid ester such as sorbitan trioleate; lecithin; lysolecithin; phosphatidylserine; phosphatidylinositol; sphingomyelin; phosphatidylethanolamine (cephalin); cardiolipin; phosphatidic acid; cerebrosides; dicetylphosphate; dipalmitoylphosphatidylglycerol; stearylamine; dodecylamine; hexadecyl-amine; acetyl palmitate; glycerol ricinoleate; hexadecyl stearate; isopropyl myristate; tyloxapol; poly(ethylene glycol)5000-phosphatidylethanolamine; poly(ethylene glycol)400-monostearate; phospholipids; synthetic and/or natural detergents having high surfactant properties; deoxycholates; cyclodextrins; chaotropic salts; ion pairing agents; and combinations thereof.

3. The nanoemulsion for use in as claimed in claim 1, wherein the nanoemulsion does not have more than one oil or more than one surfactant.

4. The nanoemulsion for use as claimed in claim 1, wherein the oil and surfactant are present in a ratio ranging from 1.5 - 2.0 or 0.5-1.0.

5. The nanoemulsion for use as claimed in claim 1, wherein the percent of oil in the nanoemulsion ranges from 1% - 10%, 7%, 6%, 5% or wherein the percent of surfactant in the nanoemulsion ranges from 1% - 20%, 1% - 10%, or 9% or 10%.

6. The nanoemulsion for use as claimed in claim 1, wherein the nanoemulsion is provided as a pharmaceutical composition comprising the nanoemulsion and at least one pharmaceutically acceptable excipient, optionally wherein the composition is selected from the group consisting of a cream, a lotion, a liniment, a gel, an ointment, a spray, a powder, an emollient, an aerosol, and combinations thereof or wherein the composition is administered transdermally using an adhesive patch or a deodorant stick

7. The nanoemulsion for use in as claimed claim 1, wherein the majority of particles have a range of diameters between 10 and 200 nanometers, 10 and 150 nanometers, 10 and 120 nanometers, 10 and 100 nanometers or 10 and 50 nanometers.

8. The nanoemulsion for use as in claimed in claim 1, wherein the population of particles is substantially free of particles having a diameter in excess of 300 nm, 200 nm, or 120 nm.

9. The nanoemulsion for use in as claimed in claim 1, wherein fewer than 50%, 25%, 10%, 5%, or 1% of the particles have a diameter in excess of 300 nm, 200 nm, or 120 nm.

10. The nanoemulsion for use in as claimed in claim 1, wherein the difference between the minimum particle diameter and the maximum particle diameter does not exceed 600 nm, 300 nm, 100 nm or 50 nm.

11. The nanoemulsion for use in as claimed in claim 1, wherein the particles have an average diameter of 100 nm, 75 nm or wherein the particles have an average diameter ranging between 100 - 300 nm, 50-250 nm, 70 - 130 nm, 10 - 100 nm, 50 - 100 nm.

12. The nanoemulsion for use as claimed in claim 1, wherein the nanoemulsion is substantially free of toxic solvents or the nanoemulsion comprises less than 50%, 25%, 10%, 5% or 1% of toxic solvents.

## Patentansprüche

1. Nanoemulsion, umfassend eine Population von Partikeln, wobei der größere Teil der Partikel Durchmesser zwischen 10 und 300 Nanometern besitzt und wobei die Nanoemulsion ein Öl, ein Tensid und ein Botulinumtoxin umfasst,
wobei das Öl und das Tensid in einem Verhältnis im Bereich von 0,5-2,0 vorhanden sind,
wobei die Nanoemulsion zur Verwendung bei der Verringerung mindestens eines Symptoms einer Störung, die mit der Dermisebene der Haut in Zusammenhang steht, dient,
wobei die Nanoemulsion die oberste Hautschicht ohne Verwendung von chemischen oder mechanischen Hautdurchdringungsverstärkern oder Schleifmitteln eindringen kann,
wobei die mit der Dermisebene der Haut in Zusammenhang stehende Störung Rosacea oder eine übermäßig Sebum produzierende Störung ist und wobei gegebenenfalls das Botulinumtoxin
a) in den Partikeln eingekapselt ist,
b) auf der Oberfläche der Partikel adsobiert ist,
c) mit der Partikelgrenzfläche assoziiert ist,
d) aus der Typ A, Typ B, Typ C1, Typ C2, Typ D, Typ E, Typ F und Typ G umfassenden Gruppe ausgewählt ist,
e) ein Botulinumtoxin-Komplex ist,
f) in eine Albumin-Matrix eingebracht ist,
g) nicht in eine Albumin-Matrix eingebracht ist,
h) ein gereinigtes Botulinumtoxinprotein oder ein Fragment davon ist,
i) von anderen Proteinen isoliert oder im Wesentlichen isoliert ist,
j) von Nicht-Toxin-Proteinen isoliert oder im Wesentlichen isoliert ist,
k) chemisch synthetisiert ist,
l) rekombinant produziert ist,
m) ein Fragment eines Wildtyp-Toxins ist oder
n) mindestens eine Mutation in Bezug auf das Wildtyp-Toxin enthält.

2. Nanoemulsion zur Verwendung nach Anspruch 1, wobei das Öl aus der Gruppe ausgewählt ist bestehend aus Mandel-, Aprikosenkern-, Avocado-, Babassu-, Bergamotte-, Schwarze Johannisbeerensamen-, Borretsch-, Kade-, Kamillen-, Canola-, Kümmel-, Carnauba-, Rizinus-, Zimt-, Kakaobutter-, Kokosnuss-, Dorschleber-, Kaffee-, Mais-, Baumwollsamen-, Emu-, Eukalyptus-, Nachtkerzen-, Fisch-, Leinsamenöl, Geraniol, Kürbis-, Traubenkern-, Haselnuss-, Ysopöl, Isopropylmyristat, Jojoba-, Kukuinuss-, Lavandin-, Lavendel-, Zitronen-, Litsea cubeba-, Macadamianuss-, Malven-, Mangosamen-, Douglas-Sumpfblume-Samen-, Nerz-, Muskatnuss-, Oliven-, Orangen-, Kaiserbarsch-, Palmen-, Palmkern-, Pfirsichkern-, Erdnuss-, Mohnsamen-, Kürbiskern-, Raps-, , Reiskleie-, Rosmarin-, Saflor-, Sandelholz-, Sasquana-, Bohnenkraut-, Sanddorn-, Sesam-, Sheabutter-, , Silikon-, Sojabohnen-, Sonnenblumen-, Teebaum-, Distel-, Tsubaki-, Vetiver-, Walnuss-, Weizenkeim- und 1349-Öl und Kombinationen davon oder ein mittelkettiges Triglycerid ist oder aus der Gruppe ausgewählt ist bestehend aus Butylstearat, Caprylsäuretriglycerid, Caprinsäuretriglycerid, Cyclomethicon, Diethylsebacat, Dimethicon 360, Isopropylmyristat, Mineralöl, Octyldodecanol, Oleylalkohol, Silikonöl und Kombinationen davon oder wobei das Tensid ein nichtionisches Detergenz ist oder aus der Gruppe ausgewählt ist bestehend aus Phosphoglyceriden, Phosphatidylcholinen, Dipalmitoylphosphatidylcholin (DPPC), Dioleylphosphatidylethanolamin (DOPE), Dioleyloxypropyltriethylammonium (DOTMA), Dioleoylphosphatidylcholin, Cholesterin, Cholesterinester, Diacylglycerin, Diacylglycerinsuccinat, Diphosphatidylglycerin (DPPG), Hexandecanol; Fettalkoholen wie Polyethylenglykol (PEG); Polyoxyethylen-9-laurylether, einer oberflächenaktiven Fettsäure wie Palmitinsäure oder Ölsäure, Fettsäuren, Fettsäuremonoglyceriden, Fettsäurediglyceriden, Fettsäureamiden, Sorbitantrioleat (Span 85) Glykocholat, Sorbitanmonolaurat (Span 20), Polysorbat 20 (TWEEN® 20), Polysorbat 60 (TWEEN® 60), Polysorbat 65 (TWEEN® 65), Polysorbat 80 (TWEEN® 80), Polysorbat 85 (TWEEN® 85), superraffiniertem Polysorbat 20 (SR TWEEN® 20), superraffiniertem Polysorbat 60 (SR TWEEN® 60), superraffiniertem Polysorbat 65 (SR TWEEN® 65), superraffiniertem Polysorbat 80 (SR TWEEN® 80), superraffiniertem Polysorbat 85 (SR TWEEN® 85), Polyoxyethylenmonostearat, Surfactin, einem Poloxomer, einem Sorbitanfettsäureester wie Sorbitantrioleat; Lecithin, Lysolecithin, Phosphatidylserin, Phosphatidylinositol, Sphingomyelin, Phosphatidylethanolamin (Kephalin), Cardiolipin, Phosphatidsäure, Cerebrosiden, Dicetylphosphat, Dipalmitoylphosphatidylglycerin, Stearylamin, Dodecylamin, Hexadecylamin, Acetylpalmitat, Glycerinricinoleat, Hexadecylstearat, Isopropylmyristat, Tyloxapol, Poly(ethylenglykol)5000-phosphatidylethanolamin, Poly(ethylenglykol)400-monostearat, Phospholipiden, synthetischen und/oder natürlichen Detergenzien mit hohen Tensideigenschaften, Desoxycholaten, Cyclodextrinen, chaotropen Salzen, Ionenpaarungsmitteln und Kombinationen davon.

3. Nanoemulsion zur Verwendung nach Anspruch 1, wobei die Nanoemulsion nicht mehr als ein Öl oder mehr als ein Tensid aufweist.

4. Nanoemulsion zur Verwendung nach Anspruch 1, wobei das Öl und das Tensid in einem Verhältnis im Bereich von 1,5-2,0 oder 0,5-1,0 vorhanden sind.

5. Nanoemulsion zur Verwendung nach Anspruch 1, wobei der prozentuale Anteil an Öl in der Nanoemulsion im Bereich von 1%-10%, 7%, 6%, 5% liegt oder wobei der prozentuale Anteil an Tensid in der Nanoemulsion im Bereich von 1%-20%, 1%-10% oder 9% oder 10% liegt.

6. Nanoemulsion zur Verwendung nach Anspruch 1, wobei die Nanoemulsion als pharmazeutische Zusammensetzung bereitgestellt wird, die die Nanoemulsion und mindestens einen pharmazeutisch annehmbaren Exzipienten umfasst, wobei gegebenenfalls die Zusammensetzung aus der Gruppe ausgewählt ist bestehend aus einer Creme, einer Lotion, einem Einreibemittel, einem Gel, einer Salbe, einem Spray, einem Pulver, einem Aufweichungsmittel, einem Aerosol und Kombinationen davon oder wobei die Zusammensetzung transdermal unter Verwendung eines Klebepflasters oder eines Deodorantstifts verabreicht wird.

7. Nanoemulsion zur Verwendung nach Anspruch 1, wobei der größere Teil der Partikel einen Bereich von Durchmessern zwischen 10 und 200 Nanometern, 10 und 150 Nanometern, 10 und 120 Nanometern, 10 und 100 Nanometern oder 10 und 50 Nanometern aufweist.

8. Nanoemulsion zur Verwendung nach Anspruch 1, wobei die Population von Partikeln im Wesentlichen frei von Partikeln mit einem Durchmesser von mehr als 300 nm, 200 nm oder 120 nm ist.

9. Nanoemulsion zur Verwendung nach Anspruch 1, wobei weniger als 50%, 25%, 10%, 5% oder 1% der Partikel einen Durchmesser von mehr als 300 nm, 200 nm oder 120 nm aufweisen.

10. Nanoemulsion zur Verwendung nach Anspruch 1, wobei die Differenz zwischen dem minimalen Partikeldurchmesser und dem maximalen Partikeldurchmesser nicht mehr als 600 nm, 300 nm, 100 nm oder 50 nm beträgt.

11. Nanoemulsion zur Verwendung nach Anspruch 1, wobei die Partikel einen durchschnittlichen Durchmesser von 100 nm, 75 nm besitzen oder wobei die Partikel einen durchschnittlichen Durchmesser im Bereich zwischen 100-300 nm, 50-250 nm, 70-130 nm, 10-100 nm, 50-100 nm besitzen.

12. Nanoemulsion zur Verwendung nach Anspruch 1, wobei die Nanoemulsion im Wesentlichen frei von toxischen Lösungsmitteln ist oder die Nanoemulsion weniger als 50%, 25%, 10%, 5% oder 1% toxische Lösungsmittel umfasst.

## Revendications

1. Nanoémulsion comprenant une population de particules, dans laquelle la majorité des particules ont des diamètres compris entre 10 et 300 nanomètres et la nanoémulsion comprenant une huile, un tensioactif et une toxine botulique,
dans laquelle l'huile et le tensioactif sont présents dans un rapport dans la plage de 0,5 à 2,0,
la nanoémulsion étant pour utilisation dans la réduction d'au moins un symptôme d'un trouble associé au niveau dermique de la peau,
la nanoémulsion pouvant pénétrer la couche supérieure de la peau sans l'utilisation d'adjuvants de perméation ou d'abrasifs chimiques ou mécaniques ;
le trouble associé au niveau dermique de la peau étant l'acné rosacée ou un trouble de production d'excès de sébum ; et la toxine botulique étant facultativement
a) encapsulée dans les particules,
b) adsorbée sur la surface des particules,
c) associée à l'interface des particules,
d) choisie dans le groupe comprenant les type A, type B, type C1, type C2, type D, type E, type F et type G,
e) un complexe de toxine botulique,
f) incorporée dans une matrice d'albumine,
g) non incorporée dans une matrice d'albumine,
h) une protéine de toxine botulique purifiée ou un fragment de celle-ci,
i) isolée, ou sensiblement isolée, des autres protéines,
j) isolée, ou sensiblement isolée, des protéines non-toxine,
k) chimiquement synthétisée,
l) produite de façon recombinante,
m) un fragment d'une toxine de type sauvage, ou
n) contient au moins une mutation par rapport à la toxine de type sauvage.

2. Nanoémulsion pour utilisation selon la revendication 1, dans laquelle l'huile est choisie dans le groupe constitué des huiles d'amande, noyau d'abricot, avocat, babassu, bergamote, graine de cassis, bourrache, cade, camomille, canola, carvi, carnauba, ricin, cannelle, beurre de cacao, noix de coco, foie de morue, café, maïs, graine de coton, émeu, eucalyptus, onagre, poisson, graine de lin, géraniol, gourde, pépin de raisin, noisette, hysope, myristate d'isopropyle, jojoba, noix de kukui, lavandin, lavande, citron, Litsea cubeba, noix de macadamia, mauve, graine de mangue, graine de limnanthe, vison, noix de muscade, olive, orange, hoplostèthe orange, palme, palmiste, noyau de pêche, cacahuète, graine de pavot, graine de citrouille, colza, son de riz, romarin, carthame, bois de santal, sasquana, sarriette, argousier, sésame, beurre de karité, silicone, soja, tournesol, théier, chardon, tsubaki, vétiver, noyer, germe de blé, et 1349, et des combinaisons de celles-ci ou est un triglycéride à chaîne moyenne, ou est choisie dans le groupe constitué des stéarate de butyle, triglycéride caprylique, triglycéride caprique, cyclométhicone, sébacate de diéthyle, diméthicone 360, myristate d'isopropyle, huile minérale, octyldodécanol, alcool oléylique, huile de silicone, et des combinaisons de ceux-ci ou dans laquelle le tensioactif est un détergent non ionique, ou est choisi dans le groupe constitué des phosphoglycérides ; phosphatidylcholines ; dipalmitoylphosphatidylcholine (DPPC) ; dioléylphosphatidyléthanolamine (DOPE) ; dioléyloxypropyltriéthylammonium (DOTMA) ; dioléoylphosphatidylcholine ; cholestérol ; ester de cholestérol ; diacylglycérol ; diacylglycérolsuccinate ; diphosphatidylglycérol (DPPG) ; hexanedécanol ; alcools gras tels que le polyéthylène glycol (PEG) ; éther polyoxyéthylène-9-laurylique ; acide gras tensioactif, tel que l'acide palmitique ou l'acide oléique ; acides gras ; monoglycérides d'acide gras ; diglycérides d'acide gras ; amides d'acide gras ; trioléate de sorbitane (Span 85) glycocholate ; monolaurate de sorbitane (Span 20) ; polysorbate 20 (TWEEN®20) ; polysorbate 60 (TWEEN®60) ; polysorbate 65 (TWEEN®65) ; polysorbate 80 (TWEEN®80) ; polysorbate 85 (TWEEN®85) ; polysorbate super-raffiné 20 (SR TWEEN®20) ; polysorbate super-raffiné 60 (SR TWEEN®60) ; polysorbate super-raffiné 65 (SR TWEEN®65) ; polysorbate super-raffiné 80 (SR TWEEN®80) ; polysorbate super-raffiné 85 (SR TWEEN®85) ; monostéarate de polyoxyéthylène ; surfactine ; poloxamère ; ester d'acide gras de sorbitane tel que le trioléate de sorbitane ; lécithine ; lysolécithine ; phosphatidylsérine ; phosphatidylinositol ; sphingomyéline ; phosphatidyléthanolamine (céphaline) ; cardiolipine ; acide phosphatidique ; des cérébrosides ; phosphate de dicétyle ; dipalmitoylphosphatidylglycérol ; stéarylamine ; dodécylamine ; hexadécylamine ; palmitate d'acétyle ; ricinoléate de glycérol ; stéarate d'hexadécyle ; myristate d'isopropyle ; tyloxapol ; poly(éthylène glycol) 5000-phosphatidyléthanolamine ; monostéarate de poly(éthylène glycol) 400 ; phospholipides ; détergents synthétiques et/ou naturels ayant des propriétés tensioactives élevées ; désoxycholates ; cyclodextrines ; sels chaotropes ; agents d'appariement d'ion ; et des combinaisons de ceux-ci .

3. Nanoémulsion pour utilisation selon la revendication 1, la nanoémulsion ne comportant pas plus d'une huile ou plus d'un tensioactif.

4. Nanoémulsion pour utilisation selon la revendication 1, dans laquelle l'huile et le tensioactif sont présents dans un rapport dans la plage de 1,5 à 2,0 ou de 0,5 à 1,0.

5. Nanoémulsion pour utilisation selon la revendication 1, dans laquelle le pourcentage d'huile dans la nanoémulsion est dans la plage de 1 % à 10 %, 7 %, 6 %, 5 % ou dans laquelle le pourcentage de tensioactif dans la nanoémulsion est dans la plage de 1 % à 20 %, 1 % à 10 %, ou 9 % ou 10 %.

6. Nanoémulsion pour utilisation selon la revendication 1, la nanoémulsion étant fournie sous la forme d'une composition pharmaceutique comprenant la nanoémulsion et au moins un excipient pharmaceutiquement acceptable, la composition étant facultativement choisie dans le groupe constitué d'une crème, une lotion, un liniment, un gel, une pommade, un spray, une poudre, un émollient, un aérosol, et des combinaisons de ceux-ci ou la composition étant administrée par voie transdermique au moyen d'un timbre adhésif ou un stick de déodorant.

7. Nanoémulsion pour utilisation selon la revendication 1, dans laquelle la majorité des particules ont une plage de diamètres comprise entre 10 et 200 nanomètres, 10 et 150 nanomètres, 10 et 120 nanomètres, 10 et 100 nanomètres ou 10 et 50 nanomètres.

8. Nanoémulsion pour utilisation selon la revendication 1, dans laquelle la population de particules est sensiblement exempte de particules ayant un diamètre supérieur à 300 nm, 200 nm ou 120 nm.

9. Nanoémulsion pour utilisation selon la revendication 1, dans laquelle moins de 50 %, 25 %, 10 %, 5 % ou 1 % des particules ont un diamètre supérieur à 300 nm, 200 nm ou 120 nm.

10. Nanoémulsion pour utilisation dans selon la revendication 1, dans laquelle la différence entre le diamètre de particule minimal et le diamètre de particule maximal ne dépasse pas 600 nm, 300 nm, 100 nm ou 50 nm.

11. Nanoémulsion pour utilisation dans selon la revendication 1, dans laquelle les particules ont un diamètre moyen de 100 nm, 75 nm ou dans laquelle les particules ont un diamètre moyen compris entre 100 à 300 nm, 50 à 250 nm, 70 à 130 nm, 10 à 100 nm, 50 à 100 nm.

12. Nanoémulsion pour utilisation selon la revendication 1, la nanoémulsion étant sensiblement exempte de solvants toxiques ou bien la nanoémulsion comprend moins de 50 %, 25 %, 10 %, 5 % ou 1 % de solvants toxiques.
